(19) Europäisches Patentamt
European Patent Office
Office européen des brevets

(11) EP 2 905 278 A1

(12) **EUROPEAN PATENT APPLICATION**
published in accordance with Art. 153(4) EPC

(43) Date of publication:
**12.08.2015 Bulletin 2015/33**

(21) Application number: **13841119.4**

(22) Date of filing: **27.09.2013**

(51) Int Cl.:
*C07D 231/12* (2006.01) *C07D 233/54* (2006.01)
*C07D 239/20* (2006.01) *C07D 401/06* (2006.01)
*C07D 413/06* (2006.01) *C07D 419/06* (2006.01)
*A61P 25/06* (2006.01)

(86) International application number:
**PCT/BR2013/000372**

(87) International publication number:
**WO 2014/047707 (03.04.2014 Gazette 2014/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.09.2012 BR 102012024778**

(71) Applicant: **Cristália Produtos Químicos Farmacêuticos LTDA.**
**13970-000 Itapira (BR)**

(72) Inventors:
- **PACHECO, Ogari**
  **CEP: 13970-000 Itapira - SP (BR)**
- **GARCÍA, Ariel**
  **CEP: 13970-000 Itapira - SP (BR)**
- **OLIVEIRA, Kesley**
  **CEP: 13970-000 Itapira - SP (BR)**
- **GODOY, Lidiane**
  **CEP: 13970-000 Itapira - SP (BR)**
- **MOREIRA, Roberto**
  **CEP: 13970-000 Itapira - SP (BR)**

(74) Representative: **Alves Moreira, Pedro**
**Rua do Patrocinio, 94**
**1399-019 Lisbon (PT)**

(54) **HETEROAROMATIC COMPOUNDS, METHOD FOR PREPARING THE COMPOUNDS, PHARMACEUTICAL COMPOSITIONS, USES AND METHOD FOR TREATING ACUTE AND CHRONIC PAIN**

(57) The present invention refers to a compound of formula (I)

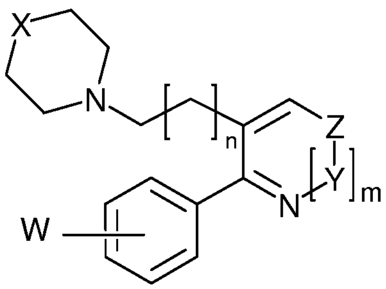

(I)

or a pharmaceutically acceptable salt thereof, having analgecic activity. Particularly, the compounds of the instant invention are useful to treat or prevent acute and chronic pain, especially neuropathic pain. Additionally, the present invention provides a procces for preparing the compounds, a pharmaceutical composition that comprises a compound of formula (I), and a method for treatment of acute and chronic pain.

EP 2 905 278 A1

## Description

### Technical field of the invention

The present invention refers to heteroaromatic compounds having analgesic activity, effective in the treatment of acute and chronic pain, particularly neuropathic pain.

Pain is a symptom of several clinical disorders and affects great share of the population. It is usually responsible for a significant share of health services demand and constitutes a multifactorial phenomenon, involving psychosocial, behavioral and physiopathological processes.

According to International Association for the Study of Pain (IASP) pain is described as "An unpleasant sensory and emotional experience associated with actual or potential tissue damage, or described in terms of such damage." [Crombie I.K. The potencial of Epidemiology. In: Crombie I.K., Croft P.R., Linton S.J., Le Resche L, Von Korff M, editors. Epidemiology of pain: a report of the Task Force on Epidemiology. Seattle: IASP Press; 1999. cap.1, p. 1-5].

From the clinical point of view, pain is classified according to its duration and evolution, and can be of two types: acute or chronic. Acute pain has a physiological nature, being triggered by tissue injury and has a warning and defense role. It is mediated by thermal, mechanical or chemical stimulation of the nociceptors. It usually has a short duration, and may be of two types: neurogenic, when it is caused by a peripheral nociceptive stimulus, or inflammatory, when it is caused by an inflammatory reaction. Therefore, the pain has a well-determined cause-effect relationship.

Chronic pain has a pathological nature and it is associated with the alteration of the central mechanisms of nociception, which results in its progressive centralization. Chronic pain is a persistent pain, lasting for at least three months, which becomes gradually incapacitating. Due to its long duration, chronic pain loses its function to maintain homeostasis and to be an alert signal, since it may exist or persist even in the absence of a real injury, producing persistent changes in the psychomotor (emotional) behavior and causing functional impairment, suffering, progressive disability and socio-economic cost [Almeida, RF, Roizenblatt, S., Tufik, S. 2004. Afferent pain pathways: a neuroanatomical review. Brain Res 1000: 40-56]. The most common chronic pains include those of oncologic origin, those associated with myofascial pain syndrome, headache, fibromyalgia, pain associated with rheumatoid arthritis, phantom limb pain, central pain syndromes and neuropathic pain [Ashburn, M. A.; Staats, P. S. 1999. Management of chronic pain. Lancet, v. 353, p. 1865-1869,].

Neuropathic pain is frequently described as having a continuous or sting burning nature, and is commonly associated with hyperalgesia and allodynia. Hyperalgesia is defined as an exacerbated sensitivity to a painful (injurious) stimulus and allodynia is defined as pain in response to a stimulus that usually does not cause pain (non-injurious).

Currently, opioids and non-sterldal anti-inflammatory drugs (NSAIDs) are effective drugs for the treatment of acute pain. However, the high incidence of adverse effects, such as the tolerance phenomenon, dependency, nausea, constipation, and respiratory depression, may be limiting factors for the therapeutic use of opioids. Although NSAIDs do not cause dependence and are also effective in chronic pain, they are associated with significant adverse effects, such as gastrointestinal lesions, nephrotoxicity, and platelet aggregation inhibition, among others.

Therapeutic options for neuropathic pains are less effective or less tolerable. Due to their distinct pathophysiological mechanisms, drugs that are useful in the treatment of other types of pain have only limited efficacy in the treatment of neuropathic pain. Alternatively, some classes of analgesics adjuvant have been used to aid the treatment of neuropathic pain, including antidepressants, anticonvulsants, local anesthetics, muscle relaxants and sympatholytics, among others.

In a general context, the currently available treatments have shown limited efficacy, since the total pain relief is rarely achieved and there are high incidence of known adverse effects. Thus, the research for new drugs, more effective and safer, still presents a challenge to the pharmaceutical industry.

U.S. Patent No. 6,403,575 B1 (Holladay et al., 2002) describes heteroaromatic compounds with analgesic activity in acute and chronic pain, but these compounds were not effect in hyperalgesia and allodynia phases of neuropathic pain.

European Patent EP 1065205 B1 (Nakazato et al., 2003) describes heteroaromatic compounds as dopamine D4 receptor antagonists useful only as antipsychotics drugs.

The objective of the present invention is to provide novel heteroaromatic compounds, effective and safe, for the treatment of acute and chronic pain, particularly neuropathic pain.

### Description of the figures

Descriptions of the Figures that accompany this detailed description are presented below, for better understanding and illustration of the present invention.

**Figure 1:** Analgesic activity (%) of compounds C3, C9, C12, and C11, in acute pain, compared to morphine sulfate,

in the hot plate model, in mice, during 100 minutes. Compounds were diluted in dimethylsulfoxide (DMSO) and administered through intraperitoneal (ip) route at a dose of 32.25 μmol/kg. Data presented as mean ± SEM (standard error of the mean) (n = 10/compound).

**Figure 2:** Analgesic activity (%) of compounds C13, C17, and C15, in acute pain, compared to morphine sulfate, in hot plate model, in mice, during 100 minutes. Compounds were diluted in water and administered through intraperitoneal (ip) route at a dose of 32.25 μmol/kg. Data presented as mean ± SEM (standard error of the mean) (n = 10/compound).

**Figure 3:** Analgesic effect of amitriptyline hydrochloride in the chronic constriction injury (CCI) of the sciatic nerve model of rat's left paw **(A). (B)** represents the effect on rat's right paw (contralateral), with no CCI. The compound was diluted in water and administered at a dose of 32.25 μmol/kg through ip route during 7 days. Latency(s) = paw withdrawal latency in seconds. Data presented as mean ± SEM (n=7). Treatment started 7 days after the surgery. *p<0.05; **p<0.005; ***p<0.0005 vs. "control before surgery" and #p<0.05; ###p<0.005 vs "control post-surgery".

**Figure 4:** Analgesic effect of compound C3 in the CCI of the sciatic nerve model of rat's left paw **(A). (B)** represents rat's right paw (contralateral) with no CCI. The compound was diluted in water and administered at a dose of 32.25 μmol/kg through ip route during 7 days. Data presented as mean ± SEM (n=8). Latency(s) = paw withdrawal latency in seconds. **p<0.005 and ***p<0.0005 vs. "control before surgery"; #p<0.05 and ##p<0.005 vs. "control post-surgery".

**Figure 5:** Analgesic effect of compound C3, in the CCI of the sciatic nerve model of rat's left paw (dose-response curve). The compound was diluted in water and administered through intraperitoneal route during 7 days, at the doses of 3.22, 16.12 and 32.25 μmol/kg (1, 5, and 10 mg/kg, respectively). Data presented as mean ± SEM of 4-5 animals per dose. Latency(s) = paw withdrawal latency in seconds. *p<0.05 or **p<0.005 vs "control before surgery" (day 0); #p<0.05 or ##p<0.005 vs "control post-surgery" (day 7).

**Figure 6:** Analgesic effect of compound C9, in the CCI of the sciatic nerve model of rat's left paw **(A). (B)** represents rat's right paw (contralateral) with no CCI. The compound was diluted in saline and administered at the dose of 32.25 μmol/kg through ip route during 7 days. Data presented as mean ± SEM (n=5). Treatment started 7 days after surgery. Latency(s) = paw withdrawal latency in seconds. *p<0.05 and **p<0.01 vs "control before surgery"; #p<0.05 vs "control post-surgery".

**Figure 7:** Analgesic effect of compound C12, in the CCI of the sciatic nerve model of rat's left paw **(A). (B)** represents rat's right paw (contralateral) with no CCI. The compound was diluted in saline and administered at the dose of 32.25 μmol/kg through ip route during 7 days. Data presented as mean ± SEM (n=6). Treatment started 7 days after surgery. Latency(s) = paw withdrawal latency in seconds. **p<0.005 and *p<0.05 vs. "control before surgery"; #p<0.05 vs "control post-surgery".

**Figure 8:** Analgesic effect of compound C11, in the CCI of the sciatic nerve model of rat's left paw **(A). (B)** represents rat's right paw (contralateral) with no CCI. The compound was diluted in water and administered at the dose of 32.25 μmol/kg through ip route during 7 days. Data presented as mean ± SEM (n=6). Treatment started 7 days after surgery. Latency(s) = paw withdrawal latency in seconds. *p<0.005 or **p<0.005 and p<0.0005 vs. "control before surgery"; ##p<0.005 vs "control post-surgery".

**Figure 9:** Analgesic effect of compound C18, in the CCI of the sciatic nerve model of rat's left paw **(A). (B)** represents rat's right paw (contralateral) with no CCI. The compound was diluted in water and administered at the dose of 32.25 μmol/kg through ip route during 7 days. Data presented as mean ± SEM (n=5). Treatment started 7 days after surgery. Latency(s) = paw withdrawal latency in seconds. *p<0.05 and ***p<0.0005 vs. "control before surgery"; ##p<0.005 vs "control post-surgery".

**Figure 10:** Analgesic effect of compound C5, in the CCI of the sciatic nerve model of rat's left paw **(A). (B)** represents rat's right paw (contralateral) with no CCI. The compound was diluted in water and administered at the dose of 32.25 μmol/kg through ip route during 7 days. Data presented as mean ± SEM (n=5). Treatment started 7 days after surgery. Latency(s) = paw withdrawal latency in seconds. *p<0.05 and **p<0.005 vs "control before surgery"; ##p<0.005 vs "control post-surgery".

**Figure 11:** Analgesic effect of compound C17, in the CCI of the sciatic nerve model of rat's left paw **(A). (B)** represents

rat's right paw (contralateral) with no CCI. The compound was diluted in DMSO and administered at the dose of 32.25 μmol/kg through ip route during 7 days. Data presented as mean ± SEM (n=6). Treatment started 7 days after surgery. Latency(s) = paw withdrawal latency in seconds. *p<0.05; **p<0.005 and ***p<0,0005 vs "control before surgery"; #p<0.05 and ##p<0.005 vs "control post-surgery".

**Figure 12:** Analgesic effect of compound C6, in the CCI of the sciatic nerve model of rat's left paw **(A). (B)** represents rat's right paw (contralateral) with no CCI. The compound was diluted in water and administered at the dose of 32.25 μmol/kg through ip route during 7 days. Data presented as mean ± SEM (n=6). Treatment started 7 days after surgery. Latency(s) = paw withdrawal latency in seconds. *p<0.05 and **p<0.005 vs "control before surgery"; #p<0 05 vs "control post-surgery".

**Figure 13:** Analgesic effect of compound C15, in the CCI of the sciatic nerve model of rat's left paw **(A). (B)** represents rat's right paw (contralateral) with no CCI. The compound was diluted in DMSO and administered at the dose of 32.25 μmol/kg through ip route during 7 days. Data presented as mean ± SEM (n=5). Treatment started 7 days after surgery. Latency(s) = paw withdrawal latency in seconds. *p<0.05 and **p<0.005 vs "control before surgery"; #p<0.05 and ##p<0.005 vs "control post-surgery".

**Figure 14:** Analgesic effect of compound C16, in the CCI of the sciatic nerve model of rat's left paw **(A). (B)** represents rat's right paw (contralateral) with no CCI. The compound was diluted in DMSO and administered at the dose of 32.25 μmol/kg through ip route during 7 days. Data presented as mean ± SEM (n=5). Treatment started 7 days after surgery. Latency(s) = paw withdrawal latency in seconds. *p<0.05, **p<0.005 vs. "control before surgery"; #p<0.05 or ##p<0.005 vs "control post-surgery".

**Figure 15:** Analgesic effect of amitriptyline hydrochloride on thermal hypersensitivity or hyperalgesia in rats subjected or not (sham) to spinal nerve ligation (SNL) model. Amitriptylin hydrochloride was diluted in water and administered through ip route during 7 days. Treatment started at day 7 after surgery and the dose administered was 32.25 μmol/kg. Data presented as mean ± SEM (n=6). Latency(s) = paw withdrawal latency in seconds. *p<0.05, ***p<0.0005 vs "control before surgery" (day 0); #p<0.05 vs "control post-surgery".

**Figure 16:** Analgesic effect of amitriptyline hydrochloride on mechanical allodynia in rats subjected or not (sham) to SNL model. Amitriptylin hydrochloride was diluted in water and administered through ip route during 7 days. Treatment started at day 7 after surgery and the dose administered was 32.25 μmol/kg. Data presented as mean ± SEM (n=6). Threshold (g) = paw withdrawal threshold in grams. ***p<0.0005 vs "control before surgery" (day 0).

**Figure 17:** Analgesic effect of compound C9 on thermal hypersensitivity or hyperalgesia in rats subjected or not (sham) to SNL model. The compound was diluted in water and administered through ip route during 7 days. Treatment started at day 7 after surgery and the dose administered was 32.25 μmol/kg. Data presented as mean ± SEM (n=5). Latency(s) = paw withdrawal latency in seconds. **p<0.005 and ***p<0.0005 vs "control before surgery" (day 0); ##p<0.005 vs "control post-surgery" (day 7).

**Figure 18:** Analgesic effect of compound C9 on mechanical allodynia in rats subjected or not (sham) to SNL model. The compound was diluted in water and administered through ip route during 7 days. Treatment started at day 7 after surgery and the dose administered was 32.25 μmol/kg. Data presented as mean ± SEM (n=5). Threshold (g) = paw withdrawal threshold in grams. *p<0.05, **p<0.005 and ***p<0.0005 vs. "control before surgery" (day 0); #p<0.05 vs (day 7) "control post-surgery".

**Figure 19:** Analgesic effect of compound C12 on thermal hypersensitivity or hyperalgesia in rats subjected or not (sham) to SNL model. The compound was diluted in water and administered through ip route during 7 days. Treatment started at day 7 after surgery and the dose administered was 32.25 μmol/kg. Data presented as mean ± SEM (n=5). Latency(s) = paw withdrawal latency in seconds. *p<0.05, **p<0.005 vs "control before surgery" (day 0); ##p<0.005 vs "control post-surgery" (day 7).

**Figure 20:** Analgesic effect of compound C12 on mechanical allodynia in rats subjected or not (sham) to SNL model. The compound was diluted in water and administered through ip route during 7 days. Treatment started at day 7 after surgery and the dose administered was 32.25 μmol/kg. Data presented as mean ± SEM (n=6). Threshold (g) = paw withdrawal threshold in grams. ***p<0.0005 vs "control before surgery" (day 0); #p<0.05 vs (day 7) "control post-surgery".

**Figure 21:** Analgesic effect of compound C11 on thermal hypersensitivity or hyperalgesia in rats subjected or not (sham) to SNL model. The compound was diluted in water and administered through ip route during 7 days. Treatment started at day 7 after surgery and the dose administered was 32.25 μmol/kg. Data presented as mean ± SEM (n=6). Latency(s) = paw withdrawal latency in seconds. **p<0.005 and ***p<0.0005 vs "control before surgery" (day 0); ##p<0.005 vs "control post-surgery" (day 7).

**Figure 22:** Analgesic effect of compound C11 on mechanical allodynia in rats subjected or not (sham) to SNL model.The compound was diluted in water and administered through ip route during 7 days. Treatment started at day 7 after surgery and the dose administered was 32.25 μmol/kg. Data presented as mean ± SEM (n=6). Threshold (g) = paw withdrawal threshold in grams. ***p<0.0005 vs "control before surgery" (day 0); ##p<0.005 vs "control post-surgery" (day 7).

**Figure 23:** Analgesic effect of compound C17 on thermal hypersensitivity or hyperalgesia in rats subjected or not (sham) to SNL model. The compound was diluted in DMSO and administered through ip route during 7 days. Treatment started at day 7 after surgery and the dose administered was 32.25 μmol/kg. Data presented as mean ± SEM (n=6). Latency(s) = paw withdrawal latency in seconds. **p<0.005 vs "control before surgery" (day 0); ##p<0.005 vs "control post-surgery" (day 7).

**Figure 24:** Analgesic effect of compound C17 on mechanical allodynia in rats subjected or not (sham) to SNL model. The compound was diluted in water and administered through ip route during 7 days. Treatment started at day 7 after surgery and the dose administered was 32.25 μmol/kg. Data presented as mean ± SEM (n=6). Threshold (g) = paw withdrawal threshold in grams. ***p<0.0005 vs "control before surgery" (day 0); ##p<0.005 vs "control post-surgery" (day 7).

**Figure 25:** Analgesic effect of compound C15 on thermal hypersensitivity or hyperalgesia in rats subjected or not (sham) to SNL model. The compound was diluted in DMSO and administered through ip route during 7 days. Treatment started at day 7 after surgery and the dose administered was 32.25 μmol/kg. Data presented as mean ± SEM (n=6-7). Latency(s) - paw withdrawal latency in seconds. *p<0.05, **p<0.005 and ***p<0.0005 vs "control before surgery" (day 0); #p<0.05 or ##p<0.0005 vs "post-surgery control" (day 7).

**Figure 26:** Analgesic effect of compound C15 on mechanical allodynia in rats subjected or not (sham) to SNL model. The compound was diluted in DMSO and administered through ip route during 7 days. Treatment started at day 7 after surgery and the dose administered was 32.25 μmol/kg. Data presented as mean ± SEM (n=6-7). Threshold (g) = paw withdrawal threshold in grams. **p<0.005 and ***p<0.0005 vs "control before surgery" (day 0); #p<0.05 vs "post surgery control" (day 7).

**Figure 27:** Analgesic effect of compound C3 on thermal hypersensitivity or hyperalgesia in rats subjected or not (sham) to SNL model. The compound was diluted in water and administered through ip route during 7 days. Treatment started at day 7 after surgery and the dose administered was 32.25 μmol/kg. Data presented as mean ± SEM (n=6). Latency(s) = paw withdrawal latency in seconds. **p<0.005 vs "control before surgery" (day 0); #p<0.05 and ##p<0.005 vs "Post surgery control" (day 7).

**Figure 28:** Analgesic effect of compound C3 on mechanical allodynia in rats subjected or not (sham) to SNL model. The compound was diluted in water and administered through ip route during 7 days. Treatment started at day 7 after surgery and the dose administered was 32.25 μmol/kg. Data presented as mean ± SEM (n=6-7). Threshold (g) = paw withdrawal threshold in grams. *p<0,05 or **p<0.005 and ***p<0.0005 vs "control before surgery" (day 0); #p<0.05 or ##p<0.005 vs "control post-surgery" (day 7).

**Figure 29:** Analgesic effect of compound C3 on thermal hypersensitivity or hyperalgesia in rats subjected or not (sham) to SNL model. The compound was diluted in water and administered through oral route during 7 days. Treatment started at day 7 after surgery and the dose administered was 30 mg/kg. Data presented as mean ± SEM (n=5). Latency(s) = paw withdrawal latency in seconds. *p<0.05, **p<0.005 vs "control before surgery" (day 0); ###p<0.0005 vs "control post-surgery" (day 7).

**Figure 30:** Analgesic effect of compound C3 on mechanical allodynia in rats subjected or not (sham) to SNL model. The compound was diluted in water and administered through oral route during 7 days. Treatment started at day 7 after surgery and the dose administered was 30 mg/kg. Data presented as mean ± SEM (n=6). Threshold (g) = paw withdrawal threshold in grams. **p<0.005 and ***p<0.0005 vs "control before surgery" (day 0); #p<0.05 or

##p<0.005 or ###p<0.0005 vs "control post-surgery" (day 7)

**Figure 31:** Analgesic effect of compound C3 on thermal hypersensitivity or hyperalgesia in rats subjected or not (sham) to SNL model. The compound was diluted in water and administered through oral route during 7 days. Treatment started at day 7 after surgery and the dose administered was 100 mg/kg. Data presented as mean ± SEM (n=5). Latency(s) = paw withdrawal latency in seconds. ***p<0.0005 vs "control before surgery" (day 0); #p<0.05 vs "control post surgery" (day 7).

**Figure 32:** Analgesic effect of compound C3 on mechanical allodynia in rats subjected or not (sham) to SNL model. The compound was diluted in water and administered through oral route during 7 days. Treatment started at day 7 after surgery and the dose administered was 100 mg/kg. Data presented as mean ± SEM (n=6). Threshold (g) = paw withdrawal threshold in grams. *p<0.05, ***p<0.0005 vs "control before surgery" (day 0); ##p<0.005 vs "control post-surgery" (day 7).

## Summary of the Invention

The present invention provides a heteroaromatic compound of formula (I)

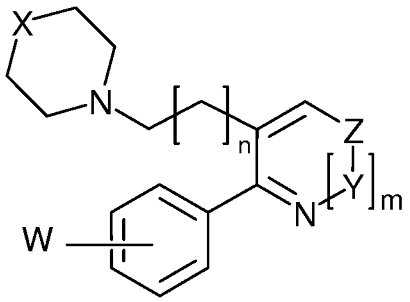

(I)

or a pharmaceutically acceptable salt thereof, wherein:

**n** is 0 or 1;

**m** is 0 or 1;

**Z** is O, -N or NH;

**Y** is C**R** or N, wherein **R** is selected from H, $C_{1-6}$-alkyl, SH, S-$C_{1-6}$-alkyl, OH, O-$C_{1-6}$-alkyl, Cl, Br, and $NH_2$.

**X** is selected from CH$\mathbf{R_1}$, C$\mathbf{R_1R_2}$, O, S, and N$\mathbf{R_3}$, wherein:

$\mathbf{R_1}$ is selected from H, $C_{1-6}$-alkyl, $C_{3-6}$-cycloalkyl, aryl, substituted-aryl, CN, OH, halogen, $CO_2R_4$, and $CONR_4R_5$;

$\mathbf{R_2}$ is selected from CN, OH, halogen, $CO_2R_4$, and $CONR_4R_5$;

$\mathbf{R_3}$ is selected from H, $C_{1-6}$-alkyl, $C_{3-6}$-cycloalkyl, aryl, substituted-aryl, and heteroaryl;

$\mathbf{R_4}$ and $\mathbf{R_5}$ are independently selected from H, $C_{1-6}$-alkyl, $C_{3-6}$-cicloalkyl, aryl, substituted aryl and heteroaryl; and

**W** is H or halogen. Preferably, the halogen is F.

The compounds of the instant invention are useful for the treatment or prevention of acute and chronic pain, particularly neuropathic pain.

The present invention also provides a process for preparing the compounds of formula (I), according to the detailed description of the invention.

The present invention also provides a pharmaceutical composition comprising at least one compound of formula (I), or a pharmaceutically acceptable salt thereof, and one or more pharmaceutically acceptable excipients.

The present invention provides a combination comprising a compound of formula (I), or a pharmaceutically

acceptable salt thereof, and one or more active compounds selected from the group consisting of NMDA receptor antagonists, LDOPA, non-steroidal anti-inflammatory drugs, opioids, antipsychotics, anticonvulsants, corticosteroids, local anesthetics, antidepressants, selective serotonin reuptake inhibitors, selective norepinephrine reuptake inhibitors, alpha-adrenergic agonists.

The present invention provides the use of a compound of formula (I), or a pharmaceutically acceptable salt thereof, in the preparation of a medicament for the treatment or prevention of acute and chronic pain, particularly neuropathic pain.

Additionally, the present invention provides a method of treating or preventing acute and chronic pain, particularly neuropathic pain, or conditions in which such pain predominates, comprising administering to a human being or animal, in need of such treatment, a compound of formula (I), or a pharmaceutically acceptable salt thereof, or a pharmaceutical composition containing the same.

The features of the present invention will become apparent from the following detailed description.

## Detailed Description of the Invention

For purposes of this invention, the terms recited in the descrition and claims are defined below:

"$C_{1-6}$-alkyl", as used herein, refers to straight or branched chain hydrocarbon radicals comprising 1 to 6 carbon atoms, such as methyl, ethyl, n-propyl, i-propyl, n-butyl and i-butyl, s-butyl and t-butyl. "Alkyl" may contain optionally, one or more unsaturated carbon-carbon bonds.

"$C_{3-6}$ cycloalkyl", as used herein, either alone as a suffix or prefix, refers to a monovalent ring-containing hydrocarbon radical comprising at least 3 up to 6 carbon atoms.

"Halogen", as used herein, refers to fluorine, chlorine, bromine or iodine atoms.

"Aryl" as used herein refers to a monovalent hydrocarbon radical having one or more polyunsaturated carbon rings having aromatic character comprising 5 up to about 14 carbon atoms. Examples are phenyl, naphthyl and anthracene.

"Heteroaryl", as used herein, refers to a heterocyclic group, mono or polycyclic, having aromatic character, comprising one or more heteroatoms independently selected from N, O or S.

"Heteroaromatic", as used herein, refers to a compound containing a 5 or 6 membered aromatic heterocyclic ring comprising two to three heteroatoms independently selected from N or O.

"Pharmaceutically acceptable salts", as used herein, refers to salts of mineral or organic acid, such as, but not limited to, sulfate, hydrochloride, dihydrochloride, phosphate, carbonate, nitrate, acetate oxalate, lactate, tartrate, bitartrate, fumarate, maleate, succinate, benzenesulfonate (besylate), and methanesulfonate (mesylate).

"Effective amount", as used herein, refers to an amount of a compound of formula (I) which provides the desired analgesic activity when administered through a specific administration route.

"Pharmaceutically acceptable excipients", as used herein, refers to ingredients compatible with the other ingredients, which have no therapeutic effect and which are not harmful to human beings or animals. They are used for the purpose of bulking-up formulations, giving shape the active pharmaceutical ingredient.

"Pharmaceutically acceptable derivatives", as used herein, refers to enantiomers, polymorphs, pseudopolimorphs (solvates and hydrates), prodrugs, such as carbonates, carbamates, citrates, bicarbonates, borates, oleate, lactate, gluconate, palmitate, palmoates, glutamates, salicylates, phosphates, phosphonates, glycosides, sulfates, sulfonates, nitrates and stearates, among others, including ethers and esters.

The term "to treat", as used herein, refers to revert, alleviate, inhibit, prevent, or diminish the progress of pain in human beings or animals. The term "treatment", as used in the present invention, refers to the act of treating as defined above as well as to prevention.

"Disorders or conditions in which pain predominates, including acute and chronic pain", as used herein, are selected from the group, but not limited to, which consists of tissue injury (soft and peripheral), such as traumatic osteoarticular and muscular lesions, traumatic nervous system lesions, including radicular or medullar lesions, and mechanical lesions, due to radiation, surgical procedures, thermal, chemical or electrical burns; pain of oncologic or non-oncologic origin with acute or chronic evolution, deriving from a somatic (for example, nociceptive), neuropathic or psychogenic mechanism; osteoarthritis, rheumatoid arthritis; musculoskeletal pain, particularly after trauma; orofacial pain, for example odontalgia; primary and secondary headaches, including migraine; abdominal pain; pain deriving from oncologic disease, including benign and malignant neoplasias, for example, cancer pain; postoperative pain; pain deriving from neurodegenerative diseases, including multiple sclerosis and amyotrophic lateral sclerosis; pain deriving from neuropathies associated with degenerative diseases, including diabetes and intervertebral disc hernia; pain deriving from metabolic alterations, including diabetes mellitus, hypotiroidism or hypertiroidism; pain deriving from repetitive strain injuries (RSI); pain deriving from congenital or genetic disorders; pain deriving from infectious or parasitic diseases,

including Hansen's disease, herpes zoster, acquired immunodeficiency syndrome (AIDS), intoxications, including heavy metals; deafferentation pain, central pain, phantom limb pain, reflex sympathetic dystrophy, postherpetic neuralgia, diabetic mononeuropathy, ischemic neuropathy, polyarteritis nodosa, post-radiotherapy pain, polyneuropathies, multiple mononeuritis, infectious and neurodegenerative myelopathies, toxic neuropathies, vasculitides and syringomyelia.

In a first embodiment, the invention provides a heteroaromatic compound of formula (I)

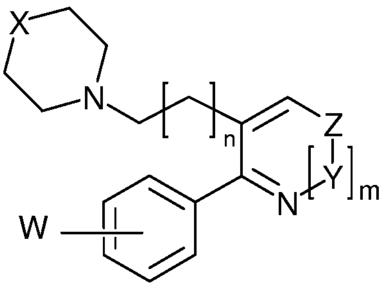

(I)

or a pharmaceutically acceptable salt thereof, wherein:

n is 0 or 1;

m is 0 or 1;

Z is O, -N or NH;

Y is CR or N, wherein R is selected from H, $C_{1-6}$-alkyl, SH, S-$C_{1-6}$-alkyl, OH, O-$C_{1-6}$-alkyl,chlorine, bromine, and $NH_2$.

X is selected from CH$\mathbf{R_1}$, C$\mathbf{R_1}\mathbf{R_2}$, O, S, and N$\mathbf{R_3}$, wherein:

$\mathbf{R_1}$ is selected from H, $C_{1-6}$-alkyl, $C_{3-6}$-cycloalkyl, aryl, substituted-aryl, CN, OH, halogen, $CO_2R_4$, and $CONR_4R_5$;

$\mathbf{R_2}$ is selected from CN, OH, halogen, $CO_2R_4$, and $CONR_4R_5$;

$\mathbf{R_3}$ is selected from H, $C_{1-6}$-alkyl, $C_{3-6}$-cycloalkyl, aryl, substituted-aryl, and heteroaryl;

$\mathbf{R_4}$ and $\mathbf{R_5}$ are independently selected from H, $C_{1-6}$-alkyl, $C_{3-6}$-cicloalkyl, aryl, substituted-aryl, and heteroaryl; and

**W** is H or halogen. Preferably, the halogen is F.

Preferably, the invention provides a heteroaromatic compound of formula (I), or a pharmaceutically acceptable salt thereof, wherein:

**n** is 1;

**m** is 1;

**Z** is -N;

**Y** is CR or N, wherein **R** is selected from H, $C_{1-6}$-alkyl, SH, S-$C_{1-6}$-alkyl, OH, O-$C_{1-6}$-alkyl, chlorine, bromine, and $NH_2$.

**X** is selected from CH$\mathbf{R_1}$, C$\mathbf{R_1}\mathbf{R_2}$, O, S, and N$\mathbf{R_3}$, wherein:

$\mathbf{R_1}$ is selected from H, $C_{1-6}$-alkyl, $C_{3-6}$-cycloalkyl, aryl, substituted-aryl, CN, OH, halogen, $CO_2R_4$, and $CONR_4R_5$;

$\mathbf{R_2}$ is selected from CN, OH, halogen, $CO_2R_4$, and $CONR_4R_5$;

$\mathbf{R_3}$ is selected from H, $C_{1-6}$-alkyl, $C_{3-6}$-cycloalkyl, aryl, substituted-aryl, and heteroaryl;

**$R_4$** and **$R_5$** are independently selected from H, $C_{1-6}$-alkyl, $C_{3-6}$-cycloalkyl, aryl, substituted-aryl, and heteroaryl; and

**W** is H or halogen.

More preferably, the invention provides a heteroaromatic compound of formula (I), or a pharmaceutically acceptable salt thereof, wherein:

**n** is 1;

**m** is 0;

**Z** is selected from O, NH, and -N;

**X** is selected from CH**$R_1$**, C**$R_1$$R_2$**, O, S and N**$R_3$**, wherein:

**$R_1$** is selected from H, $C_{1-6}$-alkyl, $C_{3-6}$-cycloalkyl, aryl, substituted-aryl, CN, OH, halogen, $CO_2R_4$, and $CONR_4R_5$;

**$R_2$** is selected from CN, OH, halogen, $CO_2R_4$, and $CONR_4R_5$;

**$R_3$** is selected from H, $C_{1-6}$-alkyl, $C_{3-6}$-cycloalkyl, aryl, substituted-aryl, and heteroaryl;

**$R_4$** and **$R_5$** are independently selected from H, $C_{1-6}$-alkyl, $C_{3-6}$-cycloalkyl, aryl, substituted-aryl, and heteroaryl; and

**W** is H or halogen.

In a second preferred embodiment, the invention provides a process for preparing a compound of formula (I)

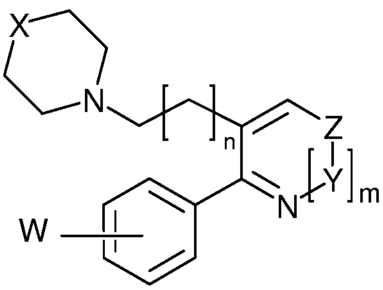

(I)

wherein when **n** is 1, the compound has the formula (II) with the proviso that when **m** is 1, **Z** is -N.

**Scheme I** below illustrates a process for obtaining a compound of formula (II).

## Scheme I

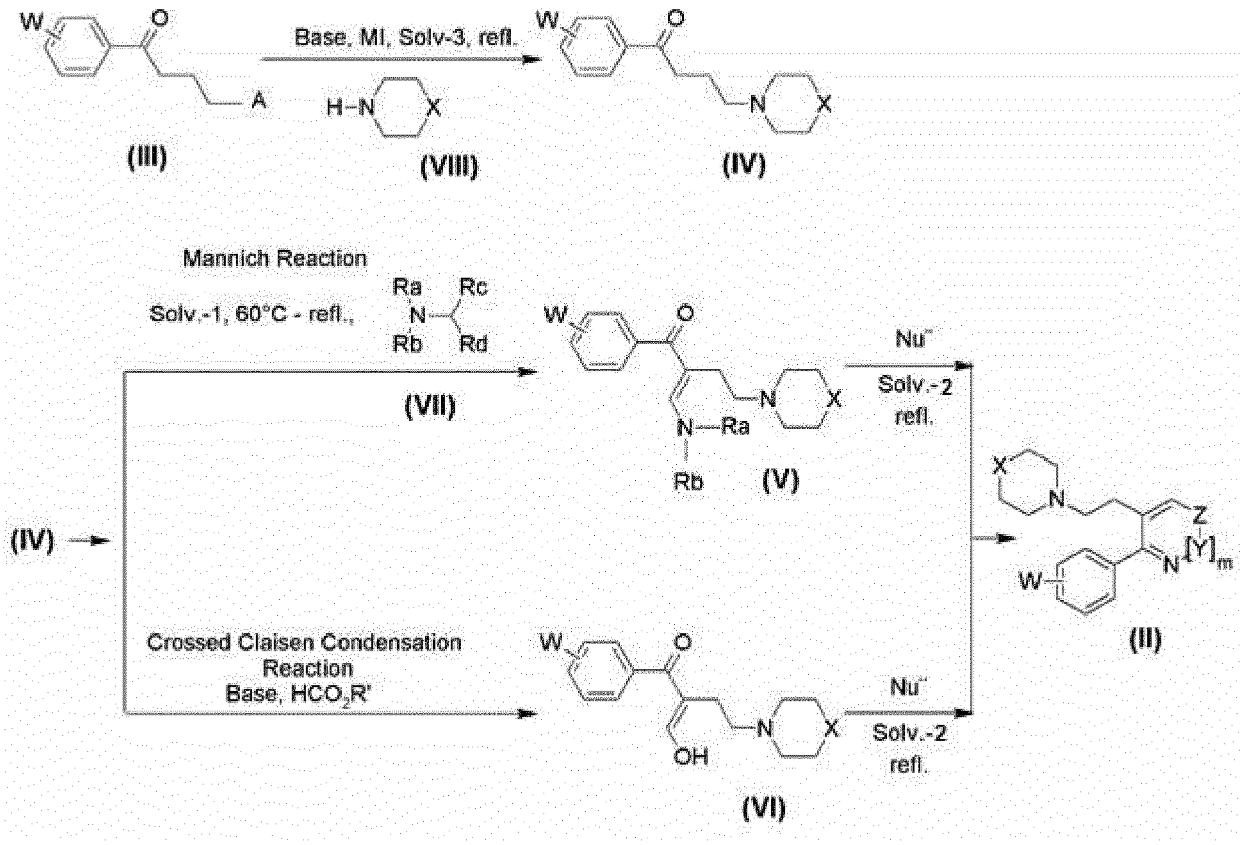


(III) (VIII) (IV) (VII) (V) (IV) (II) (VI)

In the **Scheme I,** m and the substituents Z, Y, X, and W are as defined for compound of formula (I); Ra and Rb are independently $C_{1-6}$-alkyl; Rc and Rd are independently $OC_{1-6}$-alkyl or NRaRb; R' is a $C_{1-4}$-alkyl, preferably ethyl or methyl; A represents a good leaving group, selected from the group consisting of chloride, bromide, iodide, mesyl and tosyl, preferably chloride, bromide or iodide, more preferably chloride or bromide.

In this embodiment, the invention provides a process for preparing a compound of formula **(II)** through an intermolecular cyclocondensation reaction of a ketoenamine of formula **(V),** wherein Ra and Rb are independently $C_{1-6}$-alkyl, or ketoenol of formula **(VI)** with an ambident nucleophile (or dinucleophile) selected from the group consisting of hydrazine hydrate, hydroxylamine hydrochloride, sodium azide, amidines, guanidine, O-alkylisourea and S-alkylisothiourea [El-Sayed, M.A.-A.; Abdel-Aziz, N.I.; Abdel-Aziz, A.A.-M. ; El-Azab, A.S.; Eltahir, K.E.H. Bioorg. Med Chem. 2012, 20, 3306-3316. US6806275B2].

The solvent (Solv.-2) is selected from the group consisting of $C_{1-6}$-alcohol, for example methanol, ethanol, isopropanol; ether, for example, tetrahydrofuran, 1,4-dioxane; glycol ether, such as 1,2-dimethoxyethane, diglyme [Bis(2-methoxyethyl)ether]; ketone, for example, acetone, methylethylketone, methyl isobutyl ketone; aromatic hydrocarbon, such as toluene, xylene; amide, such as N,N-dimethylformamide; acetonitrile, water, and mixtures thereof. Preferably, the solvent is $C_{1-6}$-alcohol, such as methanol, ethanol, and isopropanol.

The reaction temperature is in the range between 25°C and the reflux temperature of the solvent employed, preferably the reaction temperature is between 50°C and the reflux temperature of the solvent, most preferably it is the reflux temperature of the solvent.

This reaction may be catalyzed by acids or bases and, when non-symmetric dinucleophiles are used, its regioselectivity can be controlled by the solution pH. The base is selected from the group consisting of triethylamine diisopropylethylamine, pyridine, sodium or potassium bicarbonate, sodium or potassium carbonate, sodium or potassium methoxide, sodium or potassium ethoxide, potassium tert-butoxide, potassium tert-amyloxide, lithium diisopropylamide, sodium or lithium hexamethyldisilazide, sodium amide and sodium hydride. The acid is selected from the group consisting of hydrochloric acid, sulfuric acid, acetic acid and p-toluenesulfonic acid.

Ketoenamines (enaminones or vinylogous amides) may be prepared by Mannich reaction by methods well known in the art [Gupton, J.T. et al. Tetrahedron 2006, 62, 8243-8255. WO200705425].

The preparation of the ketoenamine or vinylogous amide intermediate of formula **(V)** comprises reacting an aminoketone of **formula (IV)**

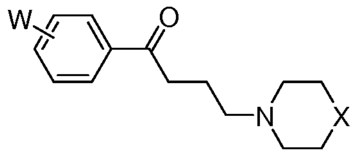

**(IV)**

or a salt thereof, with an amine of formula (VII)

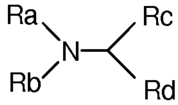

**(VII)**

The amine **(VII)** comprises a dimethylformamide dialkyl acetal, tert-butoxy-bis(dimethylamino) methane (Bredereck's reagent), methoxy-bis(dimethylamino)methane or tris(dimethylamino)methane.

This reaction can be carried out in an inert solvent (Solv-1) in the presence or absence of a base, in the presence or absence of a cyclic secondary amine, in a temperature range between 60°C and the reflux temperature of the solvent employed. The base is selected from the group consisting of triethylamine, diisopropylethylamine, pyridine, sodium or potassium carbonate, sodium or potassium bicarbonate, and sodium acetate. The cyclic amine is selected from the group consisting of pyrrolidine, piperidine, morpholine, N-methylpiperazine, and N-ethylpiperazine. The inert solvent (Solv-1) is selected from the group consisting of ether, for example, tetrahydrofuran (THF) or 1,4-dioxane; aromatic hydrocarbon, for example toluene, xylene; aliphatic hydrocarbons, such as cyclohexane; amide, for example, dimethylformamide (DMF), dimethylacetamide; acetonitrile and mixtures thereof.

The preparation of the intermediate ketoenol of formula **(VI)** by crossed Claisen condensation reaction comprises reacting a compound of formula **(IV)** with a compound of formula $HCO_2R'$, wherein R' is a $C_{1-4}$- alkyl, preferably methyl or ethyl, in the presence of a base selected from the group consisting of sodium or potassium ethoxide, sodium or potassium methoxide, potassium tert-butoxide, potassium tert-amyloxide, lithium diisopropylamide (LDA), and sodium or lithium hexamethyldisilazide.

The preparation of an aminoketone of formula **(IV)** may be carried out by aliphatic nucleophilic substitution reaction, which comprises reacting a compound of formula **(III),** where the ketone group is protected or not, in the form of enol ether or ketal, preferably in the form of a cyclic ketal type 1,3-dioxane or 1,3-dioxolane:

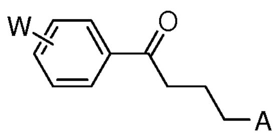

**(III)**

with a 6-membered cyclic amine of formula (VIII),

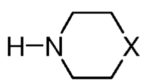

**(VIII)**

In the formula **(III), A** represents a good leaving group selected from the group consisting of chloride, bromide, iodide, mesyl and tosyl. Preferably, **A** is chloride, bromide or iodide; more preferably chloride or bromide and **X** and **W** are as defined in the Summary of the Invention. When **A** is a chlorine or bromine atom an iodide of an alkali or alkaline earth metal (MI) can be used as catalyst, where M is the metal, preferably an alkali metal, for example sodium or potassium iodide, involving a Filkenstein's reaction *in situ,* where chlorine or bromine is first replaced by iodine atom. The organic iodide formed *in situ,* in its turn, is substituted by nucleophilic amine, giving rise to the compound of formula **(IV).**

This reaction is carried out preferably at the reflux temperature of the solvent employed (Solv-3), in basic

medium. The base is selected from the group consisting of sodium or potassium carbonate, sodium or potassium bicarbonate, triethylamine, diisopropylethylamine and sodium acetate. Solvent (Solv-3) is selected from the group consisting of $C_{1-6}$-alcohol, for example methanol, ethanol, isopropanol; ether, for example, 1,4-dioxane, 1,2-dimethoxyethane, diglyme; ketone, for example acetone, methylethylketone, methyl isobutyl ketone; amide, such as, dimethylformamide, N,N-dimethylacetamide; acetonitrile and mixtures thereof; preferably methyl isobutyl ketone, 1,4-dioxane, and 1,2-dimethoxyethane.

The compounds of formula **(III),** used as raw materials, are commercially available or can be prepared by methods well known in the state of the art.

In a third embodiment, the invention provides an alternative process for the preparation of a compound of formula **(II)** according to **Scheme II.** In the **Scheme II,** m and the substituents Z, Y, X, and W are as defined for compound of formula (I); Ra and Rb are independently $C_{1-6}$-alkyl; Rc and Rd are independently $C_{1-6}$-alkyl or NRaRb; R' is a $C_{1-4}$-alkyl, preferably ethyl or methyl; **A** represents a good leaving group, selected from the group consisting of chloride, bromide, iodide, mesyl and tosyl, preferably chloride, bromide or iodide, more preferably chloride or bromide.

## Scheme II

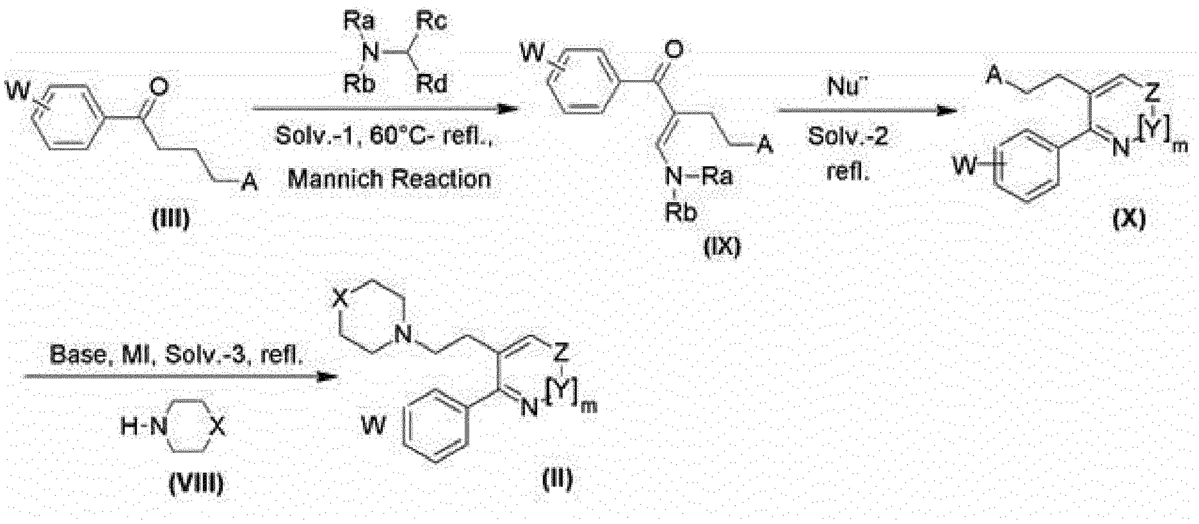


In this embodiment, the invention provides a process for preparing a compound of formula **(II)** through an aliphatic bimolecular nucleophilic substitution reaction which comprises reacting a heterocompound of formula **(X)** with a 6-membered cyclic amine of formula **(VIII),** wherein the substituents, 6-membered cyclic amine, and the reaction conditions, such as basic medium, catalyst, solvent (Solv-3), and temperatures are as defined in the description of this type of reaction according to Scheme I.

The preparation of a compound of formula **(X)** through an intermolecular cyclocondensation reaction comprises reacting a ketoenamine of formula **(IX)** with an ambident nucleophile, wherein the substituents, ambident nucleophile, and reaction conditions, such as solvent (Solv-2), and temperature, are as defined in the description of this type of reaction according to Scheme I.

This reaction may be catalyzed by acids or bases and, when non-symmetric dinucleophiles are used, its regioselectivity can be controlled by the solution pH. The base is selected from the group consisting of trimethylamine, diisopropylethylamine, pyridine, sodium or potassium bicarbonate, sodium or potassium carbonate, sodium or potassium ethoxide, sodium or potassium methoxide, potassium tert-butoxide, potassium tert-amyloxide, lithium diisopropylamide, sodium or lithium hexamethyldisilazide, sodium amide, and sodium hydride. The acid is selected from the group consisting of hydrochloric acid, sulfuric acid, acetic acid, and p-toluenesulfonic acid.

The preparation of the ketoenamine or vinylogous amide intermediate of formula **(IX),** via Mannich reaction, comprises reacting an ketone of formula **(III)** with an amine of formula **(VII),** wherein the substituents and reaction conditions, such as solvent (Solv-1) and temperature, are as defined in the description of this type of reaction according to Scheme I.

In a fourth embodiment, the invention provides an alternative process for the preparation of a compound of formula **(II),** according to **Scheme III** below, where **m, Z, Y, W** and **X** are as defined for the compound of formula (I).

**Scheme III**

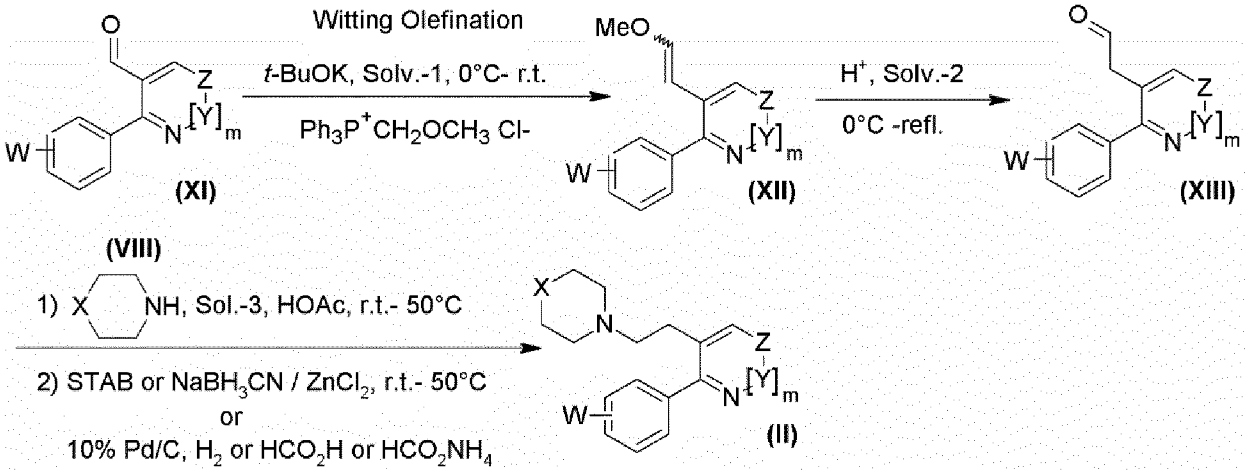


In this embodiment, the invention provides a process for preparing a compound of formula **(II)** through Borch reductive amination reaction, which comprises reacting a compound of formula **(XIII)** with a 6-membered cyclic amine of formula **(VIII),** wherein the substituents are as defined for the compound of formula **(I).**

The reaction is carried out in the presence of a reducing agent and inert solvent. The reducing agent is selected from the group consisting of sodium triacetoxyborohydride (STAB), sodium cyanoborohydride, sodium borohydride, borane, palladium on carbon, and a hydrogen source such as hydrogen, formic acid or ammonium formate [Larock, R.C., Comprehensive Organic Transformations: A Guide to Functional Group Preparations, 2nd Ed; John Wiley & Sons: New York, 1989].

Solvent (Solv-3 of Scheme III) is selected from the group consisting of hydrocarbons, such as petroleum ether, toluene or xylene; chlorinated hydrocarbons, such as 1,2-dichloroethane, chloroform or dichloromethane; alcohols such as methanol, ethanol, isopropanol, n-propanol, n-butanol; ethers such as diethyl ether, diisopropyl ether, tetrahidrofunane (THF) or 1,4-dioxane; glycol ethers such as 1,2-dimethoxyethane, diglyme; amides such as acetamide, N,N-dimethylformamide (DMF) or N-dimethylacetamide; nitriles, such as acetonitrile; esters such as ethyl acetate, isopropyl acetate; and mixtures thereof.

The carbaldehyde of formula **(XIII)** can be obtained through two steps, which comprise: 1st step - homologation, via the Wittig olefination, of a carbaldehyde of formula **(XI)** with methoxymethyltriphenylphosphonium chloride, as the ylide precursor, in the presence of potassium tert-butoxide and solvent (Solv-1) selected from the group consisting of THF, dioxane, 1,2-dimethoxyethane (1,2-DME), ether, and diglyme; 2nd step - a methylenolether of formula **(XII)** obtained in the 1st step is hydrolysed in acidic medium, in the presence of solvent (Solv-2), to produce the carbaldehyde **(XIII).** The acid is selected from the group consisting of sulfuric acid, hydrochloric acid, and p-toluenesulfonic acid. The solvent is selected from the group consisting of toluene, ether, 1,2-DME, diglyme, THF, 1,4-dioxane, and acetone [Miao, L., Shu, H., Noble, A.R., Fournet, S.P., Edwin D., Stevens, E.D., Trudell, M.L. ARKIVOC 2010, iv, 6-14. Stehl, A., Seitz, G., Schulz, K. Tetrahedron 2002, 58, 1343-1354. Springer, D.M. et al. Bioorg. Med Chem. 2000, 8, 1087-1109].

In a fifth embodiment, the carbaldehyde of formula **(XI)** can be prepared, generally, according to **Scheme IV** below, wherein **m, Z, Y, W** and **X** are as defined for the compound of formula (I); Ra and Rb are independently $C_{1-6}$-alkyl; Rc and Rd are independently $C_{1-6}$-alkyl, or NRaRb; $R_6$ is $C_{1-6}$-alkyl.

## Scheme IV

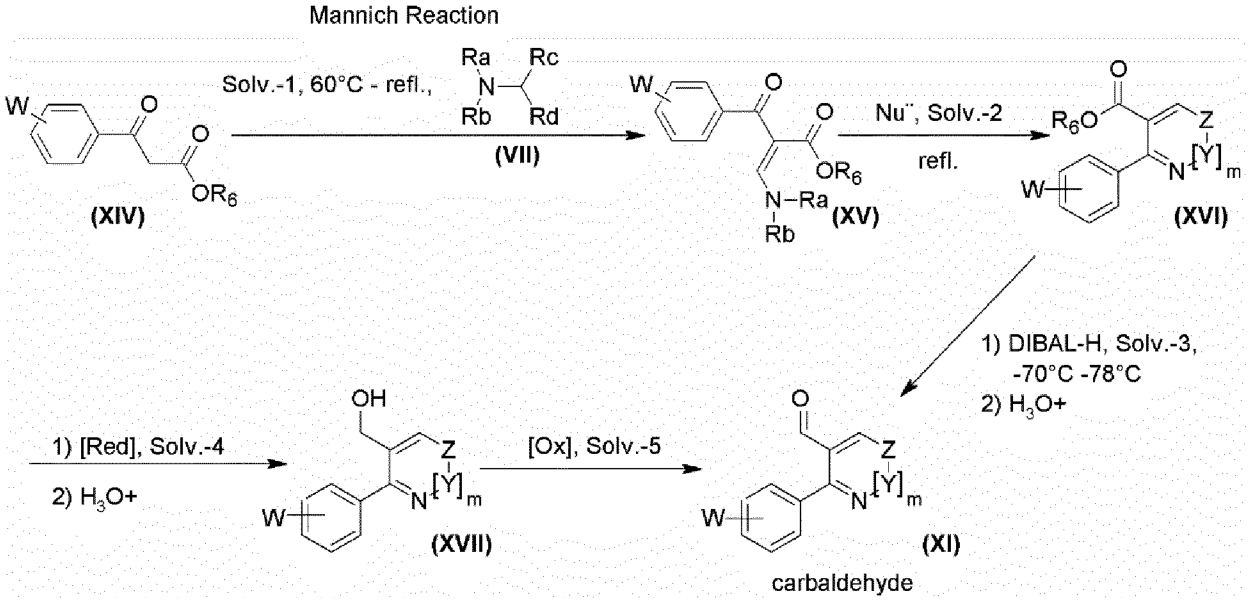


(XIV) (XV) (XVI) (XVII) (XI) carbaldehyde

The preparation of the vinylogous amide of formula **(XV)** through Mannich reaction comprises reacting an aryl-*β*-ketoester of formula **(XIV),** with an amine of formula **(VII),** wherein the substituents are as previously defined [Holub, J.M.; Burnham, B.S. et al. Molecules 2004, 9, 135-157. WO2005075458].

This reaction is carried out at a temperature in the range between 60°C and the reflux temperature of the solvent employed, in the presence or absence of a base. The base is selected from the group consisting of triethylamine, diisopropylethylamine, pyridine, sodium or potassium carbonate, sodium or potassium bicarbonate, and sodium acetate. Solvent (Solv-1) is selected from the group consisting of tetrahydrofuran, 1,4-dioxane, 1,2-dimethoxyethane, diglyme, acetonitrile, N,N-dimethylformamide, N,N-dimethylacetamide, toluene, xylene, cyclohexane, and mixtures thereof.

The preparation of an ester of formula **(XVI),** through an intermolecular cyclocondensation reaction, comprises reacting a vinylogous amide of formula **(XV)** with an ambident nucleophile selected from the group consisting of hydrazine hydrate, hydroxylamine hydrochloride, sodium azide, guanidine, amidine, *O*-alkylisourea, and S-alkylisothiourea.

This reaction can be carried out at temperatures from 25°C to the reflux temperature of the solvent employed, preferably between 50°C and the reflux temperature of the solvent, more preferably at the reflux temperature of the solvent. The solvent (Solv-2) is selected from the group consisting of methanol, ethanol, isopropanol, acetonitrile, tetrahydrofuran, dioxane, 1,2-dimethoxyethane, diglyme, toluene, acetone, methylethylketone, methyl isobutyl ketone, N,N-dimethylformamide, water, and mixture thereof. Preferably, the solvent is methanol, ethanol or isopropanol.

This reaction may be catalyzed by acids or bases and, when non-symmetric dinucleophiles are used, its regioselectivity can be controlled by the solution pH. The base is selected from the group consisting of triethylamine diisopropylethylamine, pyridine, sodium or potassium bicarbonate, sodium or potassium carbonate, sodium or potassium methoxide, sodium or potassium ethoxide, potassium tert-butoxide, potassium tert-amyloxide, lithium diisopropylamide, sodium or lithium hexamethyldisilazide, sodium amide, and sodium hydride. The acid is selected from the group consisting of hydrochloric acid, sulfuric acid, acetic acid and p-toluenesulfonic acid.

The ester of formula **(XVI)** may be reduced directly to an carbaldehyde **(XI)** using a reducing agent including diisobutylaluminum hydride (DIBAL-H), in a solvent (Solv-3) selected from the group consisting of dichloromethane, toluene, ether, cyclohexane, and hexane, in a temperature in the range between -70°C and -78°C.

Alternatively, the ester of formula **(XVI)** can be totally reduced to the corresponding alcohol of formula **(XVII)** using a reducing agent known in the art [Larock, R.C., Comprehensive Organic Transformations: A Guide to Functional Group Preparations, 2nd Ed; John Wiley & Sons: New York, 1989], for example, lithium and aluminum hydride, super hydride (lithium triethylborohydride), diisobutylaluminum hydride (DIBAL-H) or lithium borohydride, in the presence of an anhydrous inert solvent (Solv-4) selected from group consisting of THF, ether, 1,4-dioxane, 1,2-dimethoxyethane, dichloromethane, and toluene, in a temperature between 20°C and 85°C. Subsequently, the alcohol **(XVII)** can be oxidized to the corresponding carbaldehyde **(XI)** with a oxidizing agent selected from the group consisting of manganese dioxide, o-iodoxybenzoic acid (IBX), pyridinium chlorochromate (PCC), pyridinium dichromate (PDC), Dess-Martin periodinane (DMP), tetrapropylammonium perruthenate (TPAP), Swern reagent, among others, in the presence of a solvent selected from the group consisting of chloroform, dichloromethane, toluene, acetonitrile, ether, tetrahydrofuran, 1,4-dioxane, and mixture thereof.

*β*-ketoesters of the formula **(XIV)** are commercially available or can be synthesized with excellent yields by methods known in the art [US20100249094. Jiang, Y. et al. Angew. Chem. Int Ed, 2011, 50, 7304-7307]. For example, **Scheme V** below, wherein W is H or halogen and $R_6$ is $C_{1-6}$-alkyl, depicts the preparation of a *β*-ketoester of formula **(XIV)** through crossed Claisen condensation reaction, comprising reacting an acetophenone of formula **(XVIII)** with a dialkyl carbonate, preferably methyl or ethyl carbonate.

**Scheme V**

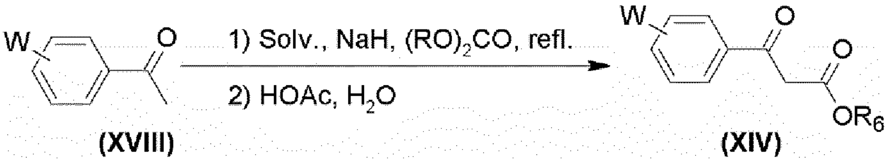


The reaction is carried out in the presence of a base selected from the group consisting of sodium hydride, sodium or potassium ethoxide, sodium or potassium methoxide, potassium tert-butoxide, potassium tert-amyloxide, lithium diisopropylamide (LDA), and sodium or lithium hexamethyldisilazide, preferably sodium hydride, and potassium tert-butoxide; and in the presence of an inert solvent selected from the group consisting of toluene, xylene, tetrahydrofuran, and 1,4-dioxane.

The carbaldehyde of formula **(XI)** may, alternatively, be prepared according to **Scheme VI** below. In the **Scheme VI,** m and the substituents Z, Y, X, and W are as defined for the compound of formula **(I);** Ra and Rb are independently $C_{1-6}$-alkyl; Rc and Rd are independently $C_{1-6}$-alkyl or NRaRb;

**Scheme VI**

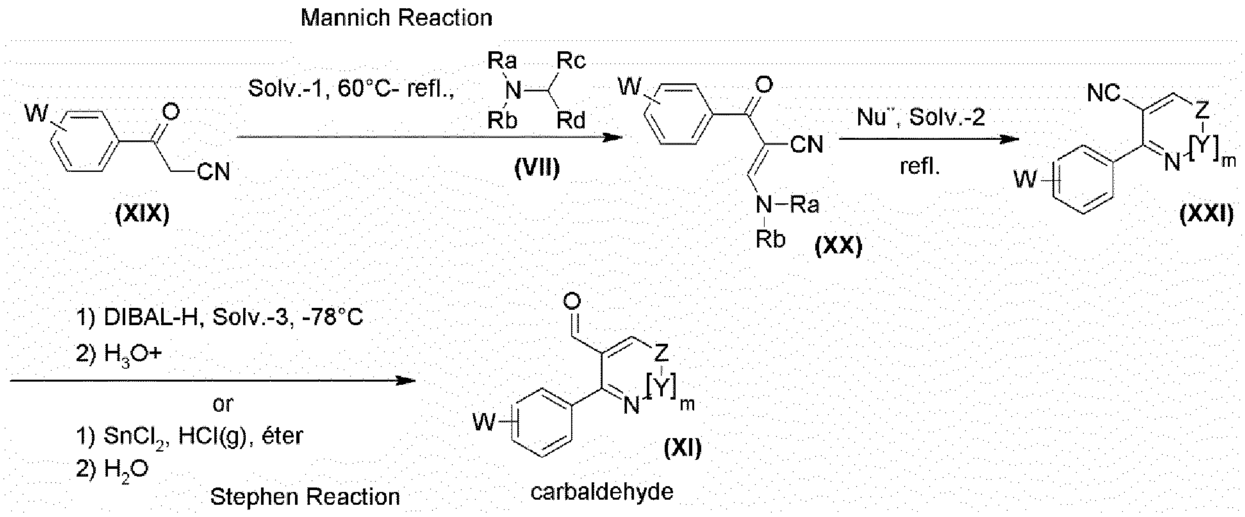


The preparation of vinylogous amide of formula **(XX)** through Mannich reaction comprises reacting a 2-cyanoacetophenone of formula **(XIX)** with an amine of formula **(VII)** selected from the group consisting of dimethylformamide dialkyldimethylacetal, tert-butoxy bis(dimethylamino)methane (Bredereck's reagent), methoxy-bis(dimethylamino)methane, and tris(dimethylamino) methane, in the presence or absence of a base, in a temperature in the range between 60°C and the reflux temperature of the solvent employed.

In this reaction, the base is selected from the group consisting of triethylamine, diisopropylethylamine, pyridine, sodium or potassium carbonate, sodium or potassium bicarbonate, and sodium acetate. The inert solvent (Solv-1) is selected from the group consisting of ether, for example, tetrahydrofuran (THF) or 1,4-dioxane; aromatic hydrocarbon, for example toluene, xylene; aliphatic hydrocarbons, such as cyclohexane; amide, e.g., N,N-dimethylformamide (DMF), N,N-dimethylacetamide; acetonitrile; and mixtures thereof.

The preparation of heteroarylnitrile of formula (XXI) through an intermolecular cyclocondensation reaction comprises reacting a vinylogous amide of formula **(XX)** with an ambident nucleophile selected from the group consisting of hydrazine hydrate, hydroxylamine hydrochloride, sodium azide, guanidine, amidines, *O*-alkylisourea and S-alkylisothiourea.

This reaction can be performed in temperatures from 25°C to the reflux temperature of the solvent employed, preferably between 50°C and the reflux temperature of the solvent, more preferably at the reflux temperature of the solvent. Solvent (Solv-2) is selected from the group consisting of methanol, ethanol, isopropanol, acetonitrile, tetrahydrofuran, 1,4-dioxane, 1,2-dimethoxyethane, diglyme, toluene, acetone, methylethylketone, methyl isobutyl ketone, N,N-dimethylformamide, N,N-dimethylacetamide, water, and mixture thereof. Preferably, the solvent is methanol, ethanol or isopropanol.

This reaction may be catalyzed by acids or bases and, when non-symmetric dinucleophiles are used, its regioselectivity can be controlled by the solution pH. The base is selected from the group consisting of triethylamine, diisopropylethylamine, pyridine, sodium or potassium bicarbonate, sodium or potassium carbonate, sodium or potassium methoxide, sodium or potassium ethoxide, potassium tert-butoxide, potassium tert-amyloxide, lithium diisopropylamide, sodium or lithium hexamethyldisilazide, sodium amide, and sodium hydride. The acid is selected from the group consisting of hydrochloric acid, sulfuric acid, acetic acid, and p-toluenesulfonic acid.

The heteroaryl nitriles **(XXI)** may be reduced directly to carbaldehydes **(XI)** using diisobutylaluminum hydride (DIBAL-H) in toluene, ether or hexane, in a temperature of -78°C; or using the Stephen reaction conditions: anhydrous tin (II) chloride with hydrochloric acid gas to generate the aldimine intermediate, followed by aldimida chloride hydrolysis with water.

The starting materials 2-cyanoacetophenones of formula **(XIX)** are known or can be prepared according to conventional procedures in the art, starting from known compounds [EP1316546, WO2011076744].

In the reactions described for carbaldehydes **(XI)** and **(XIII)** synthesis, when m is zero and Z is NH, protection of certain reactive functional groups, for example, the amino group may be required, as shown in Scheme VII for the preparation of a compound of formula **(XXVI).** A large variability of protection groups, as well as the appropriated reaction to their introduction and removal are described in the literature [Greene, T. W; Wuts, P.G.M. Protetive Groups in Organic Synthesis, 3rd ed.; John Wiley & Sons: New York, 1991].

**Scheme VII** below illustrates a process for obtaining a compound of formula **(XXVI).** In the **Scheme VII,** W and X are as defined for the compound of formula (I).

Scheme VII

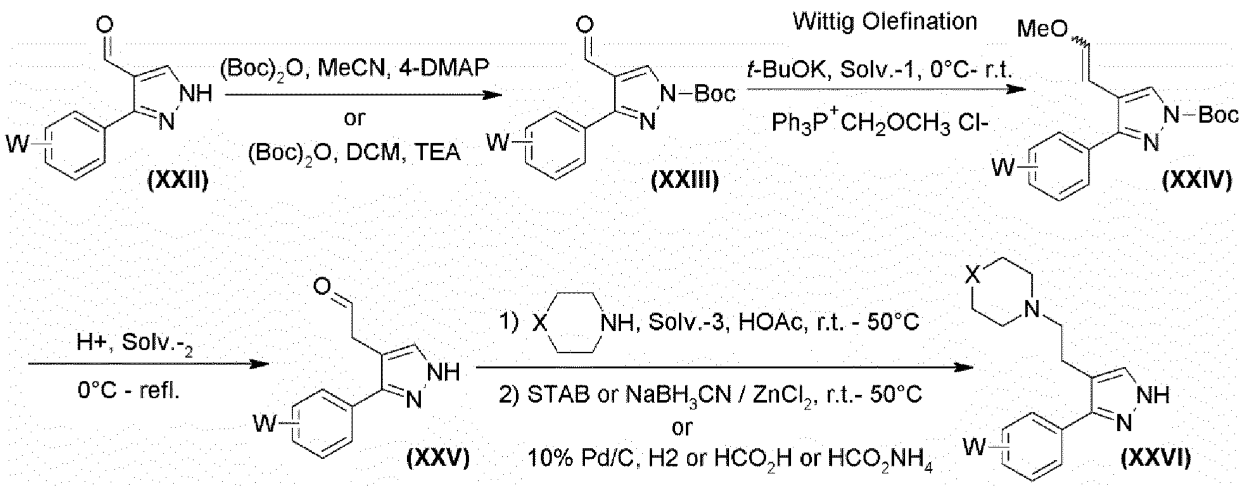


The amino group of the pyrazole ring of the pyrazole-carbaldehyde of formula **(XXII)** is preferably protected with, but not limited to, di-tert-butyl dicarbonate, before performing the Wittig olefination, in analogy to the procedure described in Scheme III. Different protecting groups known in the art may be used, with the characteristic that they can be removed easily and without unwanted reactions occur. Different alternatives could be easily selected by a person skilled in the art.

The process for preparing a compound of formula **(XXVI)** is carried out through Borch reductive amination reaction, comprising reacting a compound of formula **(XXV)** with a 6-membered cyclic amine, in which the substituents are X and W are as defined for the compound of formula (I).

The reaction is carried out in the presence of a reducing agent and an inert solvent. The reducing agent is selected from the group consisting of sodium triacetoxyborohydride (STAB), sodium cyanoborohydride, sodium borohydride, borane, palladium on carbon, and a hydrogen source such as hydrogen, formic acid, and ammonium formate.

Solvent (Solv-3) is selected from the group consisting of hexane, petroleum ether, benzene, toluene or xylene; chlorinated hydrocarbons, such as trichlorethylene, 1,2-dichloroethane, tetrachloromethane, chloroform, or dichloromethane; alcohols such as methanol, ethanol, isopropanol, n-propanol, n-butanol or tert-butanol; ethers such as diethyl ether, diisopropyl ether, tetrahydrofuran (THF) or dioxane; glycol ethers, such as ethylene glycol monomethyl, or monoethyl ether, or ethylene glycol dimethyl ether (diglyme); ketones, such as acetone or butanone; amides such as acetamide, dimethylacetamide or dimethylformamide (DMF); nitriles, such as acetonitrile; sulfoxides such as dimethyl sulfoxide (DMSO); nitro compounds such as nitromethane or nitrobenzene; esters such as ethyl acetate; and mixtures thereof.

The carbaldehyde of formula **(XXV)** can be obtained through two steps, which comprise: 1st step - homologation, via the Wittig olefination, of a carbaldehyde of formula **(XXIII)** with (methoxymethyl)triphenylphosphonium chloride, as the ylide precursor, in the presence of potassium tert-butoxide, and solvent (Solv-1) selected from the group consisting of THF, dioxane, 1,2-dimethoxyethane (1,2-DME), ether, and diglyme; 2nd step - a methylenolether of formula **(XXIV),** obtained in the 1st step, is hydrolysed in acidic medium, in the presence of solvent (Solv-2), when occurs the nitrogen deprotection and the carbaldehyde **(XXV)** is produced. The acid is selected from the group consisting of sulfuric acid, hydrochloric acid, and p-toluenesulfonic acid. The solvent is selected from the group consisting of toluene, ether, acetone, THF, dioxane, 1,2-DME, ether, and diglyme.

The specific preparation of pyrazole-carbaldehyde **(XXII)** can be carried in a simple manner, according to **Scheme VIII** below, in which W is as defined for the compound of formula (I).

## Scheme VIII

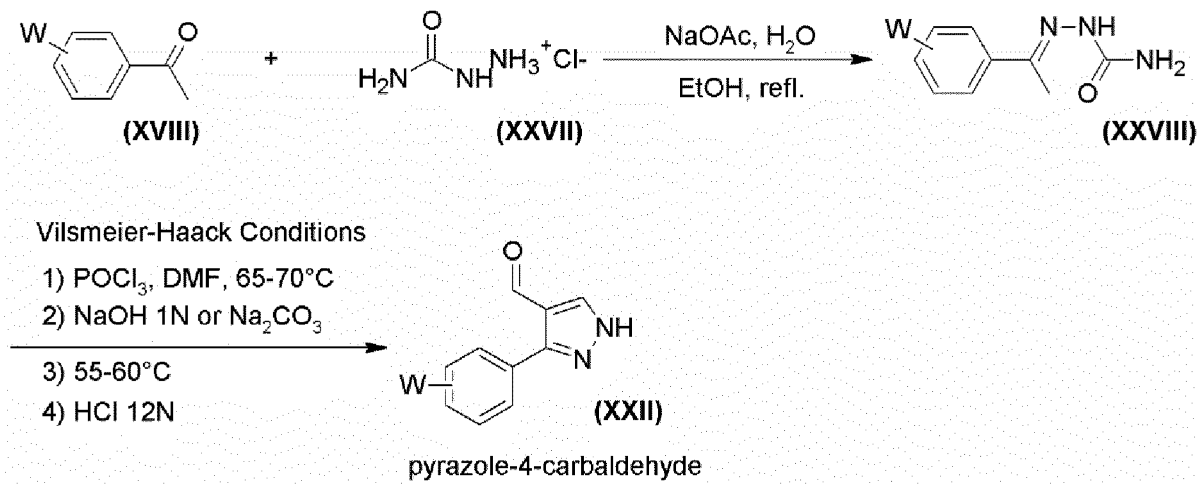


**(XVIII)** **(XXVII)** **(XXVIII)**

**(XXII)**

pyrazole-4-carbaldehyde

The preparation of the pyrazole-carbaldehyde of formula **(XXII)** comprises reacting an acetophenone of formula **(XVIII)** with a semicarbazide hydrochloride **(XXVII)** in a hydroalcoholic system in the presence of a weak base, for example sodium acetate, to give rise to the corresponding semicarbazone of formula **(XXVIII).** The obtained semicarbazone is subjected to Vilsmeier-Haack formylation conditions ($POCl_3$ and DMF) followed by the addition of an appropriate base, selected from sodium or potassium hydroxide, sodium or potassium carbonate, yielding a carbaldehyde of formula **(XXII)** [Lejbedev, A.V.; Lebedeva, A.B.; Sheludyakov, V.D.; Kovaleva, E.A.; Ustinova, O.L.; Kozhevnikov, I.B.; Russ, J. Gen. Chem. 2005, 75, 782. Malladi, S.; Isloor, A.M.; Peethambar, S.K.; Ganesh, B.M.; Goud, P.S. Der Pharma Chemica 2012, 4, 43-45. WO2008011611].

In a further embodiment, the invention provides a process for preparing a compound of formula (I)

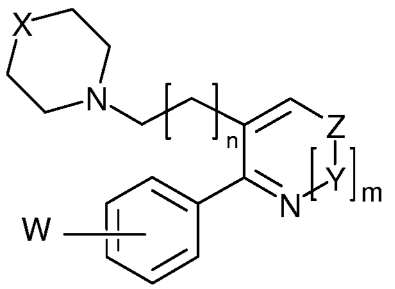

(I)

wherein when **n** is zero, the compound has the formula **(XXIX).** The **Scheme IX** below ilustrates this reaction. In the Scheme IX, m and the substituents Z, Y, X and W are as defined for the compound of formula (I).

**Scheme IX**

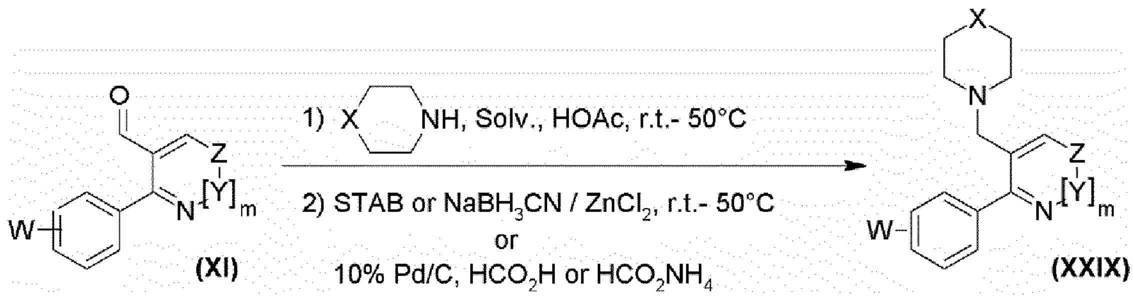


A compound of formula **(XXIX)** can be prepared through Borch reductive amination reaction, reacting a carbaldehyde of formula **(XI),** prepared as previously described, with a 6-membered aliphatic cyclic amine as defined above, in the presence of suitable reducing agent, selected from the group consisting of sodium triacetoxyborohydride (STAB), sodium cyanoborohydride, sodium borohydride, borane, palladium on carbon, and a hydrogen source selected from hydrogen, formic acid or ammonium formate, in an inert solvent selected from the group consisting of hydrocarbons, such as petroleum ether, toluene or xylene; chlorinated hydrocarbons, such as 1,2-dichloroethane, chloroform, or dichloromethane; alcohols such as methanol, ethanol, isopropanol, n-propanol, n-butanol; ethers such as diethyl ether, diisopropyl ether, tetrahydrofunan (THF) or 1,4-dioxane; glycol ethers, such as 1,2-dimethoxyethane, diglyme; amides such as acetamide, N,N-dimethylformamide (DMF), N,N-dimethylacetamide; nitriles, such as acetonitrile; esters such as ethyl acetate, isopropyl acetate; and mixtures thereof.

The pharmaceutically acceptable salts of the compounds of this invention can be prepared by addition of a suitable organic or inorganic acid selected from the group consisting of acetic acid, oxalic acid, lactic acid, fumaric acid, maleic acid, citric acid, tartaric acid, methanesulfonic acid, hydrochloric acid, sulfuric acid and phosphoric acid. Alternatively, they can be prepared using salts of the ambident nucleophiles acids selected from the group consisting of hydroxylamine hydrochloride, hydroxylamine sulfate, hydrazine hydrochloride, hydrazine sulfate, formamidine hydrochloride, and formamidine acetate.

The compounds of the present invention as free base may be obtained directly during the process or, alternatively, can be prepared from the corresponding salts by addition of a suitable base, selected from the group consisting of ammonium hydroxide, sodium or potassium hydroxide, sodium or potassium bicarbonate, sodium or potassium carbonate.

The compounds of the present invention can be prepared, or formed during the synthesis process as a pure polymorphs, pseudopolimorfos (solvates and hydrates), or mixture thereof. The different polymorphs and pseudopolimorfos can be prepared by crystallization from suitable solvents, selected from the group consisting of methanol, ethanol, isopropanol, acetone, tetrahydrofuran, water, and mixtures thereof.

The compounds of this invention may also be prepared or formed during the synthesis process, as individual isomers, mixtures of isomers or tautomers.

Thus, included in the scope of the present invention are compounds of formula (I), prodrugs, polymorphs, hydrates, solvates, tautomers, individual isomers, mixtures of isomers and pharmaceutically acceptable salts thereof.

Variations of the reactions shown, such as using ultrasonic energy, microwave or microreactors, in the presence or absence of solvents or diluents, in the presence or absence of catalysts, in the presence or absence of dehydrating agents, at normal pressure or under pressure in closed reactor; at reduced, normal or elevated temperatures; under

inert atmosphere or not; are envisaged within the scope of the invention.

The person skilled in the art will understand that the described transformations are only representative of methods for preparation of the compounds of the present invention, and that other methods known in the prior art can be similarly used.

In a further embodiment, the present invention provides a pharmaceutical composition comprising an effective amount of at least one compound of formula (I) or a pharmaceutically acceptable salt thereof, and one or more pharmaceutically acceptable excipients. Such composition is useful for treating or preventing acute and chronic pain, particularly neuropathic pain.

The present invention provides a pharmaceutical composition comprising from 0.1% to 99% w/w of a compound of formula (I) or, a pharmaceutically acceptable salt thereof.

According to the present invention, administration of a pharmaceutical composition containing a compound of formula (I), or a pharmaceutically acceptable salt thereof, can be carried out by the following administration routes: oral, sublingual, nasal, rectal, intragengival, parenteral (such as intravenous, intramuscular, intraarticular, subcutaneous), dermal (such as transdermal patch), inhalatory, transdermal, topical, and spinal (subarachnoid, intrathecal, and epidural), not limited to these. Preferred administration routes are oral and parenteral.

Pharmaceutically acceptable excipients are selected according to composition of the final presentation of the present invention, which may be in the form of capsules or tablets, oral solutions, elixirs, suspensions, powders, granules, syrups, solutions for nasal administration, injectable solutions, suppositories, aerosols lotions, creams, gels or ointments.

Conventionally, the most commonly used pharmaceutical excipients are diluents, dispersants, binders, lubricants, disintegrants, suspending agents, dyes, sweeteners, flavoring agents, humectants, preservatives, stabilizers, flavor enhancer, antioxidants, plasticizers, buffers, pH modifiers, emulsifiers, surfactants, tonicity agents, smoothing agents, chelating agents, among others.

Solid dosage forms may also contain special coatings, such as coatings to mask unpleasant taste or odor; enteric coatings, in order to protect from degrading in the stomach and allowing the release directly into the duodenum; or still release modifiers coatings, promoting the delayed and/or optimized release of the drug.

Methods of preparing various pharmaceutical compositions are well known (see Remington's Pharmaceutical Sciences, 21st ed. 2011), or will be recognized in the light of the present invention, by the art specialist in pharmaceutical technology.

The present invention provides a combination comprising two or more compounds of formula (I), or derivatives or pharmaceutically acceptable salts thereof. Such combination is useful for treating or preventing acute and chronic pain, particularly neuropathic pain.

The present invention provides a combination comprising a compound of formula (I), or a pharmaceutically acceptable salt thereof, and one or more active compounds selected from the group consisting of NMDA receptor antagonists, LDOPA, non steroidal anti-inflammatory drugs, opioids, antipsychotics, anticonvulsants, corticosteroids, local anesthetics, antidepressants, selective serotonin reuptake inhibitors, selective norepinephrine reuptake inhibitors, alpha-adrenergic agonists. Such combination is useful for treating or preventing acute and chronic pain, especially neuropathic pain.

Examples of active compounds that can be used in combination with the compound of formula (I) of the present invention include:

NMDA receptor antagonists, such as: memantine, ketamine, or budipine;

Non steroidal anti-inflammatory drugs such as: acetaminophen, dipyrone, ketoprofen, ketorolac, diclofenac, ibuprofen, indomethacin, naproxen, piroxicam, aspirin, meloxicam, or nimesulide;

Opioids, such as: morphine, codeine, methadone, tramadol, oxycodone, fentanyl, remifentanil, sufentanil, hydrocodone, levorphanol, or tapentadol;

Antipsychotics, such as: chlorpromazine, fluphenazine, clozapine, haloperidol, olanzapine, quetiapine, or risperidone;

Anticonvulsants such as: carbamazepine, gabapentin, primidone, topiramate, valproate, oxcarbazepine, phenobarbital, levetiracetam; ethosuximide, lamotrigine, or tiagabine;

Corticosteroids such as: betamethasone, dexamethasone, methylprednisolone, or prednisolone

Local anesthetics, such as: lidocaine, bupivacaine, prilocaine, or ropivacaine;

Antidepressants, such as bupropion, mirtazapine, nefazodone, trazodone, or venlafaxine;

Selective serotonin reuptake inhibitors such as: citalopram, escitalopram oxalate, fluoxetine, paroxetine, or sertraline;

Selective norepinephrine reuptake inhibitors, such as: nortriptyline, amitriptyline, clomipramine, or imiprapina; and

Alpha-adrenergic agonists, such as: clonidine, methoxamine, phenylephrine, metaraminol, midodrine, apraclonidine, guanfacine, guanabenz, methyldopa, tizanidine, amphetamine, methamphetamine, methylphenidate, or ephedrine.

The present invention provides the **use** of a compound of formula (I), or a pharmaceutically acceptable salt thereof, in the preparation of a medicament for treating or preventing acute and chronic pain, particularly neuropathic pain.

Additionally, the present invention provides a **method** for treating or preventing acute and chronic pain, particularly neuropathic pain, or conditions in which pain predominates such comprising administering to a human being or animal in need of treatment thereof, a compound of formula (I), or a pharmaceutically acceptable salt thereof, or a pharmaceutical composition containing the same.

The compounds of the present invention were subjected to *in vivo* assays whose results demonstrated significant analgesic activity, without showing adverse events related to depression of the central nervous system (blurred vision, dizziness, headache, confusion, nausea, etc.).

The results obtained for analgesia in response to both acute and chronic pain, in particular neuropathic pain, were compared with conventional drugs used to treat pain: morphine sulfate and amitriptyline hydrochloride, at a dose of 32.25 umol/kg, and the compounds of the present invention showed superior efficacy.

The in vivo assays methods used to evaluate the analgesic activity of the compounds of the present invention were the hot plate test (Hot Plate Model), specific for acute pain, and Chronic Constrinction Injury (CCI) and the Spinal Nerve Ligation (SNL) assays, specific for chronic pain, particularly neuropathic pain.

The assessment of potential adverse effects related to depression of the central nervous system was performed using the RotaRod Performance Test.

**Pharmacological Properties Evalueted *in vivo***

**<u>Hot Plate Model - assay for acute pain</u>**

The antinociceptive activity (AA) of the present invention selected compounds (C3, C9, C11, C12, C13, C15 and C17) was investigated in hot plate model [Hot Plate Model LE7406, Scientific Inc.] described by Kuraishi et *al.* [Kuraishi, Y., Harada, Y., Aratani, S., Satoh, M., Takagi, H. Brain Research 1983.273, 245-2523]. This method is valid to evaluate the analgesic activity of substances acting on the central nervous system. Swiss mice weighing 20-25 g were placed on a metal plate heated up to 52°C. The animals were withdrawn from the hot plate at the time they lead their forepaws toward the mouth to lick, being this an indicative of pain second Le Bars et *al.* [Le Bars, D., Gozariu, M., Cadden, S.W. Animal models of nociception. Pharmacol. Rev. 2001, 53 (4), 597-652]. In this way, it was analyzed how long the animal, treated or not (control) with the analgesic substance, remained on the plate at 52°C; the longest residence time of an animal on the plate was 35 seconds. This time limit (or cut off) (35 s) was determined by the average of three measurements of latency control values, obtained for each animal multiplied by three, to prevent any kind of tissue injury on the footpad of animals's paw due to long permanence on the heated surface. The protocol consisted in the intraperitoneal (ip) injection of the selected compounds of the present invention at a dose of 32.25 μmol/kg. The antinociceptive activity was evaluated at intervals of 10 minutes until effect completely disappear and, compared to morphine sulfate (MW = 758.3 g/mol) at the same dose of 32.25 μmoles/kg (12.2 mg/kg). The investigated parameter is the latency of reflex to paw withdrawal in response to thermal stimulus. The results were expressed as percentage of AA, which was calculated according to Equation 1 for each experiment.

$$\mathbf{AA} = \frac{[\text{observed latency (post-treatment)} - \text{latency control}] \times 100}{[\text{Cut off} - \text{latency control}]} \qquad \textbf{Equation 1}$$

The selected compounds of the present invention were tested in the model of the Hot Plate and the results are shown in Figures 1 (C3, C9, C11 and C12) and 2 (C13, C15 and C17). According to the obtained results, the tested compounds exhibit antinociceptive activity of intensity and kinetics similar to morphine sulfate, but with no side effects of conventional morphine.

## Chronic pain assays- neuropathic pain

### 1) Model of Chronic Constrictive Injury

The chronic constrictive injury (CCI) was induced by sciatic nerve ligation according to the method described by Bennett and Xie [Bennett. G. J.; Xie, Y.K. Pain 1998, 33: 87-107]. Male Wistar rats, weighing 180-240 g, were anesthetized with a mixture of ketamine and xylazine (60 mg/kg and 5 mg/kg, respectively), intraperitoneally. Through an incision of 0.5 cm (parallel and spaced about 10 mm from the left femur) the sciatic nerve was identified. With a thin glass rod, a small segment of sciatic nerve was exposed and four loosely ligatures were placed around the sciatic nerve spaced 1 mm each other using a 3-0 silk suture thread. After the procedure, the skin was sutured and a topical antiseptic was applied.

### a) Thermal hyperalgesia assessment - the paw withdrawal test

To assess animal behavior in response to hyperalgesic phenomenon resulting from sciatic nerve chronic constrictive injury, the model described by Hargreaves and Dubner was used [Hargreaves K., Dubner R., Brown F., Flores C., Joris J. Pain 1988, 32 (1): 77-88]. In this regard, paw withdrawal in response to a painful phenomenon was performed. This is a nociceptive test which involves a peripheral short thermal stimulus induced by the radiant heat of an incandescent lamp bulb. Several stimuli that can be used in tests of nociception, among them, the heat is more selective since it stimulates the skin receptors and thus specific categories of peripheral axons, including thermosensitive and nociceptive fibers can be excited. Radiant heat has an advantage over the other modes of thermal stimulation in that it produces no tactile stimulus. [Le Bars D., Gozariu M., Cadden S.W. Pharmacology Reviews, 2001, 53 (4), 597-652]. In this assay, a plantar test analgesimeter (ITC Life Science Inc, USA) was used.

Animals were placed in transparent acrylic boxes with tempered glass pane floor for 40 minutes to acclimate. After this period, the radiant heat was applied to the footpads of animals's hindpaws until they make the movement of paw withdrawal. The paw withdrawal latency was defined as the time recorded from the beginning of the radiant heat stimulus and the paw withdrawal. For each paw, three latency records were made, and the average value was used to define the paw withdrawal time control The maximum time that each animal was exposed to the radiant heat source (cut-off) was defined as three times the paw withdrawal time control, to avoid tissue injuries in animals's paws.

The day before surgery, rats, defined as "control before surgery", were placed in acrylic boxes to the paw withdrawal test. On the $7^{th}$ day after the surgery (CCI), the paw withdrawal test was again performed in order to verify neuropathic pain has been effectively developed in the left paw - defined as "post-surgery control."

The paw withdrawal test was assessed during the days 1, 3, and 7 of the treatment (with amitriptyline hydrochloride or the compounds of examples of the instant invetion) and on days 1, 3, and 7 after treatment interruption. **Figures 3-14** show that in the comparison between the controls, this model was able to develop neuropathic pain in rats.

The results were presented as mean ± standard error of the mean and statistically compared to control by one-way ANOVA followed by Dunnett's test. Differences were considered significant when $p<0.05$.

### Antinociceptive effect (thermal hyperalgesia) of amitriptyline hydrochloride and compounds C3, C5, C6, C9, C11, C12, C15, C16, C17, and C18 in rats subjected to sciatic nerve CCI.

Amitriptyline hydrochloride was diluted in water and administered to the rats on daily basis from day 7, at a daily dose of 32.25 μmol/kg (10.12 mg/kg), during 7 days through intraperitoneal route.

**Figure 3A** shows that amitriptyline hydrochloride significantly increased ($p<0.005$, One-way ANOVA, Dunnett) the paw withdrawal latency on day 7 of treatment, showing efficacy in reversing the signs of hyperalgesia associated with neuropathic pain, when administered after the onset of symptoms. After treatment interruption, the antinociceptive effect was observed only after the first day. **Figure 3B** shows the results obtained on the right paw (contralateral), which was not submitted to the CCI and therefore did not show significant difference between the "control before surgery" and "control post-surgery."

Compounds whose results are shown in **Figures 4 (C3), 8 (C11), 9 (C18), 10 (C5), 11 (C17), 12 (C6), 13 (C15)** and **14 (C16)** have shown antinociceptive effects in the first day of treatment, comparing to post-surgery control. For the compounds shown in **Figures 11 (C17)** and **14 (C16)** the latency increase was statistically significant ($p<0.05$ or $p<0.005$) on the first day of treatment. For the compounds shown in **Figures 4 (C3), 6 (C9), 8 (C11)** and **13 (C15),** a statistically significant increase in latency of paw withdrawal ($p<0.05$ or $p<0.005$) is observed since the $3^{rd}$ day of treatment. On the $7^{th}$ day of treatment, all tested compounds showed a significant increase ($p<0.005$, One way ANOVA, Dunnett) on latency to paw withdrawal.

**Figure 5 (C3)** shows the dose-response relationship in compound **C3** analgesic activity when administered at doses of **1, 5** and **10 mg/kg** through ip route. The results show that at a dose of 5 and 10 mg/kg during 7 days of

treatment, compound C3 was effective in reversing the signs of thermal hyperalgesia in **CCI** model in rats (showing a significant increase in latency starting on day 3 of treatment at the doses of 5 and 10 mg/kg).

**2) Spinal Nerve Ligation Model (SNL) $L_5$ in rat**

This experimental model was proposed by Kim and Chung [Kim, S.H.; Chung, J.M. Pain 1992, 50: 355-363] and consists of ligation of spinal nerves. Male Wistar rats, 180-220 g, were anesthetized with a mixture of ketamine and xylazine (60 mg/kg and 5 mg/kg, respectively) intraperitoneally. After the dorsal region asepsy, an incision was made between L5 and S1. The L6 transverse process was removed for locating the nerves L4 and L5, which were carefully isolated, and tight ligation of the L5 nerve with 6.0 silk thread was made, interrupting flow of the axon of the nerve. After this procedure, para-spinal muscles and skin were sutured, and then a topical antiseptic was applied. False operated animals (sham) were subjected to the same procedure, however the spinal nerve was not ligated.

In this neuropathic pain experimental model, allodynia and hyperalgesia were assessed.

**a) Assessment of thermal hyperalgesia**

This method aims to evaluate the animal behavior in response to hyperalgesic phenomenon resulting from the injury induced by the ligament of spinal nerve (L5). In this regard, paw withdrawal in response to thermal stimulus was used, and the thermal stimulus was induced by the radiant heat of an incandescent lamp bulb. The paw withdrawal test was performed as described for the assessment of thermal hyperalgesia in the sciatic nerve chronic constrictive injury model.

The results were presented as mean ± standard error of the mean and compared statistically with the control by one-way ANOVA followed by Dunnett's test. Differences were considered significant when $p<0.05$.

**b) Assessment of mechanical allodynia**

To evaluate the development of mechanical allodynia, digital analgesimeter (Model EFF301; Insight Equipments), digitalized version coupled to a pointed filament (*Von Frey* model variant) was used. This test aims to assess changes in tactile sensitivity in response to a mechanical stimulus as a result of a neural damage. The device consisted of a hand-held force transducer fitted with a sensor tip, through which a pressure with increasing strength is applied against the footpad of the animals hindpaw to assess the threshold of paw withdrawal, that is, the digital analgesimeter records the maximal strength, in grams (g), in which the animal withstands until the paw withdrawal reflex.

Animals were placed in individual acrylic boxes and let to acclimate for 30 min before the beginning of the test. After this period, the thresholds of hindpaw withdrawal were determinded. For each hindpaw, five records of the withdrawal thresholds were made, and the average for each hindpaw was defined as the paw withdrawal threshold control.

After 7 days of the SNL, the withdrawal latency was significantly reduced when compared to the controls: "control before surgery" vs. "control post-surgery," showing SNL model in rats operated was able to develop neuropathic pain. This was not observed with the false-operated group (Sham).

The treatment started on the $7^{th}$ day after surgery. Compounds were diluted in water or DMSO and administered through intraperitoneal route on daily basis, during 7 days, at a dose of 32.25 mol/kg. The effectiveness of tested compounds was assessed on days 1, 3, and 7 days after treatment, always before the next administration of the compound. Latencies were also assessed on days 1, 3, and 7 after treatment interruption.

The results were presented as mean ± standard error of the mean and compared statistically to the control by one-way ANOVA followed by Dunnett's test. Differences were considered significant when $p<0.05$.

**Antinociceptive effect (thermal hyperalgesia and mechanical allodynia models) of amitriptyline hydrochloride and compounds C3, C9, C11, C12, C15 and C17 in rats sunjected to SNL.**

The antinociceptive effect of amitriptyline hydrochloride administration for 7 days in rats is shown in **Figures 15 (thermal hyperalgesia)** and **16 (mechanical allodynia).** The treatment at the dose of 32.25 μmol/kg (10.12 mg/kg) significantly increased ($p<0.005$) the latency to paw withdrawal on day 7 of treatment, showing efficacy in reversing the signs of hyperalgesia associated to neuropathic pain when administered after the onset of symptoms. After treatment interruption, the analgesic effect is not maintained.

There was no significant threshold increase after 7 days of treatment with amitriptyline hydrochloride in mechanical allodynia test **(Figure 16).**

Selected compounds of the present invention for SNL model assay (compounds C9, C11, C12, C15 and C17) have the results shown in **Figures 17-26,** which show that the compounds administered through intraperitoneal route during 7 days were effective in reverse the hyperalgesia and allodynia signs associated with neuropathic pain.

The compounds whose results for thermal hyperalgesia are shown in **Figures 17 (C9), 23 (C17), 21 (C11),** and **25 (C15)** show a statistically significant antinociceptive effect after 7 days of treatment. For the compound shown in **Figure 25,** the observed effect is maintained for 1 day after treatment interruption. For the compound shown in **Figure 19 (C12)** a statistically significant increase ($p<0.05$ or $p<0.005$, One way ANOVA, Dunnet) on the latency is observed since the 3rd day of treatment when compared to control post-surgery. On the other hand, the compound shown in **Figure 27 (C3)** is able to induce a statistically significant increase in paw withdrawal latency ($p<0.05$ or $p<0.005$) since the first day of treatment, and this effect is still observed up to 1 day after treatment interruption.

Unexpected results are also observed for the mechanical allodynia model, being statistically significant for all the tested compounds after 7 days of treatment. After only 3 days of treatment, a significant ($p<0.05$) paw withdrawal threshold is observed for the compounds shown in **Figures 20 (C12), 22 (C11)** and **28 (C3).** For the compound shown in **Figure 28** analgesic effect is maintained until the first day after treatment interruption.

Additionally, the oral administration of the compound **C3** at doses of **30 mg/kg (Figures 29** and **30)** and **100 mg/kg (Figures 31** and **32)** demonstrate that the treatment significantly increased the paw withdrawal latency after 7 days of treatment (dose 30 mg/Kg) and after 1 day of treatment (100 mg/kg) in the thermal hyperalgesia model. Furthermore, the reversion of the signs of mechanical allodynia ($p<0.05$) after the compound C3 oral administration on both dosages tested was observed after the first day of treatment **(Figures 30 and 32).**

Thus, these results show the compounds of the present invention are effective in reversing signs of hyperalgesia and mechanical allodynia induced by SNL.

**Assessment of Motor Coordination in Mice - RotaRod Performance Test**

The assessment of motor coordination in mice, by the rotarod performance test, was carried out to detect any possible sedative or muscle relaxant effect caused by the compounds of the present invention, previously tested in acute and neuropathic pain models. This test was carried out according to the method of described by Dunham & Miya and animal motor coordination was evaluated using RotaRod apparatus [Dunham M.S. & Miya. R.T. (1957). J. Amer. Pharmac. Assoc. Sci. Edit. 1957, 46, 208-209].

The RotaRod apparatus (Model 412 EFF, Insight Equipements, Brazil) is a device that measures motor coordination and thus evaluates balance alterations in mice. The apparatus consists of an acrylic box splited into four compartments with a rotational cylinder among them. The cylinder rotates at constant speed driven by a motor and regulated to the constant speed mode of 5-37 revolutions per minute (rpm). In this study the speed was set up to 9 rpm.

To remain on the rotational cylinder, the animal needs to move around, so that the motor coordination can be quantified. The four compartments have drop detection system, through the impact, a microprocessor circuit, for timing the permanence of the animal at the compartment, and for counting the number of falls.

The day before the test, Swiss-type male mice (19-25 g) were placed on the rotational cylinder, set up to the constant speed mode of 9 rpm for adaptation and learning. A group of 10 mice were used for each tested compound, and the total period spent on the rotational cylinder (in seconds) was recorded for a period of 180 seconds (cut off time) and it was accept a maximum of three falls for each animal. Motor coordination was assessed at times 0 (before treatment), 15, 30, 60 and 120 minutes after compounds administration at a dose of 32.25 μmol/kg.

As the positive control for the neuropathic pain model, amitriptyline hydrochloride at a dose of 32.25 μmol/kg (10.12 mg/kg) was used, and as the positive control for the RotaRod test, diazepam was used used at a dose of 2 mg/kg. All compounds were administered intraperitoneally (ip).

The results were presented as mean ± standard error of the mean and statistically compared to control by ANOVA (one-way) followed by Kruskal-Wallis test/Dunn's. The Student's t-test was also used to compare means between two experimental groups. Differences were considered significant when $p<0.05$.

The results of RotaRod test for the compounds of the present invention are shown in **Table 1.**

**Table 1 - Assessment of Motor Coordination in Mice**

| T (min) | Mice residence time on the rotational cylinder (s) | | | | | | | | | | | |
|---|---|---|---|---|---|---|---|---|---|---|---|---|
| | saline | Amitrip | Diazep | C13 | C17 | C5 | C11 | C3 | C9 | C12 | C15 | C16 |
| **15** | 180.0 ± 0 | 128.2 ±16.1 * | N.R. | 100.9 ±21.8 * | 127.4 ±19.9 * | 180.0 ±0 # | 180.0 ±0 # | 180.0 ±0 # | 180.0 ±0 # | 179.6 ±0.8 # | 180.0 ±0 # | 178.6 ±1.4 # |
| **30** | 177.5 ±2.5 | 151.0 ±14.4 $ | 28.1 ±15.1* | 95.5 ±23.2*# | 153.3 ±12.6 $ | 178.6 ±1.4 $ | 177.0 ±2.0 $ | 178.2 ±1.8 $ | 180.0 ±0 $ | 178.6 ±1.4 $ | 179.0 ±1.0 $ | 178.8 ±0.8 $ |
| **60** | 166.8 ±13.2 | 175.8 ±2.8 | 112.3 ±27.8* | 143.7 ±18.2 | 163.2 ±9.4 | 173.4 ±5.0 | 178.5 ±1.5 $ | 178.7 ±1.3 $ | 180.0 ±0 $ | 178.2 ±0.9 $ | 179.2 ±0.8 $ | 150.8 ±13.6 |
| **120** | 164.1 ±13.7 | 161.8 ±12.9 | 177.0 ±2.9 | 152.4 ±10.4 | 175.6 ±2.3 | 179.2 ±0.8 | 176.7 ±1.3 | 180.0 ±0 | 175.3 ±3.2 | 179.8 ±0.2 | 180.0 ±0 | 126.3 ±16.0 *#$ |

N.R. Not Rated
* $P<0.05$ vs. control (saline)
# $P<0.05$ vs. amitriptyline hydrochloride
$ $P<0.05$ vs. diazepam

The data demonstrate all the nine compounds tested at the dose of 32.25 μmol/kg assessed 15 minutes after administration, did not modify the animal residence time on the rotational cylinder, when compared to amitriptyline hydrochloride.

For compounds C5, C11, C3, C9, C15, C12 and C16, the animal residence time on the rotational cylinder after 15 minutes of their administration was equal to the time observed for the negative control, and significantly higher than the time observed for the control amitriptyline hydrochloride. Amitriptyline hydrochloride at a dose of 32.25 μmol/kg (10.12 mg/kg) significantly reduced the animals residence time from 180.0 ± 0 (control - saline) to 128.2 ± 16.1 s ($p<0.05$) after 15 min.

## Examples

The present invention is illustrated, but not limited, by the following examples, which describe the preparation of compounds of formula (I) according to the invention.

### Example-1

**1-{2-[3-(4-fluorophenyl)-1*H*-pyrazol-4-yl]ethyl}piperidine** (C1):

(1) Intermediate-1. Method-A: 1-(4-fluorophenyl)-4-(piperidin-1-yl)butan-1-one hydrochloride. In a three-neck glass reactor with up to 3 liters overall capacity, fitted with a heating reflux system, a thermometer and mechanical stirring system, were added: 240 mL (2.43 mol) of piperidine, 286 g (2.70 mol; 111 mol%; 2,2 eq.) of sodium carbonate, 36.4 g (0.24 mol; 0.1 eq.) of sodium iodide, 2.4 L of methyl isobutyl ketone, and 500 mL (2.95 mol; 1.2 eq.) of 4-chloro-4'-fluorobutyrophenone. The mixture was refluxed under stirring for 3.5 h, monitoring the reaction progress by TLC. After the reaction has been concluded, the medium was cooled to 40-50°C. Water (1.6 L) was added to the reaction vessel with vigorous stirring during 15 min. The phases were let to separate; aqueous phase was discarded and organic phase, retained. The organic phase was washed with 1.6 L of water for 30 minutes. The phases were let to separate and the organic phase was treated with activated charcoal for 30 minutes and then dried over anhydrous $Na_2SO_4$ for 30 minutes. The suspension was vacuum filtered through Celite. The organic phase was cooled at 0-5°C and saturated with gaseous HCl. The mixture was stirred at 0-5°C for 1 h until complete hydrochloride precipitation. The hydrochloride formed was vacuum filtered and was macerated in 1.5 L of acetone for 30 minutes at room temperature. The solid was filtered and washed with ether. The product was dried in an oven at 50°C. It was obtained 433 g of 1-(4-fluorophenyl)-4-(piperidin-1-yl)butan-1-one hydrochloride. Mp (DSC) = polymorphic mixture: 133-135°C and 149-152°C. IR (KBr): $\nu^-$ ($cm^{-1}$) = 3036, 3001, 2940, 2862, 2809, 2736, 2623, 2532, 2508, 2486, 2411, 1690, 1600, 1510, 1466, 1409, 1380, 1288, 1215, 1158, 1094, 987, 958, 861, 836, 764, 600, 581, 559. MS: m/z (rel. intensity) = 249 $[M]^+$, 123, 111, 98 (100%).

(2) Intermediate-1. Method-B: 1-(4-fluorophenyl)-4-(piperidin-1-yl)butan-1-one hydrocloride. In a three-neck glass reactor with up to 2 liters overall capacity, fitted with heating reflux system, thermometer and magnetic stirring system, 40 mL (0.405 mol) of piperidine, 6.2 g (0.449 mol; 111 mol%; 2.2 eq.) of potassium carbonate, 6.72 g (0.04 mol; 0.1 eq.) of potassium iodide, 400 mL of methyl isobutyl ketone, and 75 mL (0.442 mol; 1.1 eq.) of 2-(3-chloropropyl)-2-(4-fluorophenyl)-1,3-dioxolane (commercially available) were added. The mixture was heated under vigorous stirring for 3.5 h. After the reaction has been completed, the medium was cooled to 40-50°C. Water (270 mL) was added with vigorous stirring for 15 min. The phases were let to separate; aqueous phase was discarded and organic phase retained. The organic phase was washed with 270 mL of water for 15 minutes. The phases were let to separate and the organic phase was extracted with 280 mL of 3N HCl. The aqueous phase was heated up to 60°C for 2 h. The solution was cooled to 10-20°C. Methyl isobutyl ketone (400 mL) and 3N NaOH (285 mL) were added until pH>10. The mixture was vigorously stirred for 30 minutes. The phases were let to separate. The organic phase was washed with 270 mL of water and dried over anhydrous $Na_2SO_4$ for 30 minutes. The suspension was vacuum filtered. The filtrate was cooled to 0-10°C and saturated with gaseous HCl. The mixture was stirred at 0-10°C for 1 h until complete hydrochloride salt precipitation. The hydrochloride salt formed was vacuum filtered and macerated in 250 mL of acetone for 30 minutes at room temperature. The solid was vacuum filtered and dried in an oven at 50°C. It was obtained 78.5 g of 1-(4-fluorophenyl)-4-(piperidin-1-yl)butan-1-one hydrochloride. Mp (DSC) = polymorphic mixture 133-135°C and 149-152°C. IR (KBr): $\nu^-$ ($cm^{-1}$) = 3036, 3001, 2940, 2862, 2809, 2736, 2623, 2532, 2508, 2486, 2411, 1690, 1600, 1510, 1466, 1409, 1380, 1288, 1215, 1158, 1094, 987, 958, 861, 836, 764, 600, 581, 559. MS: m/z (rel. intensity) = 249 $[M]^+$, 123, 111, 98 (100%).

(3) Intermediate-1 free base: 1-(4-fluorophenyl)-4-(piperidin-1-yl)butan-1-one. In a 4 L Erlenmeyer flask, 428 g (1.50 mol) of of 1-(4-fluorophenyl)-4-(piperidin-1-yl)butan-1-one hydrochloride was dissolved in 1.7 L of water. 3N NaOH (577 mL; 1.73 mol; 1.15 eq.) was added to the flask to reach a pH between 12-14. The mixture was vigorously stirred for 30 minutes. The oily product was extracted with dichloromethane (2 x 770 mL). The phases were let to separate and the organic phase was washed with water (2 x 380 mL). The phases were again separated and the organic phase was dried over anhydrous $Na_2SO_4$ for 1 h. The suspension was vacuum filtered and the filtrate concentrated on rotatory evaporator. The oily residue was dried in an oven at 50°C for 12 h. After complete drying, the material was cooled to obtain 371.7 g of 1-(4-fluorophenyl)-4-(piperidin-1-yl)butan-1-one free base, as an amorphous solid and unctuous to the touch. MS: m/z (rel. intensity) = 249 $[M]^+$, 123, 111, 98 (100%).

(4) Intermediate-2: 1-(4-fluorophenyl)-2-(hydroxymethylidene)-4-(piperidin-1-yl)butan-1-one. In a three-neck glass reactor with up to 4 L overall capacity, dried, fitted with mechanical stirring and nitrogen input to the system, 1.55 L of dry ethanol were placed and were added 115 g (5.00 mol, 3.35 eq.) of metallic sodium, previously cut into pieces. Thirty minutes after the complete addition metallic sodium, the mixture was heated up to 75-80°C until complete sodium consumption. After the total sodium solubilization, reactor was cooled under inert atmosphere at 15±5°C in cold water bath. A solution composed of 371.7 g of 1-(4-fluorophenyl)-4-(piperidin-1-yl)butan-1-one (free base) and 390 mL (4.83 mol; 3.23 eq.) of ethyl format was slowly added, using an addition funnel; controlling the flow rate so that the temperature was maintained between 20 and 30°C. The red solution obtained was maintained under stirring for at least 6 hours between 20 and 30°C. After confirming total consumption of starting material, by TLC, water was added (1.2 L). The hydro-organic phase was added with 220 g of solid $NH_4Cl$ until pH 10. The mixture was extracted with $CH_2Cl_2$ (3 x 400 mL) and the extracts were concentrated at room temperature. The residue was macerated in ether. After filtering and drying at 35°C in an oven with air circulation, it was obtained 295 g of 1-(4-fluorophenyl)-2-(hydroxymethylidene)-4-(piperidin-1-yl)butan-1-one. Mp (DSC) = 141.5-143.5°C. IR (KBr): $\nu^-$ ($cm^{-1}$) = 3052, 2949, 2916, 2867, 2797, 2400, 2286, 2268, 1600, 1522, 1497, 1408, 1384, 1366, 1327, 1311, 1265, 1221, 1166, 1044, 963, 875, 839, 803, 725, 613, 562, 530. MS (EI) : m/z (rel. intensity) = 249 $[M-CO]^+$, 123, 111, 98 (100%) .

(5) Final Product. Method-A: 1-{2-[3-(4-fluorophenyl)-1*H*-pyrazol-4-yl]ethyl} piperidine. 295 g of 1-(4-fluorophenyl)-2-(hydroxymethyliden)-4-piperidin-1-yl)butan-1-one were suspended in 1.36 L of ethanol and then 126 mL (2.22 mol; 2.1 eq.) of 55% hydrazine hydrate were added. After total addition, the mixture was stirred for 1 h at room temperature. Thereafter, the system was heated up to the reflux temperature for 5 h. After the reaction has been completed, the system was cooled to room temperature. Water (3 L) was added under stirring. The solid obtained was vacuum filtered. It was macerated in water for 30 minutes and was again vacuum filtered. The solid was dried in an oven at 50°C. It was obtained 248 g of 1-{2-[3-(4-fluorophenyl)-1*H*-pyrazol-4-yl]ethyl}piperidina. This compound can be further purified by maceration at room temperature in ether acetone or ether/acetone (3:1), with recovery of 80-90%. Mp (DSC) = 145-146°C. IR (KBr): ν- (cm-1) = 3131, 3081, 2931, 2860, 2816, 2769, 2652, 2575, 1890, 1839, 1604, 1522, 1450, 1313, 1219, 1156, 1118, 1096, 1037, 922, 840, 813, 739, 604, 534, 450. $^1H$ NMR (400 MHz, $CDCl_3$, r.t., TMS): $\delta$ = 11.81 (bs, 1H); 7.50 (m, 2H); 7.44 (s, 1H) ; 7.07 (m, 2H) ; 2.80 (m, 2H) ; 2.52 (m, 3H) ; 2.44 (m, 3H); 1,60 (m, 4H); 1.44 (m, 2H). $^{13}C$-NMR (100 MHz, $CDCl_3$, r.t., TMS) : $\delta$ = 163.7; 161.3; 129.5; 129.4; 116.2; 115.7; 115.5; 60.0; 54.5; 25.9; 24.4; 21.7. MS: m/z (rel. intensity) = 273 $[M]^+$, 98 (100%). HRMS (TOF MS ES+) calc. for $[M^+I]^+$: 274.1714; found: 274.1613. KF = 0.13% $H_2O$.

(6) Final Product. Method-B: 1-{2-[3-(4-fluorophenyl)-1*H*-pyrazol-4-yl]ethyl}piperidine. 30 g (0.108 mol) of 1-(4-fluorophenyl)2-hyroxymethyliden)-4-(piperidin-1-yl)butan-1-one were suspended in 135 mL of ethanol and 13 mL (0.23 mol, 2.1 eq.) of 55% hydrazine hydrate were added to the suspension. The pH was adjusted to the range between 5 - 6 with acetic acid. The mixture was stirred for 1 h at room temperature. Thereafter, the system was heated up to reflux temperature for 3 h. After the reaction has been concluded, the system was cooled to room temperature. 300 mL water were added under constant stirring. The pH was adjusted between 9-10 with $NH_4OH$. The solid obtained was vacuum filtered, macerated in water and again was vacuum filtered. The solid was dried in an oven at 50°C. It was obtained 26.2 g of 1-{2-[3-(4-fluorophenyl)-1*H*-pyrazol-4-yl]ethyl}piperidine, that can be further purified by maceration in ether at room temperature, acetone, or ether/acetone (3:1). Mp (DSC) = 145-146°C. IR (KBr): $\nu^-$ ($cm^{-1}$) = 3131, 3081, 2931, 2860, 2816, 2769, 2652, 2575, 1890, 1839, 1604, 1522, 1450, 1313, 1219, 1156, 1118, 1096, 1037 , 922, 840, 813, 739, 604, 534, 450. $^1H$ NMR (400 MHz, $CDCl_3$, r.t., TMS) : $\delta$ = 11.81. (Bs, 1H); 7.50 (m, 2H) ; 7.44 (s, 1H) ; 7.07 (m, 2H) ; 2.80 (m, 2H) ; 2.52 (m, 3H); 2.44 (m, 3H); 1.60 (m, 4H); 1.44 (m, 2H). MS: m/z (rel. intensity) = 273 $[M]^+$, 98 (100%). KF = 0.18% $H_2O$.

(7) Final Product. Method C: 1-{2-[3-(4-fluorophenyl)-1*H*-pyrazol-4-yl]ethyl}piperidine. In a three-neck glass reactor, dried, with up to 250 mL overall capacity, fitted with magnetic stirring system and nitrogen input, it was added 10 g (35 mmol) of 1-(4-fluorophenyl)-4-(piperidine-1-yl)butan-1-ona hydrochloride, 5.32 g (38.5 mol; 110 mol%; 2.2 eq.)

of potassium carbonate anhydrous, 18.8 mL (140.4 mmol; 4.0 eq.) of dimethylformamide dimethyl acetal, and 70 mL of N,N-dimethylformamide anhydrous. The mixture was heated up to 120°C for 5 h. After the reaction has been concluded, the mixture was cooled to room temperature. It was diluted in water and extracted with ethyl acetate three times. The organic phases were pooled and washed with saturated NaCl solution. The phases were let to separate and the organic phase was dried over anhydrous $Na_2SO_4$ for 30 minutes. The suspension was vacuum filtered and the filtrate was concentrated until dryness in a rotatory evaporator, providing the vinylogous amide intermediate 2-[(dimethylamino)methyliden]-1- (4-fluorophenyl)-4-(piperidin-1-yl)butan-1-one. This compound was dissolved in 35 mL of ethanol and then added with 4.4 mL (77.7 mmol) of 55% hydrazine hydrate. The mixture was heated up to reflux temperature for 5 h. After the reaction has been concluded, the system was cooled to room temperature. The mixture was diluted with water until complete precipitation of the product. The solid obtained was vacuum filtered. It was macerated in water for 30 min and again vacuum filtered. The solid was dried in an oven at 50°C. The dried solid was macerated in a mixture of ether/acetone (3:1) and dried again in an oven at 50°C. It was obtained 6.9 g of 1-{2-[3-(4-fluorophenyl)-1H-pyrazol-4-yl]ethyl}-piperidine. IR (KBr): $\nu^-$ ($cm^{-1}$) = 3131, 3081, 2931, 2860, 2816, 2769, 2652, 2575, 1890, 1839, 1604, 1522, 1450, 1313, 1219, 1156, 1118, 1096, 1037 , 922, 840, 813, 739, 604, 534, 450 MS: m/z (rel. intensity) = 273 $[M]^+$, 98 (100%). KF = 0.15% $H_2O$.

**Example-2**

**1-{2-[3-(4-fluorophenyl)-1H-pyrazol-4-yl]ethyl}piperidine** (C1):

In a three-neck glass reactor, dried, with up to 250 mL overall capacity, fitted with magnetic stirring system and nitrogen input, it was added 10 mL (58.96 mmol) of 4-chloro-4'-fluorobutyrophenone, 40 mL (298.8 mmol; 5.0 eq.) of dimethylformamide dimethyl acetal, and 60 mL of N,N-dimethylformamide anhydrous. The mixture was heated up to reflux temperature for 2.5 h. After the reaction has been completed, the mixture was cooled to room temperature. It was diluted with water and extracted with ethyl acetate three times. The organic phases were pooled and washed with saturated NaCl solution. The phases were let to separate. The organic phase was concentrated until dryness in a rotatory evaporator, providing the vinylogous amide intermediate 4-chloro-2-[(dimethylamino)methyliden]-1-(4-fluorophenyl)butan-1-one. This vinylogous amide was dissolved in 70 mL of ethanol and added with 3.4 mL (60.0 mmol) of 55% hydrazine hydrate. The pH was adjusted to the range between 5-6 with acetic acid. The mixture was stirred at room temperature for 2 h and then it was heated up to reflux for 6 h. After the reaction has been concluded, the solution was concentrated under vacuum until dryness. The crude compound obtained 4-(2-chloroethyl)-3-(4-fluorophenyl)-1*H*-pyrazole was redissolved in 60 mL of ethanol and added with 6.0 mL (60.75 mol) of piperidine, 22.5 mL of diisopropylethylamine (DIPEA), and 1 g (6.02 mmol) of potassium iodide. The mixture was heated up to reflux for 15 h. After the reaction has been concluded, the system was cooled to room temperature. The mixture was diluted with water and the pH was adjusted to the range between 10-11 with $NH_4OH$, under vigorous stirring. The obtained solid was vacuum filtered and then macerated in water for 0.5 h. The solid obtained was dried in an oven at 50°C. It was again homogenized with ether and dried in an oven at 50°C. It was obtained 5.52 g of 1-{2-[3-(4-fluorophenyl)-1*H*-pyrazol-4-yl]ethyl}piperidine. IR (KBr): $\nu^-$ ($cm^{-1}$) = 3131, 3081, 2931, 2860, 2816, 2769, 2652, 2575, 1890, 1839, 1604, 1522, 1450, 1313, 1219, 1156, 1118, 1096, 1037 , 922, 840, 813, 739, 604, 534, 450. MS: m/z (rel. intensity) = 273 $[M]^+$, 98 (100%). KF = 0.19% $H_2O$.

**Example-3**

**1-{2-[3-(4-fluorophenyl)-1H-pyrazol-4-yl]ethyl}piperidine dihydrochloride monohydrate** (C3):

In a three-neck glass reactor, with up to 5 L overall capacity, fitted with effective magnetic stirring system and an addition funnel, 231 g (0.84 mol) of 1-{2-[3-(4-fluorophenyl)-1*H*-pyrazol-4-yl]ethyl}piperidine (free base) was suspended in 3.5 L of acetone. Concentrated HCl (215 mL; 2.35 mol; 3 eq.) was slowly added to the suspension. The mixture was vigorously stirred for 1 h. The obtained solid was vacuum filtered and washed with 1.5 L acetone and 50 mL of ether. It was dried in an oven at 50°C. It was obtained 286.7 g of 1-{2-[3-(4-fluorophenyl)-1*H*-pyrazol-4-yl]ethyl}piperidine dihydrochloride monohydrate as a white crystalline solid. Mp (DSC) = 277-279°C. IR (KBr): $\nu^-$ ($cm^{-1}$) = 3378, broadband between 3250 and 1850 with peaks at 3091, 3064, 3024, 2950, 2546, 1862, 1751, 1605, 1515, 1464, 1444, 1232, 1160, 965, 942, 880, 846, 818, 772, 603, 533 $^1$H-NMR (250 MHz, $D_20$, r.t., TMS): $\delta$ = 7.95 (s, 1H), 7.61 (dd, J = 8.5 Hz and 5.4 Hz, 2H), 7.32 (t, J = 8.8 Hz, 2H), 3.43 (d, J = 12.3 Hz, 2H), 3.18 (m, 2H), 3.14 (m, 2H), 2.86 (td, J = 12.3 Hz and 2.2 Hz, 2H), 1, 96-1, 53 (m, 5H); 1 43 (m, 1H). $^1$H-NMR (250 MHz, DMSO-$d_6$, r.t., TMS): $\delta$ = 10.75 (bs, 1H), 8.98-7.80 (bs, 4H), 7 70 (s, 1H), 7 75-7 62 (m, 2H), 7.29 (t, J = 8.8 Hz, 2H), 3.45 (d, J = 11.7 Hz, 2H), 3.16 (m, 2H), 3.10 (m, 2H), 2.85 (m, 2H), 1.78 (m, 5H), 1.38 (m, 1H). HRMS (TOF MS ES +) calc. for $[M^+1]^+$: 274.1714, found: 274.1613 KF = 4,92% $H_2O$.

**Example-4**

**1-{2-[3-(4-fluorophenyl)-1H-pyrazol-4-yl]ethyl}piperidine dihydrochloride dihydrate** (C4):

In a reactor with 25 mL of overall capacity, fitted with magnetic stirrer, 1 g (3.658 mmol) of 1-{2-[3-(4-fluorophenyl)-1*H*-pyrazol-4-yl]ethyl}piperidine, as free base, was suspended in 15 mL of acetone. To his suspension was added 0.93 mL (10.9 mmol; 3 eq.) of concentrated HCl until pH = 1. The solid was dissolved and then precipitated. The mixture was stirred for 20 minutes. The white solid formed was vacuum filtered. It was dried in an oven at 40°C. It was obtained 1.316 of 1-{2-[3-(4-fluorophenyl)-1*H*-pyrazol-4-yl]ethyl}dihydrochloride dihydrate. $^1$H-NMR (250 MHz, DMSO-$d_6$, r.t., TMS): $\delta$ = 10.82 (bs, 1H); 7.71 (s, 1H); 7.68 (m, 2H); 7.29 (t, J = 8.8 Hz, 2H); 7.11 to 5.54 (bs, 6H); 3.45 (d, J = 11.7 Hz, 2H); 3.16 (m, 2H); 3.11 (m, 2H); 2.85 (m, 2H); 1.78 (m, 5H); 1.38 (m, 1H). KF = 9.48% $H_2O$.

**Example-5**

**1-{2-[3-(4-fluorophenyl)-1,2-oxazol-4-yl]ethyl}piperidine hydrochloride** (C5):

163 g of 1-(4-fluorophenyl)-2-(hydroxymethyliden)-4-(piperidin-1-yl)butan-1-one were suspended in 490 mL of ethanol and then added with 44.95 g (0.645 mol; 1.1 eq.) of hydroxylamine hydrochloride. After the addition has been completed, the mixture was stirred for 1 h at room temperature. Thereafter, the system was heated up to reflux for 3 h. After the reaction has been completed, the mixture was vaccum concentrated until 1/3 of the initial volume. 490 mL of methyl tert-butyl ether (MTBE) were added under stirring. The obtained solid was vacuum filtered. The solid was suspended in 390 mL of ethanol and stirred at reflux temperature until complete homogenization. The heating was turned off and it was added 390 mL of MTBE. The mixture was cooled to room temperature. The solid was vacuum filtered and dried in an oven at 50°C. It was obtained 95 g of 1-{2-[3-(4-fluorophenyl)-1,2-oxazol-4-yl]ethyl}piperidine hydrochloride. Mp (DSC) = 198.0-200.5°C (dec.). IR (KBr): $\nu^-$ ($cm^{-1}$) = 3435, 3055, 2927, 2617, 2525, 2399, 1628, 1597, 1510, 1462, 1369, 1300, 1227, 1152, 1084, 1007, 920, 885, 839, 733, 685. $^1$H NMR (500 MHz, DMSO-$d_6$, r.t., TMS): $\delta$ = 10.82 (bs, 1H), 8.70 (s, 1H), 7.87 (dd, J = 8.3 Hz and 5.7 Hz, 2H), 7.42 (t, J = 8.8 Hz, 2H), 3.50 (d, J = 11.7 Hz, 2H), 3.30 (m, 2H), 3.15 (m, 2H), 2.89 (m, 2H), 1.81 (m, 4H), 1.72 (m, 1H); 1.40 (m, 1H). $^1$H NMR (500 MHz, $D_20$, r.t., TMS): $\delta$ = 8.51 (s, 1H), 7.76 (dd, J = 8.9 Hz and 5.3 Hz, 2H), 7.35 (t, J = 8.9 Hz, 2H), 3.32 (m, 4H), 3.16 (m, 4H), 1.82 (m, 4H), 1.65 (m, 2H). HRMS (TOF MS ES+) calc. for $[M^+I]^+$: 275.1554, found: 275.1467. KF = 0.17% $H_2O$.

**Example-6**

**1-{2-[4-(4-fluorophenyl)pyrimidin-5-yl]ethyl}piperidinium acetate with 0.8 water molecules** (C6)

In a flask of 50 mL overall capacity 3.0 g (10.8174 mmol) of 1-(4-fluorophenyl)-2-(hydroxymethyliden)-4-piperidin-1-yl)butan-1-one were suspended in 25 mL of isopropanol and then 1.15 g (11.046 mmol; 1.02 eq.) of formamidine acetate were added. After addition has been completed, the mixture was heated up to reflux and the reaction monitored by TLC. After the reaction has been concluded, the mixture was concentrated until dryness. The residue was crystallized from acetone. It was vacuum filtered and dried in a oven at 50°C. It was obtained 1.35 g of 1-{2-[4-(4-fluorophenyl)pyrimidin-5-yl] ethyl}piperidinium acetate with 0.8 water molecules. Mp = 133. 5-136 isobutil ketone 5°C (dec). IR (KBr): $\nu^-$ ($cm^{-1}$) = broadband between 3650 and 2850 with peaks at 3482, 3340, 3287, 3161, 2985, 2951, 2869, 2680, 1676, 1599, 15491403, 1314, 1225, 1130, 1015, 956, 925, 847, 768. $^1$H-NMR (500 MHz, DMSO-$d_6$, r.t., TMS): $\delta$ = 7.36 (dd, J = 8.6 Hz and 5.7 Hz, 2H); 7.20 (t, J = 8.8 Hz, 2H); 6.93 (m, 2H); 2.48 (m, 6H); 2.31 (t, J = 7.5 Hz, 2H); 1.88 (s, 3H); 1.53 (m, 4H); 1.40 (m, 2H). HRMS (TOF MS ES+) calc. for $[M^+I]^+$: 286.1714; found: 286.1796. KF = 4.05% $H_2O$ (representing 0.8 water molecules).

**Example-7**

**1-{2-[3-(4-fluorophenyl)-1H-pyrazol-4-yl]ethyl}-4-methylpiperidine dihydrochlotide dihydrate** (C7):

(1) Intermediate-1: 1-(4-fluorophenyl)-4-(4-methylpiperidin-1-yl)butan-1-one hydrochloride. In a three-neck glass reactor, with up to 5 L overall capacity, fitted with heating to reflux system, a thermometer, and mechanical stirring system, it was added 380 mL (2.22 mol; 1.15 eq.) of 4-fluoro-4'-chlorobutyrophenone, 2.0 L methyl isobutyl ketone, 236.1 g (2.23 mol; 114.5 mol%, 2.3 eq.) of sodium carbonate, 240 mL (1.95 mol; 1.0 eq.) of 96% 4-methylpiperidine, and 30.4 g (0.20 mol; 0.1 eq.) of sodium iodide. The mixture was heated up to reflux under stirring for 4 h, and the

reaction monitored by TLC. After the reaction has been completed, the medium was allowed to cool to a temperature between 40-50°C. It was added 2.5 L of water under vigorous stirring during 15 min. The phases were let to separate; aqueous phase was discarded and organic phase, retained. The organic layer was washed with 1.25 L water for 30 minutes. The phases were separated and the organic phase was treated with activated charcoal for 30 minutes and then was dried over anhydrous $Na_2SO_4$ for 30 minutes. The suspension was vacuum filtered through Celite. The organic phase was cooled at 0-5°C and sufficiently saturated with gaseous HCl. The mixture was stirred at 0-5 ° C for 1 h until complete hydrochloride precipitation. The obtained solid formed was vacuum filtered and then macerated in cold acetone (2x 1.2 L) for 0.5 h. The product was dried in an oven at 50°C. It was obtained 388.5 g of 1-(4-fluorophenyl)-4-(4-methylpiperidin-1-yl)butan-1-one hydrochloride. MS: m/z (rel. intensity) = 263 $[M]^+$, 125, 123, 112 (100%).

(2) Intermediate-1 free base: 1-(4-fluorophenyl)-4-(4-methylpiperidin-1-yl)butan-1-one. In a 4 L Erlenmeyer flask capacity 388.5 g (1.3 mol) of 1-(4-fluorophenyl)-4-(4-methyl-piperidin-1-yl)butan-1-one hydrochloride was dissolved in 1.55 L of water. 3N NaOH (519 mL; 1.56 mol; 1.2 eq.) was added to the flask to reach a pH between 12-14. The mixture was vigorously stirred for 0.5 h. The oily product was extracted with dichloromethane (2 x 700 mL). The phases were let to separate and the organic phase was washed with water (2 x 300 mL). The phases were again separated and the organic phase was dried over anhydrous $Na_2SO_4$ for 1 h. The suspension was vacuum filtered and the filtrate concentrated in rotatory evaporator. The oily residue was dried in an oven at 50°C for 15 h. After complete drying, the material was cooled to obtain 1-(4-fluorophenyl)-4-(4-methylpiperidin-1-yl)butan-1-one, free base, as an amorphous solid, unctuous to the touch. MS: m/z (rel. intensity) = 263 $[M]_+$, 125, 123, 112 (100%).

(3) Intermediate-2: 1-(4-fluorophenyl)-2-(hydroxymethyliden)-4-(4-methylpiperidin-1-yl)butan-1-one. In a three-neck glass reactor with up to 5 liters overall capacity, dried, fitted with mechanical stirring and nitrogen input to the system, 1,275 L of dry ethanol were placed and 95.57 g (4.15 mol; 3.2 eq.) of metallic sodium, previously cut into pieces, were added. Thirty minutes after the complete metallic sodium addition, reactor was heated up to 75-80°C until complete sodium consumption. After the total sodium solubilization, reactor was cooled under inert atmosphere at 15±5°C in cold water bath. A solution composed of 1-(4-fluorophenyl)-4-(4-methylpiperidin-1-yl)butan-1-one (free base), 326 mL (4.04 mol; 3.1 eq.) of ethyl formate and 750 mL of tetrahydrofuran (THF) was slowly added, using an addition funnel; controlling the flow rate so that the temperature was maintained between 20 and 30°C. The cream-colored solution obtained was maintained under stirring for at least 6 hours between 20 and 30°C. After confirming total consumption of starting material, by TLC, the mixture was concentrated under vacuum at room temperature. The residue was dissolved in 1.55 mL of methyl tert-butyl ether and extracted with 2.7 L of water. The aqueous phase was treated with solid ammonium chloride. The mixture was extracted with dichloromethane (3 x 390 mL) and the extracts were pooled and concentrated at room temperature, yielding a solid residue of dark brown color. The obtained solid was macerated at room temperature with 1.5 L of ether for 0.5 h. It was then vacuum filtered and dried in an oven with air circulation at 35°C. It was obtained 318 g of 1-(4-fluorophenyl)-2-(hydroxymethyliden)-4-(4-methyl-piperidin-1-yl)butan-1-one.

(4) Final Product: 1-{2-[3-(4-fluorophenyl)-1*H*-pyrazol-4-yl] ethyl}-4-methylpiperidine dihydrochloride dihydrate. 159 g (0.546 mol) of 1-(4-fluorophenyl)-2-(hydroxymethyliden)-4-(4-methylpiperidin-1-yl)butan-1-one were suspended in 275 mL of ethanol. To this suspension, 45 mL (0.795 mol; 1.45 eq.) of 55% hydrazine hydrate were added and the pH was adjusted to the range between 5-6 with 1N HCl. The mixture was stirred at room temperature for 1 h. Thereafter, the system was heated up to the reflux temperature, monitoring the reaction by TLC. After the reaction has been completed, the mixture was vaccum concentrated. Acetone (700 mL) was added to the residue and the mixture was acidified with concentrated HCl until pH = 0-1. The obtained solid was vacuum filtered and macerated with 500 mL of acetone at reflux temperature for 1 h. The mixture was cooled to room temperature and the solid was vaccum filtered and then dried in an oven at 50°C. It was obtained 142 g of 1-{2-[3-(4-fluorophenyl)-1*H*-pyrazol-4-yl]ethyl}-4-methylpiperidine dihydrochloride dihydrate. Mp (DSC) = polymorphic Mixture: 89-103°C. IR (KBr): $\nu^-$ ($cm^{-1}$) = broadband between 3650 and 2000 with peaks at 3443, 3372, 3105, 3053, 2947, 2841, 2710, 2642, 2546, 1610, 1514, 1447, 1313, 1236, 1167, 1061, 949, 843, 731, 688, 604. $^1H$ NMR (500 MHz, $D_20$, r.t., TMS): $\delta$ = 8.03 (s, 1H); 7.63 (dd, J = 8.8 Hz and 5.2 Hz, 2H); 7.35 (t, J = 8.8 Hz, 2H); 3.46 (d, J = 12.4 Hz, 2H); 3.23 (m, 2H); 3.14 (m, 2H); 2.90 (td, J = 13.0 Hz and 2.1 Hz, 2H); 1, 89 (d, J = 14.5 Hz, 2H); 1.66 (m, 1H); 1.42-1.31 (m, 2H); 0.95 (d, J = 6.5 Hz, 3H). $^{13}C$-NMR (125 MHz, $D_20$, r.t., TMS): $\delta$ = 167.4; 165.5; 147.8; 136.5; 133.4; 133.3; 125.9; 119.4; 119.3; 117.2; 58.8; 56.0; 33.8; 30.8; 23.1; 21.0. HRMS (TOF MS ES+) calc. for $[M^+I]^+$: 288.1870; found: 288.1875. KF = 9.10% $H_2O$.

**Example-8**

**1-{2-[3-(4-fluorophenyl)-1,2-oxazol-4-yl]ethyl}-4-methylpiperidine hydrochloride** (C8):

159 g (0.546 mol) of 1-(4-fluorophenyl)-2-(hydroxymethyliden)-4-(4-methylpiperidin-1-yl)butan-1-one were suspended in 550 mL of ethanol and 42 g (0.60 mol; 1.1 e.q.) of hydroxylamine hydrochloride was added. The suspension turned a dark amber clear solution. The mixture was stirred at room temperature for 1 h. Thereafter, the system was heated up to reflux for 2 h. After the reaction has been completed, the system was cooled to room temperature. The product precipitated as a white crystalline solid. It was then vacuum filtered and macerated with acetone at reflux temperature for 1 h. The mixture was cooled to room temperature and the solid was vacuum filtered. The obtained solid was dried in an oven at 50°C. It was obtained 140.3 g of 1-{2-[3-(4-fluorophenyl)-1,2-oxazol-4-yl] ethyl}4-methylpiperidine hydrochloride. Mp (DSC) = 229.5-233.0°C. IR (KBr): $\nu^-$ ($cm^{-1}$) = 3055, 2953, 2920, 2617, 2527, 1628, 1597, 1514, 1462, 1229, 1159, 1098, 1057, 937, 841, 735, 683. $^1$H-NMR (500 MHz, $D_2O$, r.t., TMS): $\delta$ = 8.50 (s, 1H), 7.74 (dd, J = 8.8 Hz and 5.4 Hz, 2H), 7.34 (t, J = 8.8 Hz, 2H), 3.51 (m, 2H), 3.34 (m, 2H), 3.15 (m, 2H), 3.00 (m, 2H), 1.94 (d, J = 14.0 Hz, 2H), 1.72 (m, 1H), 1.42 (m, 2H). 0.99 (d, J = 6.5 Hz, 3H). $^{13}$C NMR (125 MHz, $D_2O$, r.t., TMS): $\delta$ = 166.0, 165.6, 163.6, 153.3, 130.4, 130.3, 123.9, 117.3, 117.1, 110.5, 56.0, 53.7, 31.5, 28.7, 20.9, 18.7. HRMS (TOF MS ES+) calc. for $[M^+I]^+$: 289,1711; found : 289.1642. KF = 0.05% $H_2O$.

**Example-9**

**4-{2-[3-(4-fluorophenyl)-1*H*-pyrazol-4-yl]ethyl}morpholine dihydrochloride monohydrate** (C9):

(1) Intermediate-1: 1-(4-fluorophenyl)-4-(morpholin-4-yl)butan-1-one hydrochloride. In a three-neck glass reactor, with up to 6 L overall capacity, fitted with heating to reflux system, a thermometer, and mechanical stirring system, it was added 220 mL (2.51 mol; 1.0 eq.) of morpholine, 295 g (2.78 mol; 114.29 mol%; 2.2 eq.) of sodium carbonate, 37.6 g (0.25 mol; 0.1 eq.) of sodium iodide, 2.48 L of methyl isobutyl ketone (MIBK), and 516 mL (3.04 mol; 1.2 eq.) of 4-chloro-4'-fluorobutyrophenone. The mixture was heated internally to between 100-105°C for 5 hours under stirring, monitoring the reaction by TLC. After the reaction has been completed, the medium was cooled to 40-50°C. Water (1.6 L) was added under vigorous stirring for 15 min. The phases were let to separate; aqueous phase was discarded and organic phase, retained. The organic phase was washed with 1.6 L of water for 30 minutes. The phases were separated and the organic phase was dried over anhydrous $Na_2SO_4$ for 30 minutes. The suspension was vacuum filtered. The organic phase was further diluted with 1.6 L of MIBK, and the solution was cooled to 0-10°C. The solution was saturated with gaseous HCl. The mixture was stirred at 0-10°C for 1 h, until total hydrochloride precipitation. The solid formed was vacuum filtered and washed with 2.5 L of acetone. The product was dried in an oven at 50°C. It was obtained 526.65 g of 1-(4-fluorophenyl)-4-(morpholin-4-yl)butan-1-one hydrochloride, as a light yellow solid. MS: m/z (rel. intensity) = 251 $[M]^+$, 123, 113, 100 (100%). HRMS (TOF MS ES+) for $[M^+I]^+$: 252.1394; found: 252.1305.

(2) Intermediate-1 free base: 1-(4-fluorophenyl)-4-(morpholin-4-yl)butan-1-one. In a reactor of 6 L overall capacity, 526.65 g (1.83 mol) of 1-(4-fluorophenyl)-4-(morpholin-4-yl)butan-1-one hydrochloride were dissolved in 3.2 L of water. To this solution it was added 737 mL (2.21 mol; 1.2 eq.) of 3N NaOH until pH in the range between 12-14. The mixture was vigorously stirred for 30 minutes. The compound 1-(4-fluorophenyl)-4-(morpholin-4-yl)butan-1-one, as free base, precipitated as a yellowish solid. The solid was vacuum filtered and washed with 1 L of water. The solid was macerated in 2.5 L of water for 1 h. It was then vacuum filtered and dried in an oven at 60°C. At this drying temperature the solid melts with no impairment of the quality of the material. The product was crystallized back by cooling to room temperature. It was obtained 410.5 g of 1-(4-fluorophenyl)-4-(morpholin-4-yl)butan-1-one, as the free base. Mp (DSC) = 246-274°C (dec.). IR (KBr): $\nu^-$ ($cm^{-1}$) = 3342, 3049, 3029, 2968, 2895, 2859, 2818, 1680, 1596, 1507, 1461, 1408, 1358, 1307, 1275, 1264, 1227, 1204, 1170 , 1115, 1070, 1007, 983, 866, 840, 752, 629, 597, 565. MS: m/z (rel. intensity) = 251 $[M]^+$, 123, 113, 100 (100%).

(3) Intermediate-2: 1-(4-fluorophenyl)-2-(hydroxymethyliden)-4-(morpholin-4-yl)butan-1-one. In a three-neck glass reactor, with up to 6 L overall capacity, dried, fitted with mechanical stirring system, a condenser, a thermometer, and nitrogen input to the system, it was added 1.6 L of dry ethanol and 120 g (5.22 mol; 3.2 eq.) of metallic sodium, previously cut into pieces. Thirty minutes after the complete metallic sodium addition, the mixture was heated up to 75-80°C until complete sodium consumption. After the total sodium solubilization, reactor was cooled under inert atmosphere at 15±5°C in cold water bath. A solution composed of 410.5 g of 1-(4-fluorophenyl)-4-(morpholin-4-

yl)butan-1-one (free base) and 460 mL (421.82 g; 5.69 mol; 3.48 eq.) of ethyl formate; was slowly added, controlling the flow rate so that the temperature was maintained between 20 and 30°C. The brick-red colored solution obtained was maintained under stirring for at least 6 hours between 20 and 30°C. After confirming total consumption of starting material, by TLC, the mixture was cooled to 5-15°C and it was slowly added 2.8 L of water, under stirring. The hydro-organic phase was added with 306 g (5.72 mol; 3,5 eq.) of solid $NH_4Cl$ until pH 9-10. The mixture was extracted with $CH_2Cl_2$ (3 x 600 mL) and the extracts were pooled and washed with 900 mL of water. The organic phase was dried over anhydrous $Na_2SO_4$ for 30 minutes. The suspension was vacuum filtered and the filtrate concentrated at room temperature, yielding a yellow solid residue. The residue was macerated in 250 mL of ether at room temperature and dried at 35°C in an oven with air circulation. It was obtained 292 g of 1-(4-fluorophenyl)-2- (hydroxymethyliden)-4-(morpholin-4-yl)butan-1-one. MS: m/z (rel. intensity) = 251 $[M-CO]^+$, 123, 113, 100 (100%).

(4) The final product free based: 4-{2-[3-(4-fluorophenyl)-1*H*-pyrazol-4-yl]ethyl}morpholine. 145 g of 1-(4-fluorophenyl)-2-(hydroxymethyliden)-4-(morpholin-4-yl)butan-1-one was suspended in 520 mL of ethanol and then were added 65 mL (1.15 mol; 2.2 eq.) of 55% hydrazine hydrate. After completing the addition, the mixture was stirred for 2 h at room temperature. Thereafter, the system was heated up to reflux temperature for 3.5 h, and the reaction was monitored by TLC. After the reaction has been completed, the system was cooled to room temperature and added with 2.1 L of water, under stirring. The product was extracted with $CH_2Cl_2$ (2 x 600 mL). The organic phase was washed twice with water (2 x 400 mL). The phases were let to separate and the organic phase was dried over anhydrous $Na_2SO_4$ for 30 minutes. The suspension was filtered and the filtrate was concentrated until dryness in a rotatory evaporator. It was obtained 147.8 g of 4-{2-[3-(4-fluorophenyl)-1*H*-pyrazol-4-yl]ethyl morpholine, free base, as a yellowish solid which was used as such in the next step. MS: m/z (rel. intensity) = 275 $[M]^+$, 100 (100%).

(5) Final Product: 4-{2-[3-(4-fluorophenyl)-1*H*-pyrazol-4-yl]ethyl}morpholine dihydrochloride monohydrate. The solid obtained, in the step (4) above, was suspended in 2.5 L of acetone. Concentrated HCl (143.5 mL) was added until pH between 0-1. The suspension thus formed was stirred for 0.5 h. The white solid obtained was vacuum filtered and washed with 500 mL of acetone. The solid was dried in an oven at 50°C. It was obtained 164.2 g of 4-{2-[3-(4-fluorophenyl)-1*H*-pyrazol-4-yl]ethyl}morpholine dihydrochloride monohydrate. Mp (DSC) = 113-126°C (Dec.). IR (KBr): $\nu^-$ ($cm^{-1}$) = broadband between 3650 and 2000 with peaks at 3327, 3067, 2943, 2866, 2600, 2469, 1610, 1516, 1439, 1223, 1171, 1124, 1076, 976, 906, 881, 847, 685, 608, 534. $^1$H-NMR (500 MHz, $D_2O$, r.t., TMS): $\delta$ = 8.10 (s, 1H), 7.66 (Dd, J = 8.7 Hz and 5.2 Hz, 2H); 7.37 (t, J = 8.7 Hz, 2H); 4.11 (d, J = 12.8 Hz, 2H); 3.82 (t, J = 12.5 Hz, 2H); 3.49 (D, J = 12.5 Hz, 2H); 3.37 (m, 2H); 3.20 (m, 4H). $^{13}$C-NMR (125 MHz, $D_2O$, r.t., TMS): $\delta$ = 163.4; 165.4; 148.5; 136.5; 133, 4; 133.3; 127.0; 119.4; 119.2; 116.5; 66.6; 59.3; 54.6; 20.8. HRMS (TOF MS ES+) calc. for $[M^+I]^+$: 276,1507; found: 276.1465. KF = 5.20% $H_2O$.

**Example-10**

**4-{2-[3-(4-fluorophenyl)-1,2-oxazol-4-yl]ethyl}morpholine hydrochloride hemihydrate** (C10):

145 g of 1-(4-fluorophenyl)-2-(hydroxymethyliden)-4-(morpholin-4-yl)butan-1-one was suspended in 520 mL of ethanol and subsequently added with 40 g (0.576 mol; 1.1 eq.) of hydroxylamine hydrochloride. After completing the addition, the mixture was stirred for 1 h at room temperature. Thereafter, the system was heated up to reflux temperature for 3 h. After the reaction has been completed, the mixture was concentrated under vacuum untill dryness. The solid residue was macerated in 520 mL of acetone at reflux temperature for 1 h. The heating was turned off and the mixture was cooled to room temperature. The solid was vacuum filtered and dried at 50°C in an oven. It was obtained 98.5 g of the 4-{2-[3-(4-fluorophenyl)-1,2-oxazol-4-yl]ethyl}morpholine hydrochloride hemihydrate. Mp (DSC) = 193.0-194.5°C. IR (KBr): $\nu^-$ ($cm^{-1}$) = 3495, 3416, 3281, 3080, 3046, 2976, 2932, 2870, 2673, 2569, 2463, 1651, 1609, 1514, 1462, 1232, 1086, 980, 903, 841, 725, 679, 611, 521, $^1$H NMR (500 MHz, $D_2O$, r.t., TMS) : $\delta$ = 8.48 (s, 1H), 7.69 (dd, J = 8, 6 Hz and 5.2 Hz, 2H), 7.30 (t, J = 8.6 Hz, 2H), 3.97 (m, 4H), 3.54-3.21 (m, 6H); 3 15 (m, 2H). $^{13}$C-NMR (125 MHz, $D_2O$, r.t., TMS): $\delta$ = 167.4, 166.9, 164.9, 154.7, 131.74, 131.67; 125.22, 125.20, 118.7, 118.5, 111.6, 66.0, 58.0, 54.0; 19.8. HRMS (TOF MS ES+) calc. for $[M^+I]^+$: 277.1352, found: 277.1429 KF = 2.51% $H_2O$.

**Example-11**

**1-{2-[3-(4-fluorophenyl)-1*H*-pyrazol-4-yl]ethyl}-4-methylpiperazine trihydrochloride dihydrate** (CII):

(1) Intermediate-1: 1-(4-fluorophenyl)-4-(4-methylpiperazin-1-yl)butan-1-one. In a three-neck glass reactor, with up

to 6 L overall capacity, fitted with heating to reflux system, a thermometer, and mechanical stirring system, it was added 270 mL (2.43 mol; 1.0 eq.) of 1-methylpiperazine, 286 g (2.7 mol; 110.8 mol%; 2.2 eq.) of sodium carbonate, 36.4 g (0.24 mol; 0.1 eq.) of sodium iodide, 2.4 L of methyl isobutyl ketone (MIBK), and 500 mL (2.95 mol; 1.2 eq.) of 4-chloro-4'-fluorobutyrophenone. The mixture was heated internally between 100-105°C for 3.5 hours, with the reaction being monitored by TLC. After completing the reaction, the medium was allowed to cool between 40-50°C and then added with 1.5 L of MIBK and 1.6 L of water under vigorous stirring, for 15 min. The phases were let to separate; aqueous phase was discarded and organic phase, retained. The organic phase was washed with 1.6 L of water for 15 minutes. The phases were let to separate and the organic phase was dried over anhydrous $Na_2SO_4$ for 30 minutes. The suspension was vacuum filtered and the obtained solid was washed with 600 mL of MIBK. The organic phase was cooled 0-10°C and sufficiently saturated with gaseous HCl. The mixture was stirred at 10-20°C for 1 h until total dihydrochloride precipitation. The solid formed was vacuum filtered and macerated in 2.5 L of acetone for 30 minutes. The solid was vacuum filtered and dried in an oven at 50°C. It was obtained 590 g of 1-(4-fluorophenyl)-4-(4-methylpiperazin-1-yl)butan-1-one dihydrochloride. MS: m/z (rel. intensity) = 264 $[M]^+$, 194, 165, 126 (100%), 123, 113, 95, 84, 70, 56, 42.

(2) Intermediate-1 free base: 1-(4-fluorophenyl)-4-(4-methylpiperazin-1-yl)butan-1-one. In a reactor of 5 L overall capacity, 586.7 g (1.74 mol) of 1-(4-fluorophenyl)-4-(4-methylpiperazin-1-yl)butan-1-one dihydrochloride were dissolved in 2.35 L of water. 3N NaOH (1.28 L; 3.84 mol) was added to obtain a pH in the range between 12-14. The mixture was vigorously stirred for 30 minutes. The oily product was extracted with dichloromethane (2 x 930 mL). The phases were let to separated and the organic phase was washed with water (2 x 470 mL). The phases were again separated and the organic phase was dried over anhydrous $Na_2SO_4$ for 30 minutes. The suspension was filtered and the filtrate concentrated on rotatory evaporator until dryness. The oily residue was dried in an oven at 50°C. After drying has being completed, the material was cooled to crystallize the product. It was obtained 402.9 g of 1-(4-fluorophenyl)-4-(4-methylpiperazin-1-yl)butan-1-one, as free base. Mp = 59.5-60.5°C. MS: m/z (rel. intensity) = 264 $[M]^+$, 194, 165, 126 (100%), 123, 113, 95, 84, 70, 56, 42.

(3) Intermediate-2: 1-(4-fluorophenyl)-2-(hydroxymethyliden)-4-(4-methylpiperazin-1-yl)butan-1-one. In a three-neck glass reactor, with up to 500 mL overall capacity, dried, fitted with magnetic stirring system and nitrogen input to the system, 364 mL of dry ethanol were placed and were added 27 g (1.17 mol; 3.1 eq.) of metallic sodium, previously cut into pieces. Thirty minutes after the complete metallic sodium addition, the reactor was heated up to 75-80°C until complete sodium consumption. After the total sodium solubilization, reactor was cooled at 15±5°C in cold water bath. A solution composed of 100 g (0.378 mol) of 1-(4-fluorophenyl)-4-(4-methylpiperazin-1-yl)butan-1-one (free base), 92 mL (1.139 mol; 3.0 eq.) of ethyl formate, and 100 mL of tetrahydrofuran (THF) was slowly added, using an addition funnel; controlling the flow rate so that the temperature was maintained between 20 and 30°C. The solution was left under stirring for at least 6 hours between 20 and 30°C. After confirming total consumption of starting material, by TLC, the mixture was concentrated under vacuum at room temperature. Residuous was treated with 130 mL of water and 70 mL (1.22 mol; 3.2 eq.) of acetic acid, until pH in the range between 5-6. The solution of 1-(4-fluorophenyl)-2-(hydroxymethyliden)-4-(4-methylpiperazin-1-yl)butan-1-one was used as such in the next step.

(4) Final Product: 1-{2-[3-(4-fluorophenyl)-1H-pyrazol-4-yl]ethyl}-4-methylpiperazine trihydrochloride dihydrate. To the solution obtained, in the step (3) above, 48 mL (0.848 mol; 2.2 eq.) of 55% hydrazine hydrate were added, at a temperature non higher than 30°C. The mixture was stirred for 2 h at room temperature. Thereafter, the mixture was heated up to reflux, and the reaction monitored by TLC. After the reaction has been completed, the system was cooled and 180 mL of water were added. The mixture was alkalinized with 1N NaOH to reach a pH in the range between 10-11. The mixture was stirred for about 20 minutes to give a beige-colored solid. The solid was vacuum filtered and washed with water. It was then suspended in 2.6 L of acetone and added with 130 mL of concentrated HCl with vigorous stirring, until pH 1. The suspension was stirred for 1 h. The solid was vacuum filtered and washed with 300 mL of acetone. The solid was dried in an oven at 50°C. It was obtained 141.6 g of 1-{2-[3-(4-fluorophenyl)-1H-pyrazol-4-yl]ethyl}-4-methylpiperazine trihydrochloride dehydrate. Mp (DSC) = 226-237°C (dec.). IR (KBr): $\nu^-$ ($cm^{-1}$) = broadbands between 3700 and 2000 with peaks in 3354, 3193, 3036, 2994, 2968, 2937, 2619, 2562, 2485, 2449, 1607, 1521, 1447, 1415 1232, 1158, 1087, 959, 862, 826, 727, 607, 548. $^1H$ NMR (500 MHz, $D_2O$, r.t., TMS): $\delta$ = 8.02 (s, 1H); 7.66 (dd, J = 8.8 Hz and 5.3 Hz, 2H); 7.37 (t, J = 8, 8 Hz, 2H) ; 3.91 (m, 4H) ; 3.86 (m, 4H) ; 3, 70-3, 33 (m, 4H); 3.48 (t, J = 8.2 Hz, 2H); 3.22 (t, J = 8.2 Hz, 2H), 3.05 (s, 3H). $^{13}C$ NMR (125 MHz, $D_2O$, r.t., TMS): $\delta$ = 167.4; 165.4; 148.7; 136.5; 133.4; 133.3; 127.2; 119.4; 119.3; 115.9; 59.0; 53.0; 51.6; 45.8; 21.1. MS: m/z (rel. intensity) = 288 $[M]^+$, 113 (100%), 70. HRMS (TOF MS ES+) calc. for $[M^+I]^+$: 289,1823; found: 289.1778. KF = 8.70% $H_2O$.

**Example-12**

**1-ethyl-4-{2-[3-(4-fluorophenyl)-1H-pyrazol-4-yl]ethyl}piperazine trihydrochloride trihydrate** (C12):

(1) Intermediate-1: 4-(4-ethylpiperazin-1-yl)-1-(4-fluorophenyl)butan-1-one dihydrochloride. In a three-neck glass reactor, with up to 1 L overall capacity, fitted with heating to reflux system and magnetic stirring system, it was added 36 mL (0.283 mol) of 1-ethylpiperazin, 60 g (0.566 mol; 200 mol%; 4.0 eq.) of sodium carbonate, 4.4 g (0.029 mol; 0.1 eq.) of sodium iodide, 280 mL of methyl isobutyl ketone (MIBK), and 58 mL (0.342 mol; 1.2 eq.) of 4-chloro-4'-fluorobutyrophenone. The mixture was heated up to reflux temperature for 3.5 h, monitoring the reaction by TLC. After the reaction has been concluded, the medium was allowed to cool to between 40-50°C and water (300 mL) was added under vigorous stirring for 15 min. The phases were let to separate; aqueous phase was discarded and organic phase, retained. The organic phase was washed with 300 mL of water for 15 minutes. The phases were let to separate and the organic phase was treated with activated charcoal for 30 minutes. It was then dried over anhydrous $Na_2SO_4$ for 30 minutes. The suspension was vacuum filtered through Celite. The organic phase was cooled to 0-10°C and sufficiently saturated with gaseous HCl. The mixture was stirred at 10-20°C for 1 h until total dihydrochloride precipitation. The obtained solid was vacuum filtered and was macerated in 160 mL of MIBK for 30 minutes. The solid was then vacuum filtered and dried in an oven at 50°C. It was obtained 75 g of 4-(4-ethylpiperazin-1-yl)-1-(4-fluorophenyl)butan-1-one dihydrochloride. Mp (DSC) = 211-221°C (dec.). IR (KBr) : $^{-}$ ($cm^{-1}$)= broadband between 3650 and 2000 with peaks at 3331, 3282, 3185, 3051, 2981, 2694, 2601, 2543 and 2490, 1684, 1623, 1597, 1506, 1456, 1412, 1297, 1237, 1213, 1162, 1107, 1020, 988, 831, 755, 662, 602. MS: m/z (rel. intensity) = 278 $[M]^+$, 194, 165, 140 (100%), 127, 123, 95, 84, 70, 56, 42.

(2) Intermediate-1 free base: 4-(4-ethylpiperazin-1-yl)-1-(4-fluorophenyl)butan-1-one. In an Erlenmeyer flask of 1 L overall capacity 55 g (0.156 mol) of 4-(4-ethylpiperazin-1-yl)-1-(4-fluorophenyl)butan-1-one duhydrochloride were dissolved in 165 mL of water. 1N NaOH (350 mL; 0.35 mol) was added to reach pH in the range between 12-14. The mixture was vigorously stirred for 30 minutes. The oily product was extracted with 260 mL of dichloromethane. The phases were let to separate and the organic phase was washed with water (2 x 130 mL). The phases were again separated and the organic phase was dried over anhydrous $Na_2SO_4$. The suspension was filtered and the filtrate concentrated on rotatory evaporator until dryness. The oily residue was dried in an oven at 50°C. After drying been completed, the material was cooled to crystallize the product. It was obtained 42 g of 4-(4-ethylpiperazin-1-yl)-1-(4-fluorophenyl)butan-1-one, as free base. MS: m/z (rel. intensity) = 278 $[M]^+$, 194, 165, 140 (100%), 127, 123, 95, 84, 70, 56, 42.

(3) Intermediate-2: 4-(4-ethylpiperazin-1-yl)-1-(4-fluorophenyl)-2-(hydroxymethyliden)butan-1-one. In a three-neck glass reactor, with up to 500 mL overall capacity, dried, fitted with magnetic stirring system and nitrogen input to the system, 150 mL of dry ethanol were placed and added 11.2 g (0.487 mol; 3.2 eq.) of metallic sodium, previously cut into pieces. Thirty minutes after the complete addition metallic sodium, the reactor was heated up to 75-80°C until complete sodium consumption. After the total sodium solubilization, reactor was cooled at 15±5°C in cold water bath. A solution composed of 42 g (0.151 mol) of 4-(4-ethylpiperazin-1-yl)-1-(4-fluorophenyl)butan-1-ona (free base), and 39 mL (0.482 mol; 3.2 eq.) of ethyl format was slowly added, using an addition funnel; controlling the flow rate so that the temperature was maintained between 20 and 30°C. The solution was kept under stirring for at least 6 hours between 20 and 30°C. After confirming total consumption of starting material, by TLC, the mixture was diluted in 55 mL of water and treated with 29 mL (0.506 mol; 3.35 eq.) of acetic acid, until pH in the range between 5-6. The solution of 4-(4-ethylpiperazin-1-yl)-1-(4-fluorophenyl)butan-1-ona was used as such in the next step.

(4) Final Product: 1-ethyl-4-{2 - [3-(4- fluorophenyl)-1H-pyrazol-4-yl]ethyl}piperazine trihydrochloride trihydrate. To the solution obtained, in the step (3) above, 18.8 mL (0.332 mol; 2.2 eq.) of 55% hydrazine hydrate were added, at a temperature non higher than 30°C. The mixture was stirred for 2 h at room temperature. Thereafter, the system was heated up to reflux during 4 h, and the reaction monitored by TLC. After the reaction has been completed, the system was cooled and 120 mL of water were added. The mixture was alkalinized to pH in the range between 10-11 with 1N NaOH. The mixture was stirred for about 2 h. The obtained solid was vaccum filtered and washed with water. Then the solid was macerated in water under vigorous stirring for 1 h and vaccum filtered. It was then suspended in 52 mL of concentrated HCl under vigorous stirring, until reach pH 1. The suspension was stirred for 1 h. The solid was vacuum filtered and washed with 100 mL of acetone. The solid was dried in an oven at 50°C. It was obtained 50.4 g of 1-ethyl-4-{2-[3-(4-fluorophenyl)-1*H*-pyrazol-4-yl]ethyl piperazine trihydrochloride trihydrate. Mp (DSC) = 226-237°C (dec.). IR (KBr): $\nu^{-}$ ($cm^{-1}$) = broadband between 3700 and 2000 with peaks at 3393, 3057,

2982, 2924, 2640, 2552, 2444, 1610, 1506, 1445, 1342, 1234, 1165, 1111, 1014, 968, 912, 843, 725, 685, 606. $^1$H-NMR (500 MHz, $D_2O$, r.t., TMS) : δ = 8.08 (s, 1H); 7.65 (dd, J = 8.6 Hz and 5.2 Hz, 2H) ; 7.37 (t, J = 8, 6 Hz, 2H); 3.91 (m, 4H); 3.50 (m, 6H); 3.38 (q, J = 7.6 Hz, 2H); 3.23 (t, J = 8.1 Hz, 2H); 1.37 (t, J = 7, 3 Hz, 3H). $^{13}$C-NMR (125 MHz, $D_2O$, r.t., TMS): δ = 167.2; 165.2; 148.8; 136.5; 133.3; 133.2; 127.7; 119.4; 119.2; 115.7; 59.1; 55.4; 51.6; 50.8; 21.2; 11.5. MS: m/z (rel. intensity) = 302 $[M]^+$ 127 (100%), 84.70. HRMS (TOF MS ES+) calcd for $[M^+I]^+$: 303.1979; found: 303.1936. KF = 12.65% $H_2O$.

**Example-13**

**4-(4-chlorophenyl)-1-{2-[3-(4-fluorophenyl)-1*H*-pyrazol-4-yl]ethyl}piperidin-4-ol** (C13):

(1) Intermediate-1: 4-[4-(chlorophenyl)-4-hydroxypiperidin-i-yl]-1-(4-fluorophenyl)-2-(hydroxymethyliden)butan-1-one. In a three-neck glass reactor, with up to 1 L overall capacity, dried, fitted with mechanical stirring system and nitrogen input to the system, 250 g (0.665 mol) of 4-[4-(4-chlorophenyl)-4-hydroxypiperidin-1-yl]-1-(4-fluorophenyl)butan-1-one (commercially available; mp = 150-153°C)were dissolved in 2.5 L of tetrahydrofuran. To this solution, 188 mL (2.327 mol; 3.5 eq.) of ethyl formate were added. After total solubilization, the reactor was cooled to 10±5°C in water bath. It was added 180 g (1.604 mol; 2.41 eq.) of potassium tert-butoxide, in a controlled way such the temperature was maintained between 10 and 20°C. The mixture was maintained under stirring between 20 and 30°C for 4 h, and the reaction was monitored by TLC. After confirming total consumption of starting material, by TLC, the mixture was treated with a solution of 125 g $NH_4Cl$ dissolved in 625 mL $H_2O$, until pH 9-10, and this mixture was kept under stirring for 1 h at room temperature. The phases were let to separate and the organic phase was washed with a solution of 200 g of NaCl in 600 mL of water. The organic phase was dried over anhydrous $Na_2SO_4$ for 0.5 h. The suspension was vacuum filtered and the filtrate concentrated under reduced pressure at room temperature, until the product begins to precipitate. Then 2.5 L of ether were added and the mixture was maintained under stirring for 1 h. The obtained solid was vacuum filtered and dried in an oven at a temperature between 40-45°C. The product can be macerated in ether or, alternatively, with a mixture of acetone/ether. It was obtained 210 g of 4-[4-(chlorophenyl)-4-hydroxypiperidin-1-yl]-1-(4-fluorophenyl)-2-(hydroxymethyliden)butan-1-one. Mp (DSC) = 211-214°C. IR (KBr): $\nu^-$ ($cm^{-1}$) = broadband between 3650 and 2000, 3072, 1573, 1508, 1481, 1400, 1375, 1368, 1335, 1312, 1258, 1217, 1118, 992, 962, 847, 823, 766. $^1$H-NMR (400 MHz, MeOH-$d_4$, r.t., TMS): δ = 8.64 (s, 1H), 7.55 (d, J = 8.6 Hz, 2H); 7.46 (dd, J = 8.6 Hz and 5.6 Hz, 2H), 7.37 (d, J = 8.6 Hz, 2H), 7.12 (t, J = 8.6 Hz, 2H), 3.55 (m, 2H), 3.39 (td, J = 12.3 Hz and 1.9 Hz, 2H), 3.16 (m, 2H), 2.84 (m, 2H), 2.32 (dt, J = 14.6 Hz and 4.5 Hz, 2H), 1.96 (m, 2H). $^{13}$C-NMR (100 MHz, MeOH-$d_4$, r.t., TMS): δ = 195.5, 187.8, 166.0, 163.5, 147.3, 139.01, 138, 97, 134.2, 132.0, 131.9, 129.5, 127.6; . 115.8, 115.6, 115.5, 69.6, 60.6, 50.3, 37.2, 30.7. MS (ES+) : 404.6 $[M+1]^+$.

(2) Final product : 4-(4-chlorophenyl)-1-{2-[3-(4-fluorophenyl)-1*H*-pyrazol-4-yl] ethyl}piperidin-4-ol. In a flask of 500 mL overall capacity 55 g (0.136 mol) of 4-[4-(chlorophenyl)-4-hydroxypiperidin-1-yl]-1-(4-fluorophenyl)-2-(hydroxymethyliden)butan-1-one were suspended in 275 mL of ethanol. To this suspension, 16.5 mL (0.339 mol; 2.5 eq.) of 55% hydrazine hydrate were slowly added, such as the temperature was not higher than 30°C. The mixture was stirred at room temperature for 18 h. After the reaction has been completed, it was added 275 mL of ethyl acetate. The mixture was washed with water (3 x 150 mL). The phases were let to separate and the organic phase was treated with activated charcoal for 30 minutes. It was then dried over anhydrous $Na_2SO_4$ for 30 minutes. The suspension was filtered through Celite and the filtrate was concentrated under vacuum until dryness. The residue was suspended in 155 mL of acetone and stirred for 1 h at room temperature. The white solid obtained was vacuum filtered and washed with acetone. The product was macerated in 155 mL of acetone at reflux temperature for 1 h. It was vacuum filtered and dried in an oven at 50°C. It was obtained 32.85 g of 4-(4-chlorophenyl)-1-{2-[3-(4-fluorophenyl)-1*H*-pyrazol-4-yl]ethyl}piperidin-4-ol. Mp (DSC) = 142-144°C. IR (KBr): $\nu^-$ ($cm^{-1}$) = 3570, 3136, 2925, 2818, 2674, 1603, 1524, 1492, 1450, 1374, 1311, 1227, 1156, 1133, 1096, 1044, 1012, 992, 917, 839, 821, 742, 604, 540. $^1$H-NMR (400 MHz, $CDCl_3$, r.t., TMS) : δ = 7.50 (dd, J = 8.8 Hz and 5.3 Hz, 2H) ; 7.47 (s, 1H) ; 7.43 (d, J = 8.8 Hz, 2H); 7.30 (d, J = 8.8 Hz, 2H); 7.09 (t, J = 8.8 Hz, 2H); 2.84 (m, 4H) ; 2.64 (m, 2H) ; 2.51 (t, J = 11.3 Hz, 2H) ; 2.14 (td, J = 13.7 Hz and 4.4 Hz, 2H); 1.73 (dd, J = 14.1 Hz and 2.2 Hz, 2H). $^{13}$C-NMR (100 MHz, $CDCl_3$, r.t., TMS): δ = 163.7; 161.2; 146.9; 132.7; 129.5; 129.4; 128.4; 126.2; 115.9; 115.7; 115.5; 71.0; 59.3; 49.3; 38.2; 21.7. HKMS (TOF MS ES+) calc. for $[M^+I]^+$: 400,1586; found: 400.1731. KF = 0.42% $H_2O$.

**Example-14**

**4-(4-chlorophenyl)-1-{2-[3-(4-fluorophenyl)-1H-pyrazol-4-yl]ethyl}piperidin-4-ol dihydrochloride monohydrate** (C14):

1 g of 4-(4-chlorophenyl)-1-{2-[3-(4-fluorophenyl)-1*H*-pyrazol-4-yl]ethyl}piperidin-4-ol was converted to its dihydrochloride monohydrate through its treatment with 3 equivalent of concentrated HCl in isopropanol. Mp (DSC) = polymorphic mixture: 190-202°C and 206-213°C. KF = 4.16%. IR (KBr) : $\nu^-$ ($cm^{-1}$) = broadband between 3650 and 2000 with peaks in 3480, 3410, 3124, 3067, 3007, 2974, 2934, 2672, 2642, 2613, 2488, 2407, 1607, 1520, 1486, 1436, 1403, 1244, 1166, 1099, 1037, 1013 , 985, 958, 848.

**Example-15**

**4-(4-chlorophenyl)-1-{2-[3-(4-fluorophenyl)-1,2-oxazol-4-yl] ethyl}piperidin-4-ol hydrochloride** (C15):

In a flask of 500 mL overall capacity 35 g (0.866 mmol) of 4-[4-(chlorophenyl)-4-hydroxypiperidin-1-yl]-1-(4-fluorophenyl)-2-(hydroxymethyliden)butan-1-one were suspended in 225 mL of ethanol. To this suspension it was added 3 g (0.907 mmol; 1.05 eq.) of hydroxylamine hydrochloride. After the addition has been completed, the mixture was stirred at room temperature for 1 h. The mixture was then heated up to reflux for 2 h. After the reaction has been concluded, the mixture was cooled to 50°C and added with 350 mL of acetone. The mixture was allowed to cool to room temperature under stirring. The obtained solid was vacuum filtered, washed with acetone and dried in an oven at 50°C. It was obtained 28.9 g of 4-(4-chlorophenyl)-1-{2-[3-(4-fluorophenyl)-1,2-oxazol-4-yl] ethyl}piperidin-4-ol hydrochloride, as a pale yellow solid. Mp (DSC) = 222-226°C (dec.). IR (KBr): $\nu^-$ ($cm^{-1}$) = 3366, 3074, 2926, 2540, 2419, 1603, 1506, 1458, 1229, 1152, 1094, 1042, 974, 835, 725, 681, 609, 544. $^1$H-NMR (500 MHz, DMSO-$d_6$, r.t., TMS): $\delta$ = 10.56 (bs, 1H), 8.70 (s, 1H), 7.87 (dd, J = 8.7 Hz and 5, 4 Hz, 2H), 7.50 (d, J = 8.7 Hz, 2H), 7.45 (d, J = 8.6 Hz, 2H), 7.43 (t, J = 8.8 Hz , 2H), 5.64 (s, 1H), 3.53 (d, J = 10.5 Hz, 2H), 3.48-3.34 (signal overlapped by HOD signal, 2H), 2.34 (t, J = 13.6 Hz, 2H), 1.84 (d, J = 13.6 Hz, 2H). HRMS (TOF MS ES+) calc. for $[M^{+}I]^+$: 401.1427, found: 401.1582. KF = 0.22% $H_2O$.

**Example-16**

**4-(4-chlorophenyl)-4-hydroxy-1-{2-[4-(4-fluorophenyl)pyrimidin-5-yl]ethyl}piperidinium acetate** (C16):

In a flask of 500 mL overall capacity, 35 g (0.867 mol) of 4-[4-(clorophenyl)-4-hydroxypiperidin-1-il]-1-(4-fluorophenyl)-2-(hydroxymethyliden)butan-1-ona were suspended in 175 mL of ethanol. Then 30 g (0.288 mol; 3.3 eq.) of formamidine acetate were added. After addition has been completed, the mixture was stirred at room temperature for 1 h and then it was heated up to reflux for 4 h. After the reaction has been concluded, the mixture was cooled to room temperature. The solid was vacuum filtrated and crystalized from 75 mL of ethanol at reflux temperature for 1 h. The filtrated was vacuum filtrated and washed with ether. It was obtained 19.2 g of 4-(4-chlorophenyl)-4-hydroxy-1-{2-[4-(4-fluorophenyl)pyrimidin-5-il]ethyl}piperidinium acetate. Mp (DSC) = polymorphic mixture 178-189°C. IR (KBr): $\nu$- ($cm^{-1}$) = broadbands between 3650 and 2650 with peaks in 3335, 3117, 2965, 1666, 1595, 1549, 1404, 1342, 1296, 1223, 1130, 970, 837, 772, 706, 648, 602, 548, 484. $^1$H-NMR (500 MHz, DMSO-$d_6$, r.t, TMS): $\delta$ = 7.51 (s, 1H); 7.49 (s, 1H); 7.40-7.34 (m, 4H); 7.21 (t, *J* = 8.8 Hz, 2H); 6.97-6.90 (m, 2H); 6.87 (s, 1H); 4.93 (s, 1H); 2.78 (m, 2H); 2.51 (s, 3H); 2.50-2.38 (m, 4H); 2.32 (m, 2H); 1.90 (m, 2H); 1.59 (d, *J* = 12.3 Hz, 2H). HRMS (TOF MS ES+) calc. for $[M+1]^+$: 412.1586; found: 412.1745. KF = 0.53% $H_2O$.

**Example-17**

**Ethyl 1-{2-[3-(4-fluorophenyl)-1,2-oxazol-4-yl]ethyl}-4-phenylpiperidine-4-carboxylate hydrochloride (C17):**

(1) Intermediate-1: Ethyl 1-[4-(4-fluorophenyl)-4-oxobutyl]-4-phenylpiperidine-4-carboxylate hydrochloride. In a three-neck glass reactor, with up to 2 L overall capacity, fitted with heating to reflux system and magnetic stirring system, it was added 200 g (0.757 mol) of 4-phenylpiperidin-4-ethyl carboxylate hemicarbonate (commercially available), 182 g (1.717 mol; 227 mol%; 4.5 eq.) of sodium carbonate, 13 g (0.087 mol; 0.11 eq.) of sodium iodide, 120 mL of methyl isobutyl ketone, and 175 mL (1.032 mol; 1.36 eq.) of 4-chloro-4'-fluorobutyrophenone. The mixture was heated up to reflux, and the reaction was monitored by TLC. After the reaction has been completed, the medium was allowed to cool to 50-60°C and 1 L of water was added, with vigorous stirring for 15 min. The phases were let

to separate; aqueous phase was discarded and organic phase, retained. The organic phase was washed with 500 mL of water for 15 minutes. The phases were separated and the organic phase was dried over anhydrous $Na_2SO_4$ for 30 minutes. The suspension was vacuum filtered. The organic phase was cooled 0-10°C and sufficiently saturated with gaseous HCl. The mixture was stirred at 10-20°C for 1 h until total hydrochloride precipitation. The obtained solid was vacuum filtered and macerated in methyl tert-butyl ether. The solid was filtered and dried in an oven at 50°C. It was obtained 272.5 g of 1-[4-(4-fluorophenyl)-4-oxobutyl]-4-phenyl-piperidin-4-ethyl carboxylate hydrochloride.

(2) Intermediate-1 free base: Ethyl 1-[4-(4-fluorophenyl)-4-oxobutyl]-4-phenyl-piperidin-4-carboxylate. In a flask of 5 L overall capacity 272.5 g (0.628 mol) of 1-[4-(4-fluorophenyl)-4-oxobutyl]-4-phenylpiperidin-4-ethyl carboxylate were dissolved in a mixture of 1.52 L of ethylene chloride and 1.52 L of aqueous solution of 5% $NaHCO_3$. The mixture was vigorously stirred for 30 minutes. The phases were let to separate. The organic phase was washed with water (2 x 0.5 L) and dried over anhydrous $Na_2SO_4$. It was then filtered and concentrated under vacuum. The residue was treated with 800 mL of hexane under stirring. The obtained solid was vacuum filtered and dried in an oven at 45°C. It was obtained 216 g of 1-[4-(4-fluorophenyl)-4-oxobutyl]-4-phenyl-piperidin-4-ethyl carboxylate, free base, as a pale yellow crystalline solid.

(3) Intermediate-2: Ethyl 1-{3-[(4-fluorophenyl)carbonyl]-4-hydroxybut-3-en-1-yl}-4-phenyl-piperidin-4-carboxylate. In a three-neck glass reactor, with up to 3 L overall capacity, dried, fitted with efficient magnetic stirring system and nitrogen input to the system, 0.603 L of dry ethanol were placed and added 44.86 g (1.950 mol; 3.6 eq.) of metallic sodium, previously cut into pieces. Thirty minutes after the complete addition metallic sodium, the reactor was heated up to 75-80°C until complete sodium consumption. After the total sodium solubilization, reactor was cooled at 15±5°C in cold water bath. A solution composed of 215 g (0.541 mol) of 1-[4-(4-fluorophenyl)-4-oxobutyl]-4-phenyl-piperidin-4-ethyl carboxylate (free base) 140 mL (1.733 mol; 3.2 eq.) of ethyl formate, and 1.30 L of tetrahydrofuran was slowly added, using an addition funnel; controlling the flow rate so that the temperature was maintained between 20 and 30°C. The solution was maintained under stirring for at least 20 hours at room temperature. After confirming total consumption of starting material, the mixture was concentrated under vacuum at room temperature. The residue was diluted in 1.5 L of water. The aqueous phase was treated with 83 g (1.55 mol; 2.67 eq.) of solid $NH_4Cl$ to reach a pH in the range between 9-10. The aqueous phase was extracted with $CH_2Cl_2$ (3 x 0.5 L) and the extracts were concentrated at room temperature, yielding a yellow solid. The solid was macerated in 400 mL of ether. It was vacuum filtered and washed with ether. It was obtained 173.7 g of 1-{3-[(4-fluorophenyl) carbonyl]-4-hydroxybut-3-en-1-yl}-4-phenyl-piperidin-4-ethyl carboxylate cetoenol, as a slightly yellowish solid.

(4) Final Product: Ethyl 1-{2-[3-(4-fluorophenyl)-1,2-oxazol-4-yl]ethyl}-4-phenylpiperidine-4-carboxylate hydrochloride.

100 g of ethyl 1-{3-[(4-fluorophenyl)carbonyl]-4-hydroxybut-3-en-1-yl}-4-phenylpiperidine-4-carboxylate were suspended in 400 mL of ethanol. It was slowly added 18 g (0.259 mol; 1.17 eq.) of hydroxylamine hydrochloride. The formed obtained solution was stirred at room temperature for 1 h and then heated up to 50-55°C for 2 h, monitoring the reaction by TLC. After the reaction has been concluded, the solution was concentrated until 50% volume. The product was precipitated by adding 600 mL of water. The white solid obtained was vacuum filtered and washed with water. It was purified by crystallization from ethanol/water. It was obtained 129.5 g of ethyl 1-{2-[3-(4-fluorophenyl)-1,2-oxazol-4-yl]ethyl}-4-phenylpiperidine-4-carboxylate hydrochloride. Mp (DSC) = polymorphic mixture: 142-150°C and 158-160°C (dec.). IR (KBr): $\bar{\nu}$ ($cm^{-1}$) = 3435, 3063, 2978, 2937, 2650, 2548, 2490, 2438, 1726, 1603, 1508, 1460, 1381, 1306, 1232, 1148, 1020, 932, 841 , 729, 694, 603. $^1$H-NMR (500 MHz, DMSO-$d_6$, r.t., TMS) : $\delta$ = 8.61 (s, 1H); 7.78 (dd, J = 8.8 Hz and 5.4 Hz, 2H); 7.40 (t, J = 8.8 Hz, 2H); 7.35 (m, 4H); 7.26 (m, 1H), 4.06 (q, J = 7.1 Hz, 2H), 2.83 (d, J = 10.9 Hz, 2H), 2.76 (t, J = 7.3 Hz, 2H), 2.50 (m, 2H), 2.42 (d, J = 12.7 Hz, 2H), 2.09 (t, J = 11.1 Hz, 2H), 1 , 79 (t, (J = 11.3 Hz, 2H), 1.10 (t, J = 7.1 Hz, 3H). $^1$H-NMR (500 MHz, $CDCl_3$, r.t., TMS): $\delta$ = 8.27 (s, 1H), 7.70 (dd, J = 8.6 Hz and 5.2 Hz, 2H), 7.38 (d, J = 7.5 Hz, 2H), 7.34 ( t, J = 7.5 Hz, 2H), 7.25 (m, 2H), 7.18 (t, J = 8.5 Hz, 2H), 4.13 (q, J = 7.0 Hz, 2H), 2.90 (d, J = 10.9 Hz, 2H), 2.80 (t, J = 7.5 Hz, 2H), 2.60 (m, 2H), 2.58 (t, J = 7.5 Hz, 2H), 2.23 (t, J = 11.2 Hz, 2H), 1.96 (t, J = 11.2 Hz, 2H), 1.18 (t, J = . 7.0 Hz, 3H). HRMS (TOF MS ES+) calc. for $[M+1]^+$: 423.2078, found: 423.2133. KF = 0.10% $H_2O$.

**Example-18**

**Ethyl 1-{2-[3-(4-fluorophenyl)-1*H*-pyrazol-4-yl]ethyl}-4-phenylpiperidine-4-carboxylate (C18):**

70 g (0.164 mol) of ethyl 1-{-3-[(4-fluorophenyl)carbonyl]-4-hydroxybut-3-en-1-yl}-4-phenylpiperidine-4-carboxylate were suspended in 140 mL of ethanol. To this suspension 20.5 mL (0,362 mol. 2.2 eq.) of hydrazine hydrate were

slowly added. The solution obtained was stirred at 50-55°C for 1.5 h, monitoring the reaction by TLC. After the reaction has been completed, the system was cooled to room temperature. The solid formed was vacuum filtered, letting it leach sufficiently. It was washed with water and dried in an oven at 50°C. It was obtained 59 g of ethyl 1-{2-[3-(4-fluorophenyl)-1H-pyrazol-4-yl]ethyl}-4-phenylpiperidine-4-carboxylate, as a bright white crystalline solid. Mp (DSC) = 138.0-139.5°C. IR (KBr): $\nu^-$ ($cm^{-1}$) = 3430, 3132, 2978, 2929, 2904, 2819, 2774, 2714, 2671, 1721, 1604, 1524, 1450, 1313, 1222, 1190, 1158, 1124, 1103, 1021, 923, 842, 814, 742, 697, 604, 537. $^1$H-NMR (500 MHz, $CDCl_3$, r.t., TMS): $\delta$ = 10.48 (bs, 1H), 7.50 (m, 3H), 7.38 (m, 2H), 7.32 (t, J = 7.7 Hz, 2H), 7.24 (m, 1H), 7.11 (t, J = 8, 6 Hz, 2H), 4.12 (q, J = 7, 1 Hz, 2H), 2 89 (d, J = 10.8 Hz, 2H), 2.79 (t, J = 7.5 Hz, 2H), 2.58 (d, J = 13.0 Hz, 2H), 2.54 (dd, J = 16.0 Hz and 7.5 z, 2H), 2.19 (t, J = 11.3 Hz, 2H), 1.96 (t, J = 11.3 Hz, 2H). 1.17 (t, J = 7.1 Hz, 3H). HRMS (TOF MS ES+) calc. for $[M+1]^+$: 422.2238, found: 422.2176. KF = 0.20% $H_2O$.

**Claims**

1. A heteroaromatic compound **characterized by** the fact of being of formula (I)

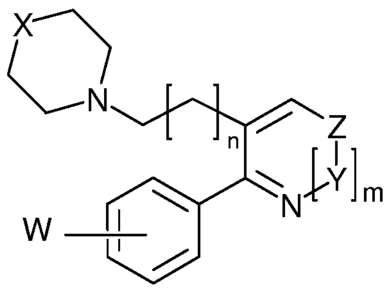

(I)

or a pharmaceutically acceptable salt thereof, wherein:

**n** is 0 or 1;
**m** is 0 or 1;
**Z** is O, -N or NH;
**Y** is CR or N, wherein **R** is selected from H, $C_{1-6}$alkyl, SH, S-$C_{1-6}$-alkyl, OH, O-$C_{1-6}$-alkyl, chlorine, bromine and $NH_2$;
**X** is selected from CH$\mathbf{R_1}$, C$\mathbf{R_1R_2}$, O, S and **NR3,** wherein $\mathbf{R_1}$ is selected from H, $C_{1-6}$-alkyl, $C_{3-6}$-cycloalkyl, aryl, substituted-aryl, CN, OH, halogen, $CO_2R_4$ and $CONR_4R_5$; $\mathbf{R_2}$ is selected from CN, OH, halogen, $CO_2R_4$ and $CONR_4R_5$; $\mathbf{R_3}$ is selected from H, $C_{1-6}$-alkyl, $C_{3-6}$-cycloalkyl, aryl, substituted-aryl and heteroaryl; $\mathbf{R_4}$ and $\mathbf{R_5}$ are independently selected from H, $C_{1-6}$-alkyl, $C_{3-6}$-cycloalkyl, aryl, substituted-aryl or heteroaryl; and **W** is H or halogen.

2. The **compound** according to claim 1, **characterized by** the fact that:

**n** is 1;
**m** is 1;
**Z** is -N;
**Y** is CR or N, wherein **R** is selected from H, $C_{1-6}$-alkyl, SH, S-$C_{1-6}$-alkyl, OH, O-$C_{1-6}$-alkyl, chlorine, bromine and $NH_2$.
**X** is selected from $CHR_1$, $CR_1R_2$, O, S and $NR_3$; wherein $\mathbf{R_1}$ is selected from H, $C_{1-6}$-alkyl, $C_{3-6}$-cycloalkyl, aryl, substituted-aryl, CN, OH, halogen, $CO_2R_4$ and $CONR_4R_5$; $\mathbf{R_2}$ is selected from CN, OH, halogen, $CO_2R_4$ and $CONR_4R_5$; $\mathbf{R_3}$ is selected from H, $C_{1-6}$-alkyl, $C_{3-6}$-cycloalkyl, aryl, substituted-aryl, and heteroaryl; $\mathbf{R_4}$ and $\mathbf{R_5}$ are independently selected from H, $C_{1-6}$-alkyl, $C_{3-6}$-cycloalkyl, aryl, substituted-aryl and heteroaryl; and **W** is H or halogen.

3. A **compound** according to claim 1, **characterized by** the fact that:

**n** is 1;
**m** is 0;
**Z** is selected from O, NH or -N;

**X** is selected from $CHR_1$, $CR_1R_2$, O, S and $NR_3$, wherein $\mathbf{R_1}$ is selected from H, $C_{1-6}$-alkyl, $C_{3-6}$-cycloalkyl, aryl, substituted-aryl, CN, OH, halogen, $CO_2R_4$ and $CONR_4R_5$; $\mathbf{R_2}$ is selected from CN, OH, halogen, $CO_2R_4$ and $CONR_4R_5$; $\mathbf{R_3}$ is selected from H, $C_{1-6}$-alkyl, $C_{3-6}$-cycloalkyl, aryl, substituted-aryl and heteroaryl; $\mathbf{R_4}$ and $\mathbf{R_5}$ are independently selected from H, $C_{1-6}$-alkyl, $C_{3-6}$-cycloalkyl, aryl, substituted-aryl and heteroaryl; and **W** is H or halogen.

**4.** The compound according to claims 1 to 3, **characterized by** the fact that W is fluorine.

**5.** The compound according to claim 1, **characterized by** being selected from the group consisting of:

1-{2-[3-(4-fluorophenyl)-1*H*-pyrazol-4-yl]ethyl}piperidine;
1-{2-[3-(4-fluorophenyl)-1,2-oxazol-4-yl]ethyl}piperidine;
1-{2-[3-(4-fluorophenyl)-1*H*-pyrazol-4-yl]ethyl}-4-methylpiperidine;
1-{2-[3-(4-fluorophenyl)-1,2-oxazol-4-yl]ethyl}-4-methylpiperidine;
4-{2-[3-(4-fluorophenyl)-1H-pyrazol-4-yl]ethyl}morpholine;
4-{2-[3-(4-fluorophenyl)-1,2-oxazol-4-yl]ethyl}morpholine;
1-{2-[3-(4-fluorophenyl)-1*H*-pyrazol-4-yl]ethyl}-4-methylpiperazine;
1-ethyl-4-{2-[3-(4-fluorophenyl)-1H-pyrazol-4-yl]ethyl}piperazine;
4-(4-chlorophenyl)-1-{2-[3-(4-fluorophenyl)-1*H*-pyrazol-4-yl]ethyl}piperidin-4-ol;
4-(4-chlorophenyl)-1-{2-[3-(4-fluorophenyl)-1,2-oxazol-4-yl]ethyl}piperidin-4-ol;
Ethyl 1-{2-[3-(4-fluorophenyl)-1,2-oxazol-4-yl]ethyl}-4-phenylpiperidine-4-carboxylate;
Ethyl 1-{2-[3-(4-fluorophenyl)-1*H*-pyrazol-4-yl]ethyl}-4-phenylpiperidine-4- carboxylate;
4-(4-fluorophenyl)-5-[2-(piperidin-1-yl)ethyl]pyrimidine;
4-(4-chlorophenyl)-1-{2-[4-(4-fluorophenyl)pyrimidin-5-yl] ethyl}piperidin-4-ol.

**6.** The compound according to claims 1 to 3, **characterized by** the fact of being in the form of prodrugs, polymorphs, hydrates, solvates, tautomers, individual isomers or mixtures of isomers.

**7.** The compound according to claim 6, **characterized by** the fact wherein the prodrugs are carbonates, carbamates, phosphates, phosphonates, glycosides, sulfates, sulfonates, ethers or esters.

**8.** The compound according to claims 1 to 3, **characterized by** the fact that it is useful in the treatment and prevention of acute and chronic pain in human beings or animals.

**9.** The compound according to claim 8, **characterized by** the fact the acute pain is neurogenic or inflammatory.

**10.** The compound according to claim 8, **characterized by** the fact that chronic pain is neuropathic pain type hyperalgesia or allodynia.

**11.** A pharmaceutical composition, **characterized by** the fact it comprises an effective amount of at least one compound according to claim 1, and one or more pharmaceutically acceptable excipients.

**12.** The pharmaceutical composition according to claim 11, **characterized by** the fact it comprises from 0.1% to 99% w/w of a compound according to claim 1.

**13.** The composition according to claim 11, **characterized by** the fact of being administered to human beings or animals by oral, sublingual, nasal, rectal, intragengival, intravenous, intramuscular, intraarticular, subcutaneous, inhalatory, transdermal, topical, spinal subarachnoid or epidural spinal route.

**14.** The composition according to claim 13, **characterized by** the fact it is preferably administered by parenteral or oral route.

**15.** A process for preparing a compound according to claim 2, **characterized by** comprising a reaction of intermolecular cycle condensation of a ketoenamine of formula **(V)** or, alternatively, a cetoenol of formula **(VI),** wherein X and W are as defined in claim 2 and Ra and Rb are independently $C_{1-6}$-alkyl, with an ambident nucleophile selected from the group consisting of hydrazine hydrate, hydroxylamine hydrochloride, sodium azide, amidines, guanidine, O-methylisourea and S-methylisothiourea.

**16.** The process for the preparation of a compound according to claim 2, **characterized by** comprising an aliphatic bimolecular nucleophilic reaction of a heteroarene of formula **(X)** with a 6-membered cyclic amine of formula **(VIII),** wherein W, X, Y, Z and m are as defined in claim 2.

**17.** The process according to claims 15 or 16, **characterized by** the fact of being carried out in presence of a solvent selected from the group consisting of $C_{1-6}$-alcohol, ether, ketone, glycol ether, aromatic hydrocarbon, amide, 1,2-dimethoxyethane, acetonitrile, water and mixtures thereof.

**18.** The process according to claims 15 or 16, **characterized by** the fact of being carried out in presence of a solvent selected from the group consisting of methanol, ethanol and isopropanol.

**19.** The process according to claims 15 or 16, **characterized by** the fact the reaction is carried out at a temperature ranging between 25°C and the reflux temperature of the solvent employed, preferably between 50°C and the reflux temperature of the solvent, more preferably at the reflux temperature of the solvent.

**20.** The process according to claims 15 or 16, **characterized by** the reaction is carried out in presence of an acid or a base as catalyst, being the base selected from the group consisting of triethylamine, diisopropylamine, pyridine, sodium or potassium bicarbonate, sodium or potassium carbonate, sodium ethoxide, sodium methoxide and sodium amide; and being the acid selected from the group consisting of hydrochloric acid, sulfuric acid, acetic acid and p-toluenesulfonic acid.

**21.** A process for preparing a compound according to claim 2, **characterized by** comprising a Borch reductive amination reaction of a compound of formula **(XIII)** with a 6-membered cyclic amine of formula **(VIII),** where W, X, Y, Z, and m are as defined in claim 2, in the presence of a reducing agent and an inert solvent.

**22.** A process for preparing a compound according to claim 3, **characterized by** comprising a Borch reductive amination reaction of a compound of formula **(XXV)** with a 6-membered cyclic amine of formula **(VIII),** wherein W, X , Y, Z, and m are as defined in claim 3, in the presence of a reducing agent and an inert solvent.

**23.** The process according to claims 21 and 22, **characterized by** the fact the reducing agent is selected from the group consisting of sodium triacetoxyborohydride, sodium cyanoborohydride, sodium borohydride, borane, palladium on carbon and a hydrogen source.

**24.** The Process according to claims 21 and 22, **characterized by** the fact the inert solvent is selected from the group consisting of hexane, petroleum ether, benzene, toluene, xylene, chlorinated hydrocarbons, alcohols, ethers, glycol ethers, ketones, amides, nitriles, sulfoxides, nitro compounds, esters, and mixtures thereof.

**25.** Use of a compound according to claims 1 to 3, **characterized by** the fact of being for the manufacture of a medicament to treat or prevent acute or chronic pain in human beings or animals.

**26.** A combination **characterized by** comprising a compound according to claim 1 and one or more active compounds selected from the group consisting of NMDA receptor antagonists, antiinflammatory drugs, opioids, antipsychotics, anticonvulsants, corticosteroids, local anesthetics, antidepressants, selective serotonin reuptake inhibitors, selective norepinephrine reuptake inhibitor, and alpha-adrenergic agonist.

**27.** A method for treating or preventing acute and chronic pain **characterized by** comprising the step of administering to a human being or animal, in need of said treatment, an effective amount of a compound of claim 1, or a pharmaceutically acceptable salt thereof.

**28.** A method according to claim 27, **characterized by** the fact that acute and chronic pain are caused by conditions or diseases selected from the group consisting of genetic or congenital disorders; trauma, surgery or burns; infectious and/or parasitic disease; metabolic disease; inflammation; autoimmune diseases; poisoning; metabolic disturbances and diseases; neurodegenerative disorders and conditions; dysfunctional conditions; psychological disorders and benign and malignant neoplasms; osteoarticular and muscular lesions, root and spinal cord injury; mechanical damage by radiation, surgeries; thermal, chemical, or electrical burns; osteoarthritis, rheumatoid arthritis; musculoskeletal pain, particularly post-traumatic; toothache; headache, migraine; abdominal pain; cancer pain; post-operative pain; multiple sclerosis, amyotrophic lateral sclerosis; intervertebral disc hernia; diabetes mellitus, hypo- or hyperthyroidism; pain due to repetitive strain injuries (RSI); pain due to congenital or genetic disorders; pain due to

leprosy, herpes zoster, acquired immunodeficiency syndrome AIDS, pain due to heavy metals poisoning; deafferentation pain, central pain, phantom limb pain, causalgia, myelopathic pain, complex regional pain syndrome, myofascial pain syndrome, fibromyalgia, pain from the amputation stump, reflex sympathetic dystrophy, post-herpetic neuralgia, diabetic mononeuropathy, ischemic neuropathy, polyarteritis, pain after radiotherapy, polyneuropathy, multiple mononeuritis, infectious and neurodegenerative myelopathy toxic neuropathies, vasculitis and syringomyelia.

Figure 1

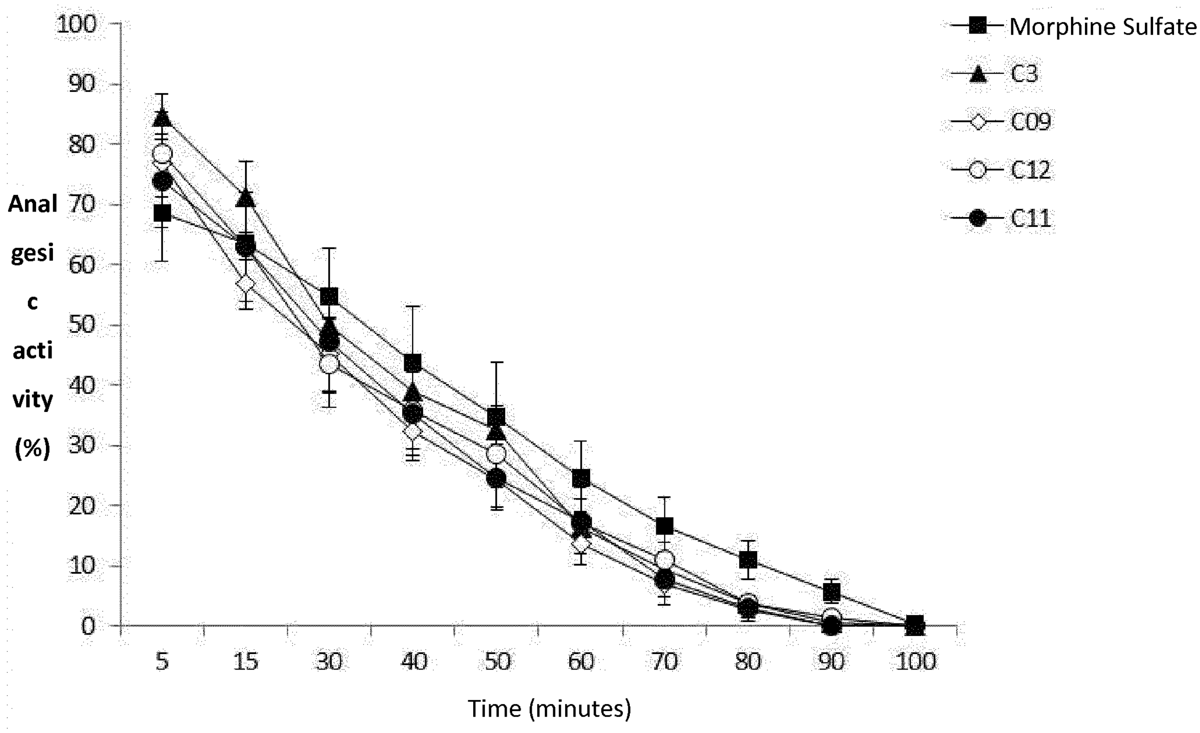
Analgesic activity (%)
100
90
80
70
60
50
40
30
20
10
0
5
15
30
40
50
60
70
80
90
100
Time (minutes)
Morphine Sulfate
C3
C09
C12
C11

Figure 2

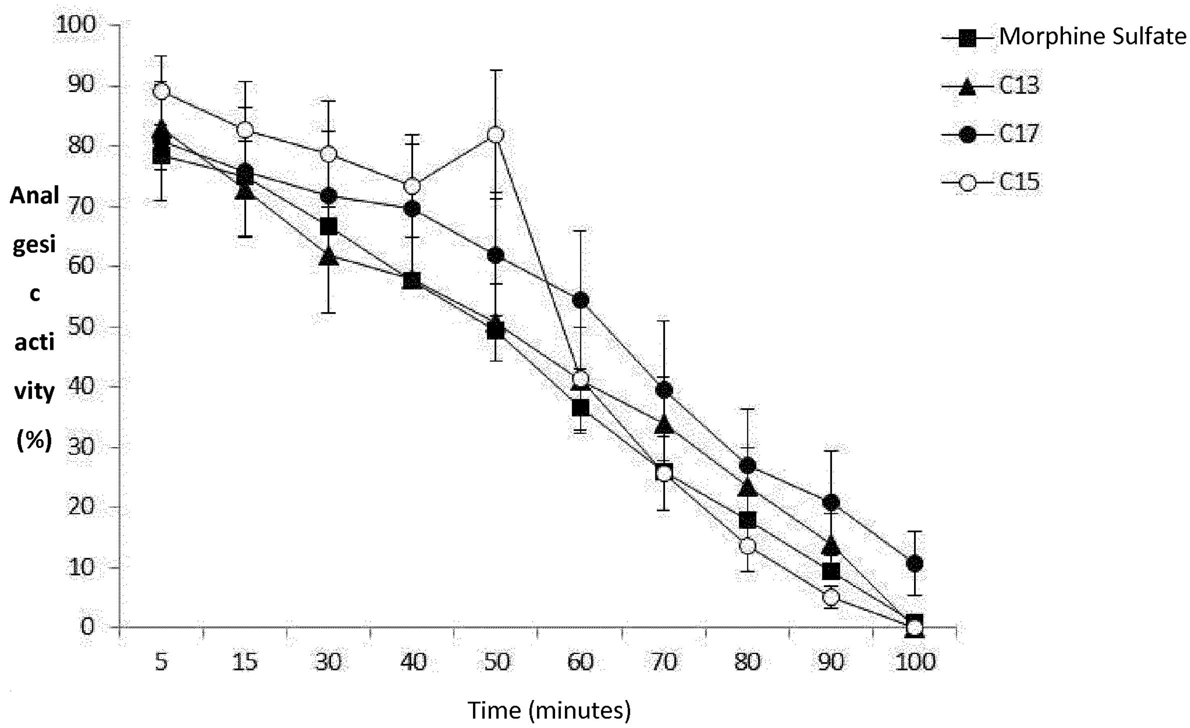
Morphine Sulfate
C13
C17
C15
Analgesic activity (%)
100
90
80
70
60
50
40
30
20
10
0
5
15
30
40
50
60
70
80
90
100
Time (minutes)

Figure 3

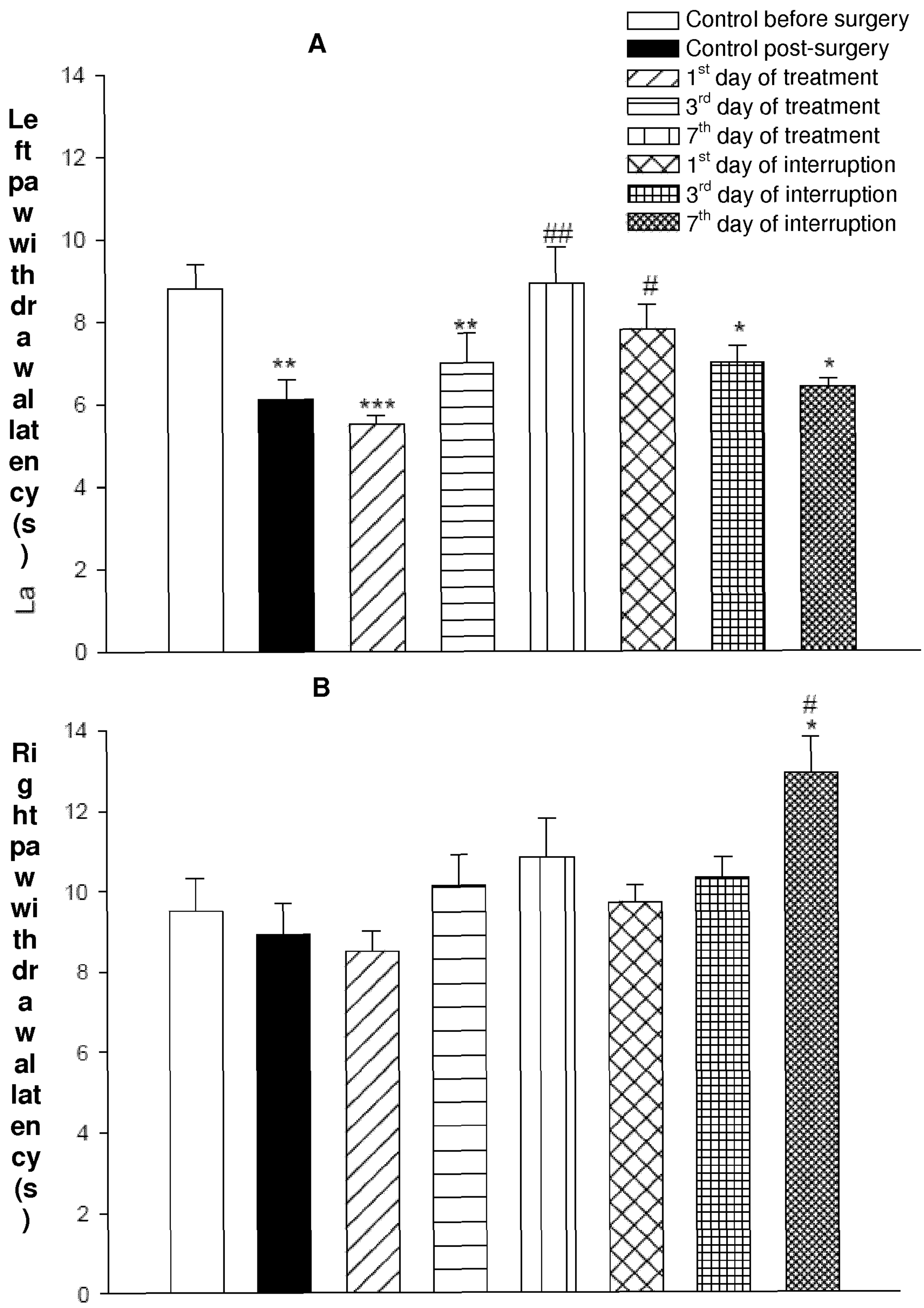
A
Control before surgery
Control post-surgery
1st day of treatment
3rd day of treatment
7th day of treatment
1st day of interruption
3rd day of interruption
7th day of interruption
Left paw withdrawal latency (s)
14
12
10
8
6
4
2
0
**
***
**
##
#
*
*
B
Right paw withdrawal latency (s)
14
12
10
8
6
4
2
0
#
*

Figure 4

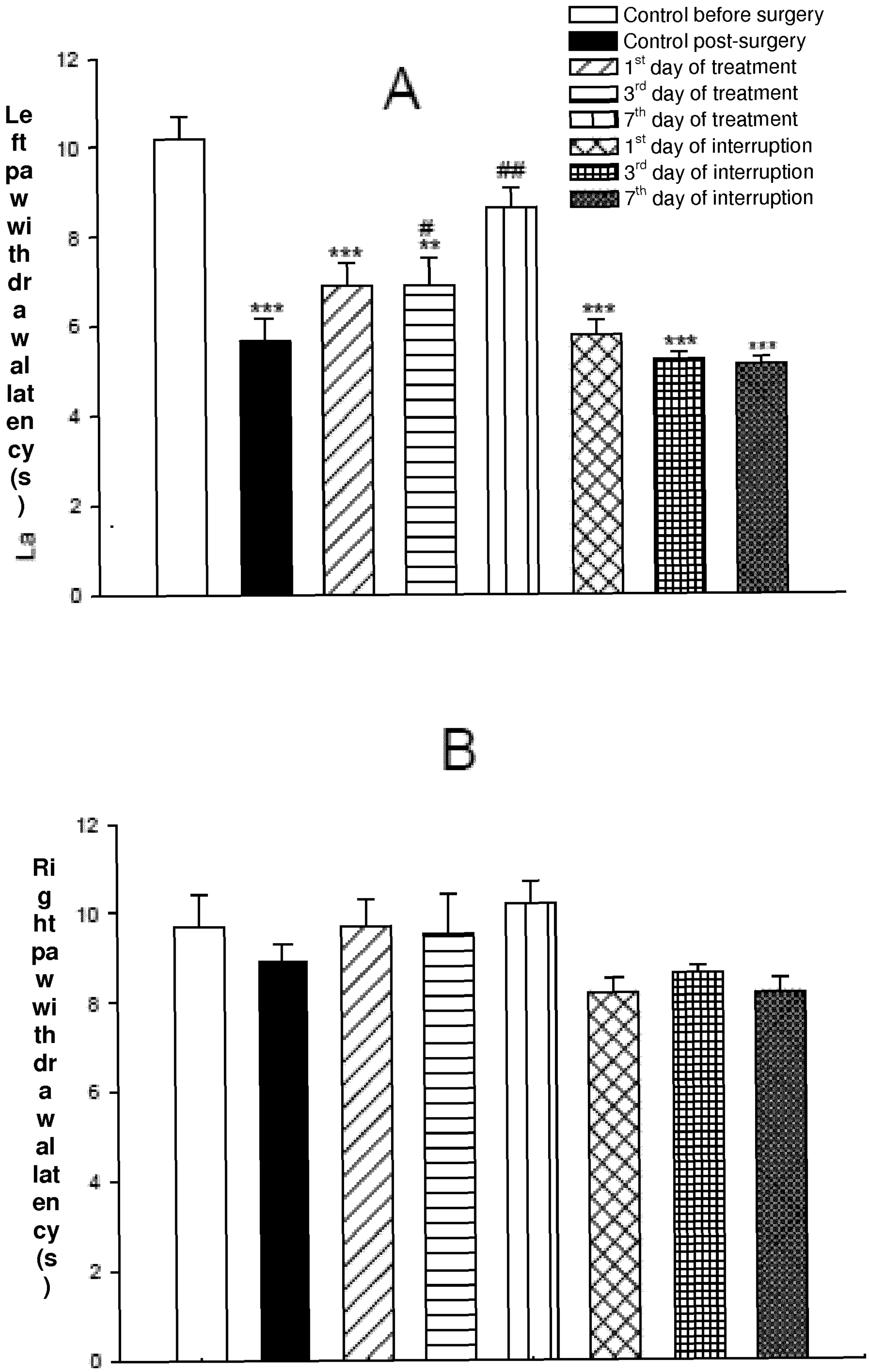
A
Control before surgery
Control post-surgery
1st day of treatment
3rd day of treatment
7th day of treatment
1st day of interruption
3rd day of interruption
7th day of interruption
Left paw withdrawal latency (s)
0
2
4
6
8
10
12
***
***
#
**
##
***
***
***
B
Right paw withdrawal latency (s)

Figure 5

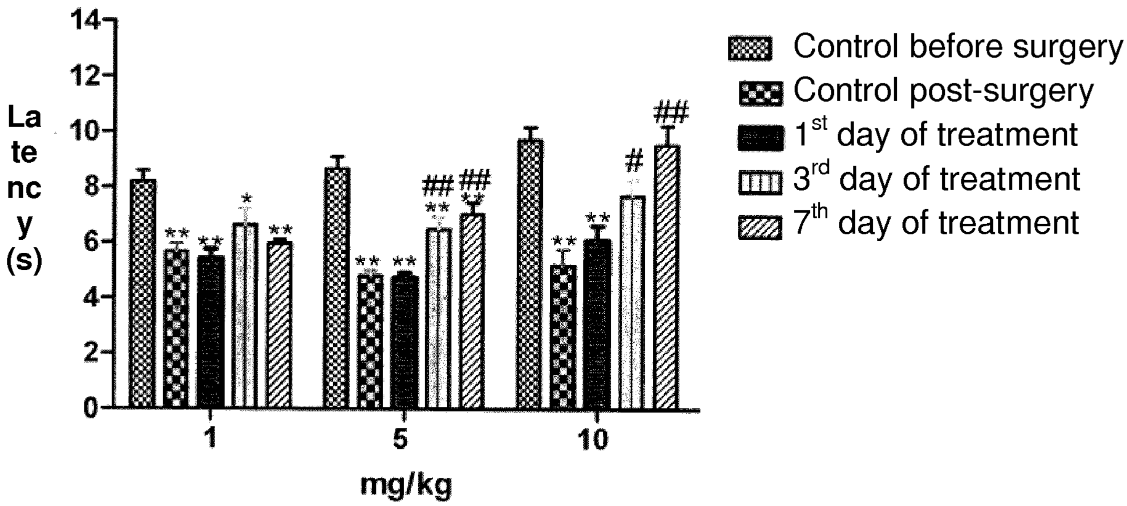
14
12
10
8
6
4
2
0
La
te
nc
y
(s)
**
*
##
#
1
5
10
mg/kg
Control before surgery
Control post-surgery
1st day of treatment
3rd day of treatment
7th day of treatment

Figure 6

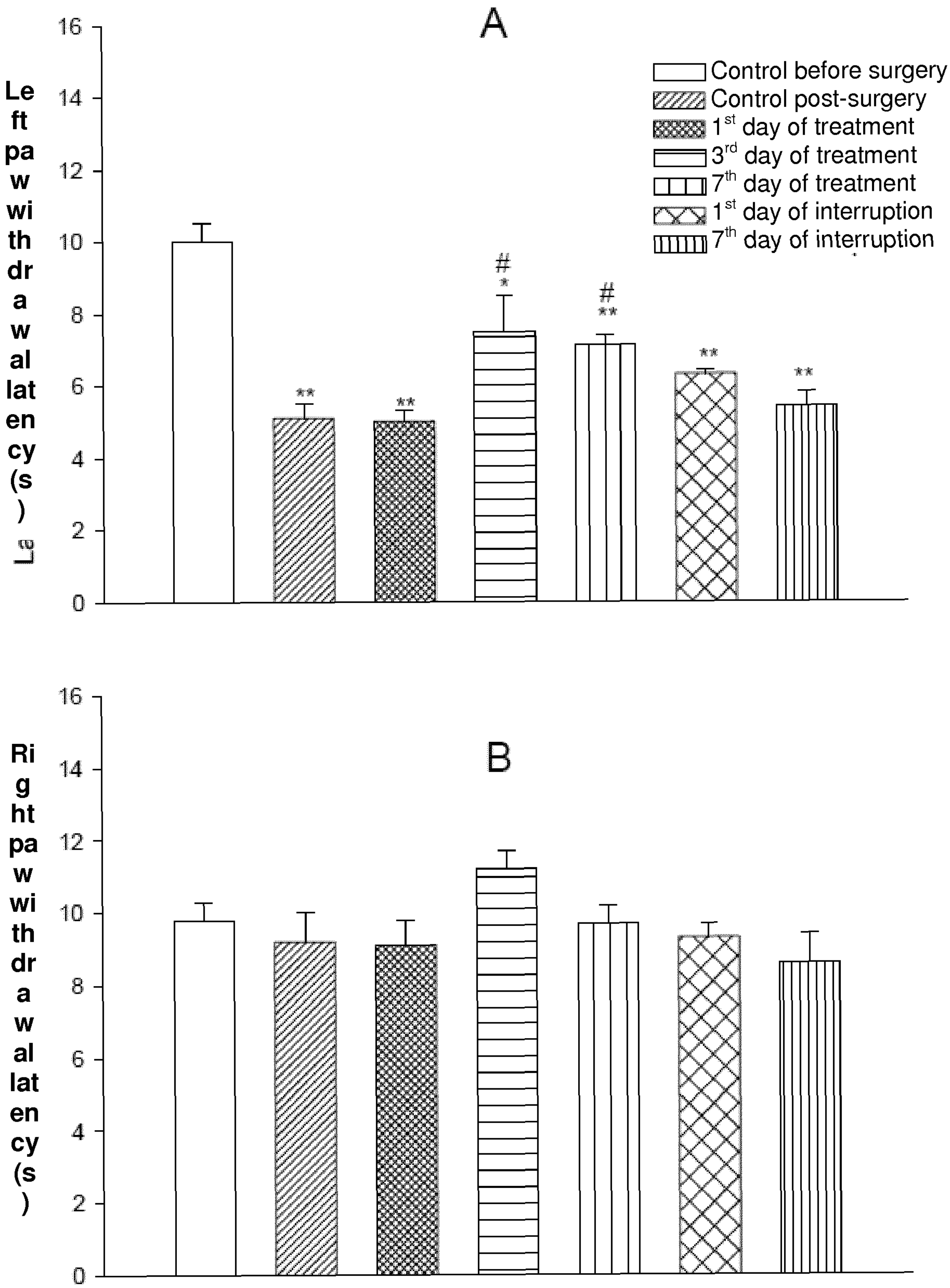
A
Left paw withdrawal latency (s)
0
2
4
6
8
10
12
14
16
Control before surgery
Control post-surgery
1st day of treatment
3rd day of treatment
7th day of treatment
1st day of interruption
7th day of interruption
**
**
#
*
#
**
**
**
B
Right paw withdrawal latency (s)
0
2
4
6
8
10
12
14
16

Figure 7

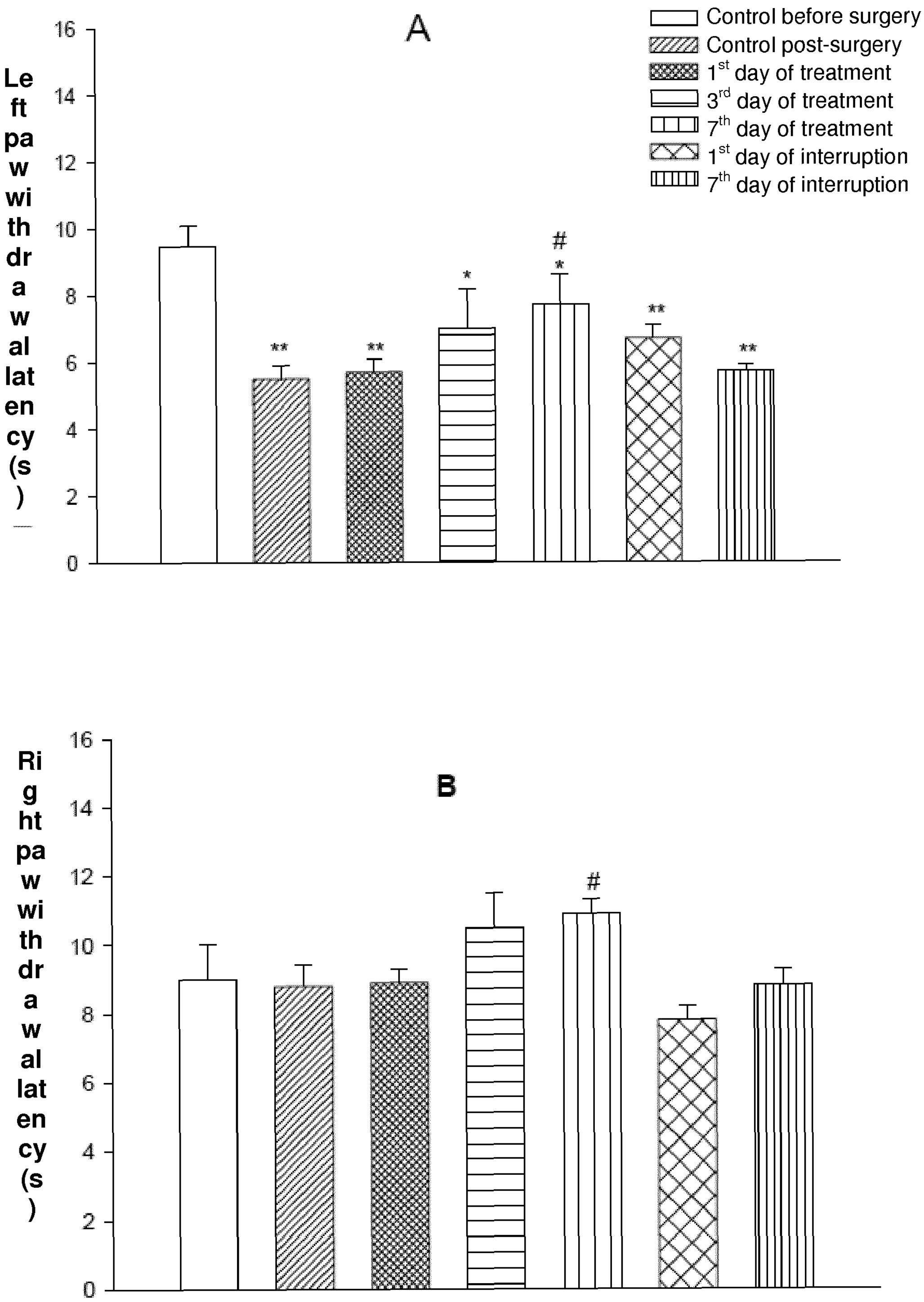
A
Control before surgery
Control post-surgery
1st day of treatment
3rd day of treatment
7th day of treatment
1st day of interruption
7th day of interruption
Left paw withdrawal latency (s)
16
14
12
10
8
6
4
2
0
**
**
*
#
*
**
**
B
Right paw withdrawal latency (s)
16
14
12
10
8
6
4
2
0
#

Figure 8

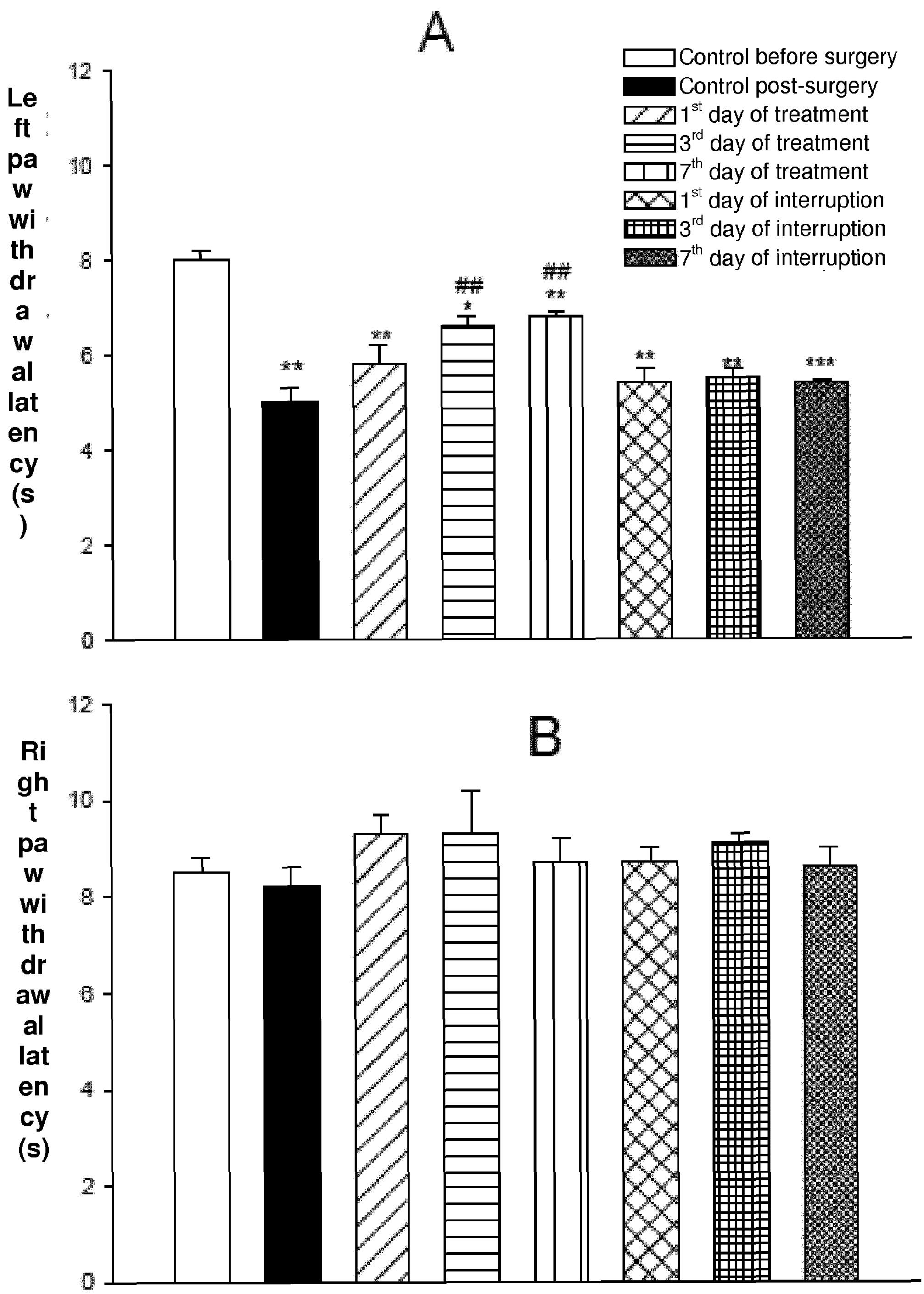
A
Control before surgery
Control post-surgery
1st day of treatment
3rd day of treatment
7th day of treatment
1st day of interruption
3rd day of interruption
7th day of interruption
Left paw withdrawal latency (s)
0
2
4
6
8
10
12
**
**
##
*
##
**
**
**
***
B
Right paw withdrawal latency (s)
0
2
4
6
8
10
12

Figure 9

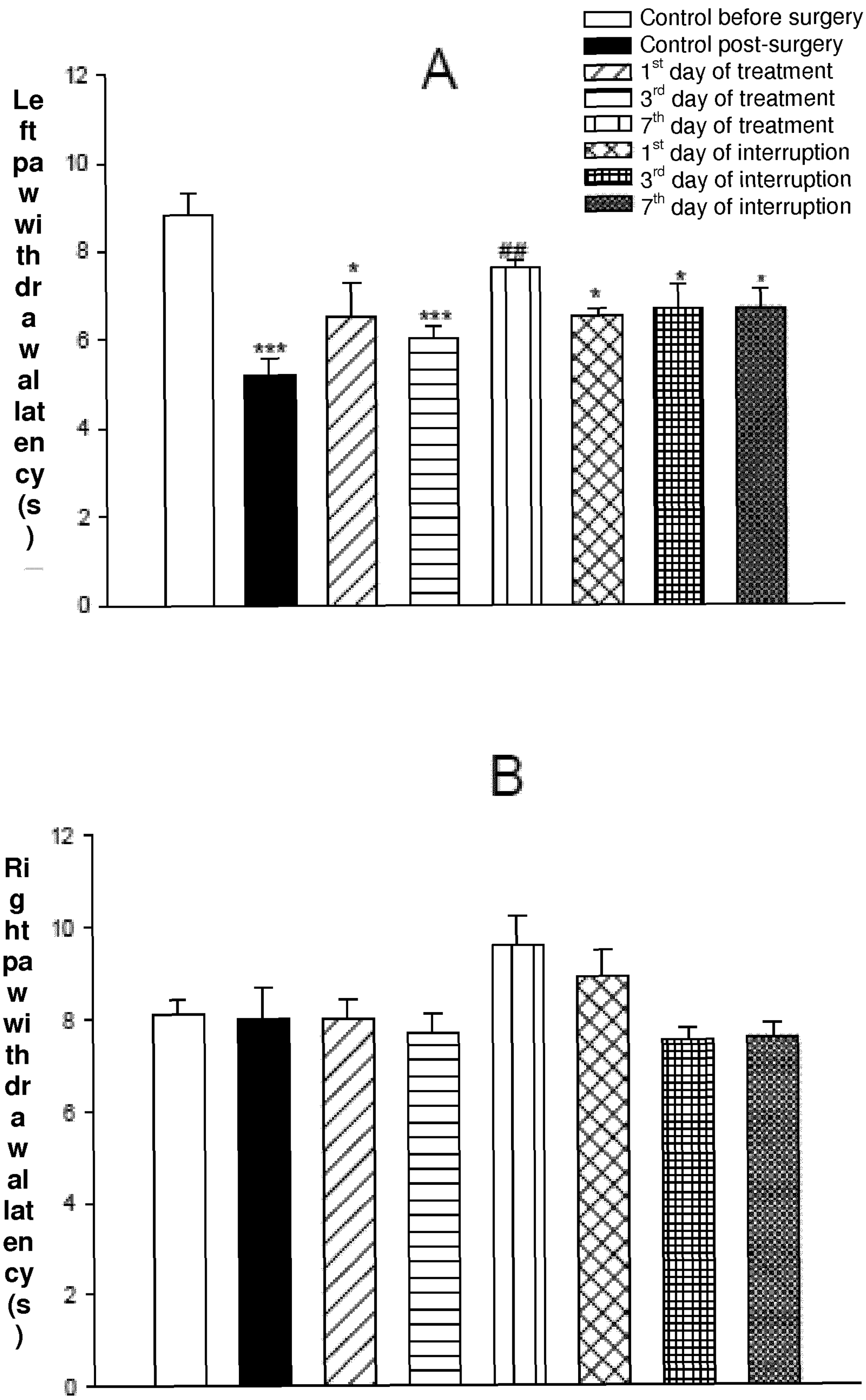
A
Control before surgery
Control post-surgery
1st day of treatment
3rd day of treatment
7th day of treatment
1st day of interruption
3rd day of interruption
7th day of interruption
Left paw withdrawal latency (s)
12
10
8
6
4
2
0
***
*
***
##
*
*
*
B
Right paw withdrawal latency (s)
12
10
8
6
4
2
0

Figure 10

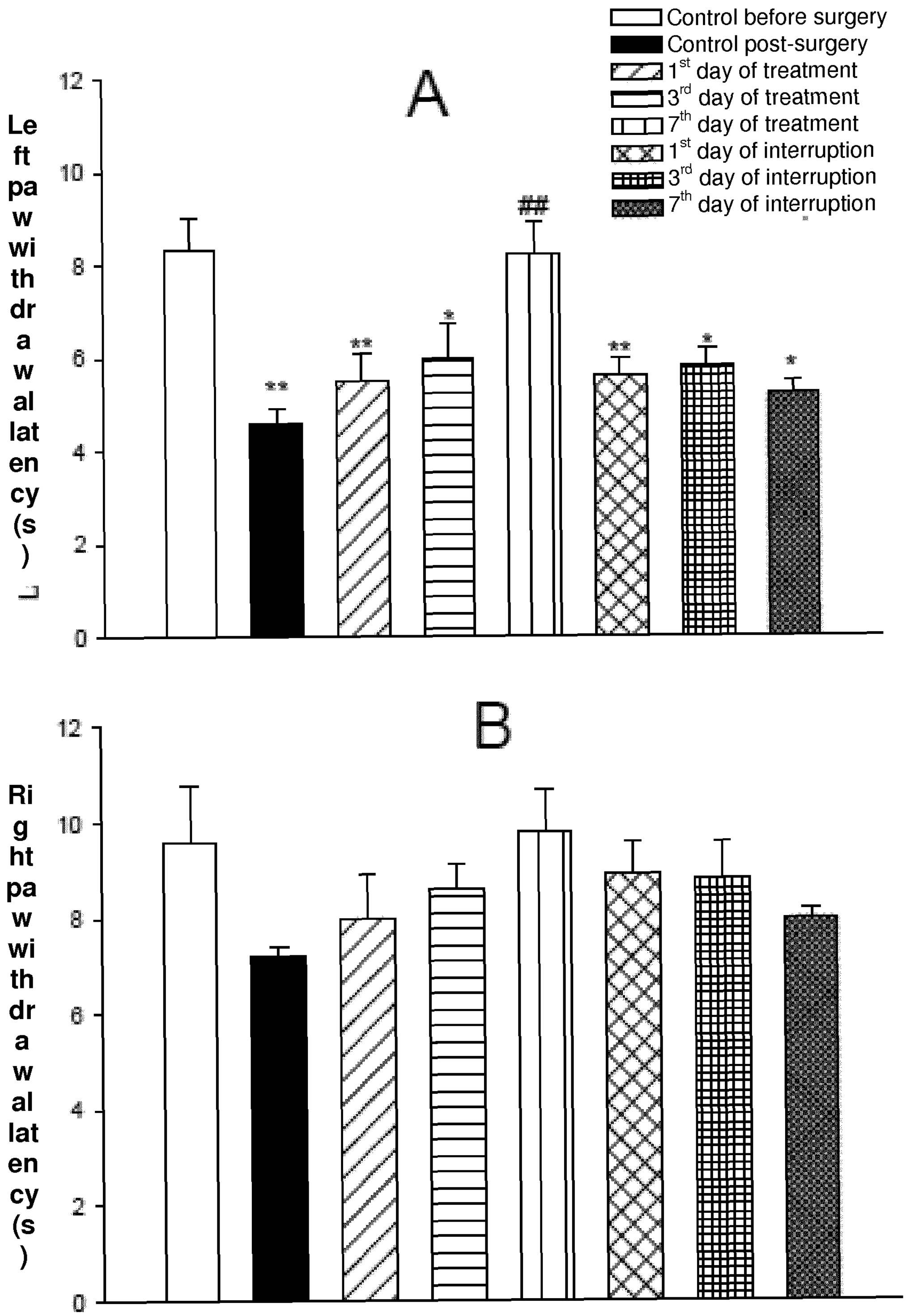
Control before surgery
Control post-surgery
1st day of treatment
3rd day of treatment
7th day of treatment
1st day of interruption
3rd day of interruption
7th day of interruption
A
Left paw withdrawal latency (s)
0
2
4
6
8
10
12
**
**
*
##
**
*
*
B
Right paw withdrawal latency (s)
0
2
4
6
8
10
12

Figure 11

A

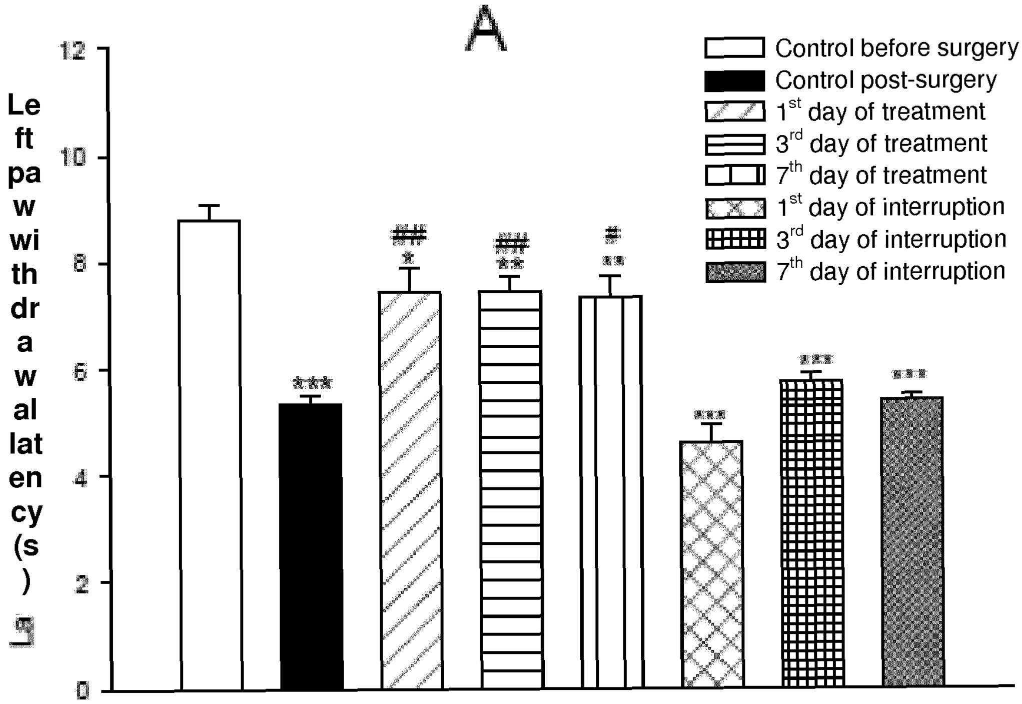
Control before surgery
Control post-surgery
1st day of treatment
3rd day of treatment
7th day of treatment
1st day of interruption
3rd day of interruption
7th day of interruption
Left paw withdrawal latency (s)
12
10
8
6
4
2
0
***
##
*
##
**
#
**
***
***
***

B

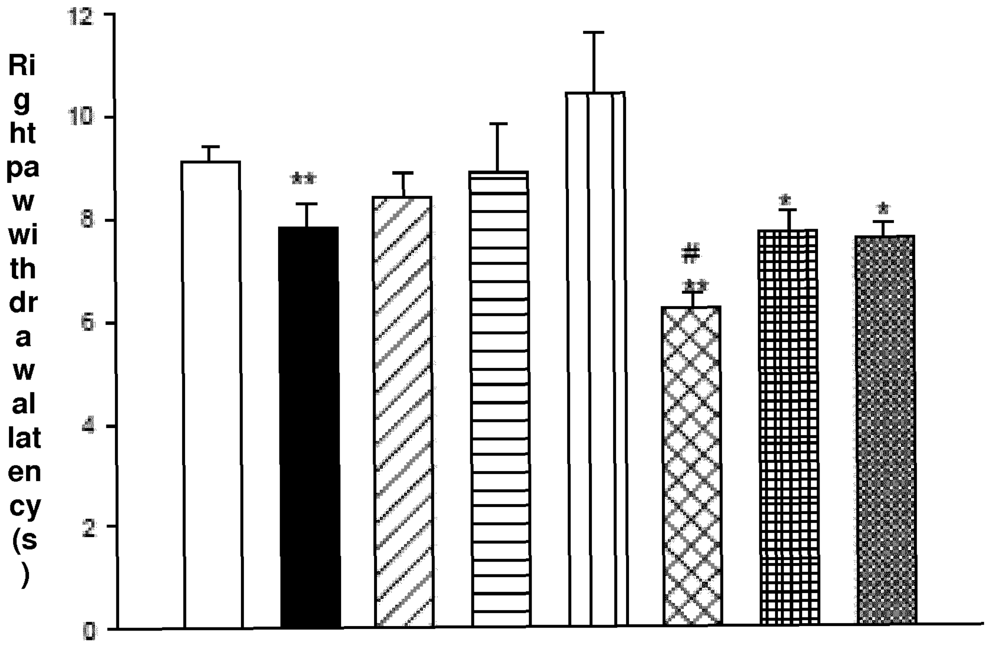
Right paw withdrawal latency (s)
12
10
8
6
4
2
0
**
#
**
*
*

Figure 12

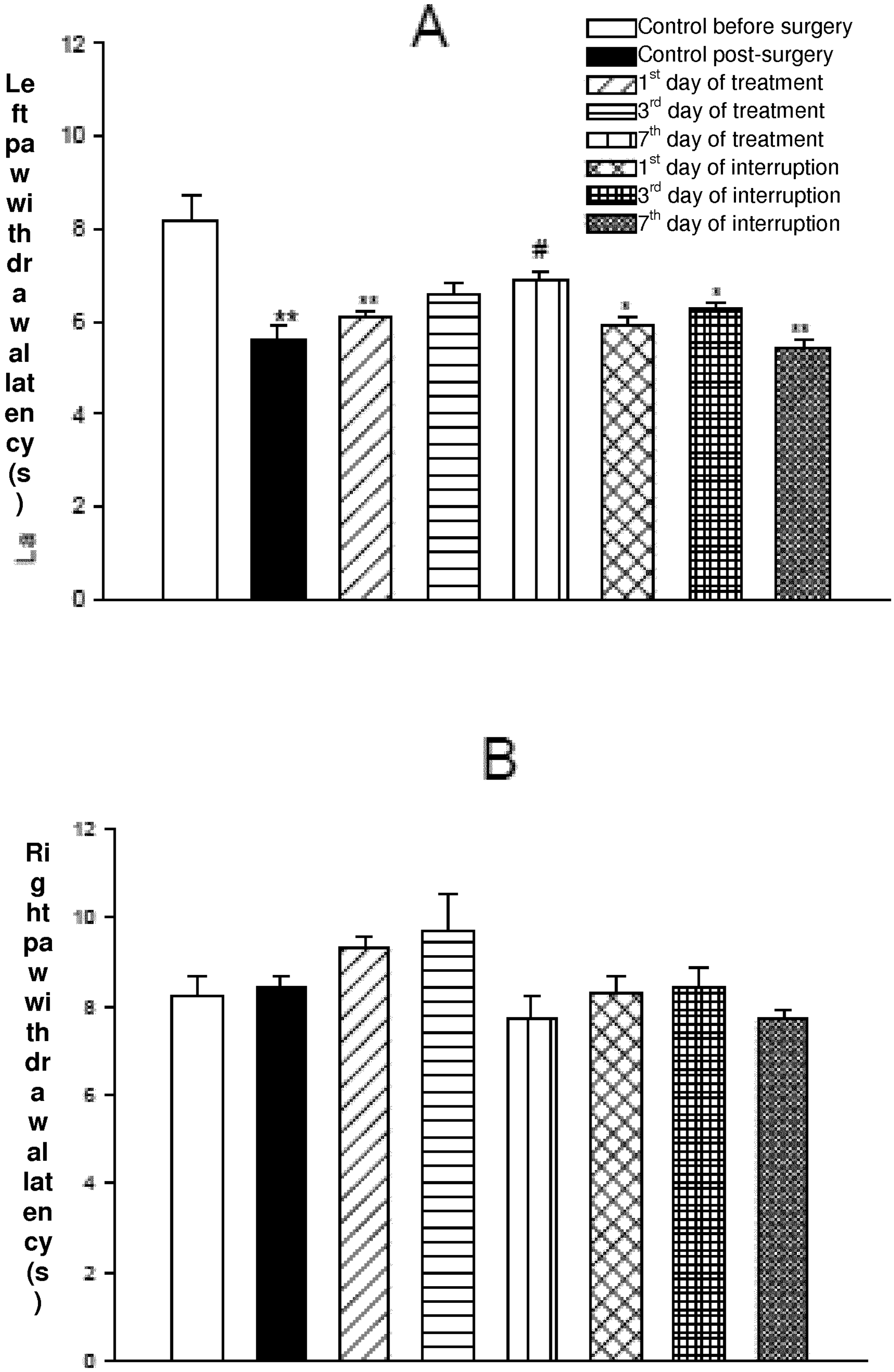
A
Control before surgery
Control post-surgery
1st day of treatment
3rd day of treatment
7th day of treatment
1st day of interruption
3rd day of interruption
7th day of interruption
Left paw withdrawal latency (s)
0
2
4
6
8
10
12
**
**
#
*
*
**
B
Right paw withdrawal latency (s)
0
2
4
6
8
10
12

Figure 13

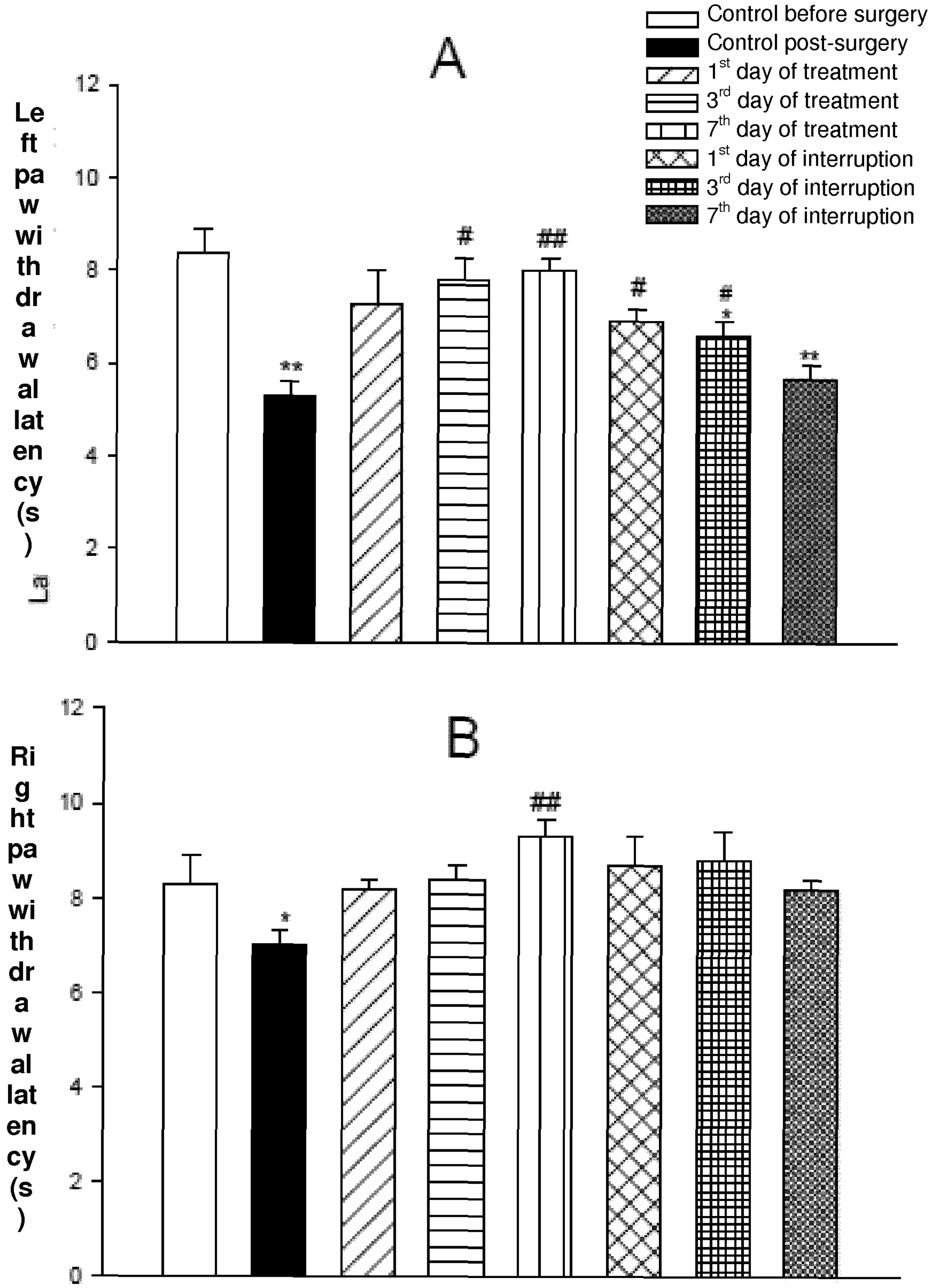
A
B
Control before surgery
Control post-surgery
1st day of treatment
3rd day of treatment
7th day of treatment
1st day of interruption
3rd day of interruption
7th day of interruption
Left paw withdrawal latency (s)
Right paw withdrawal latency (s)
0
2
4
6
8
10
12
**
#
##
#
#
*
**
*
##

Figure 14

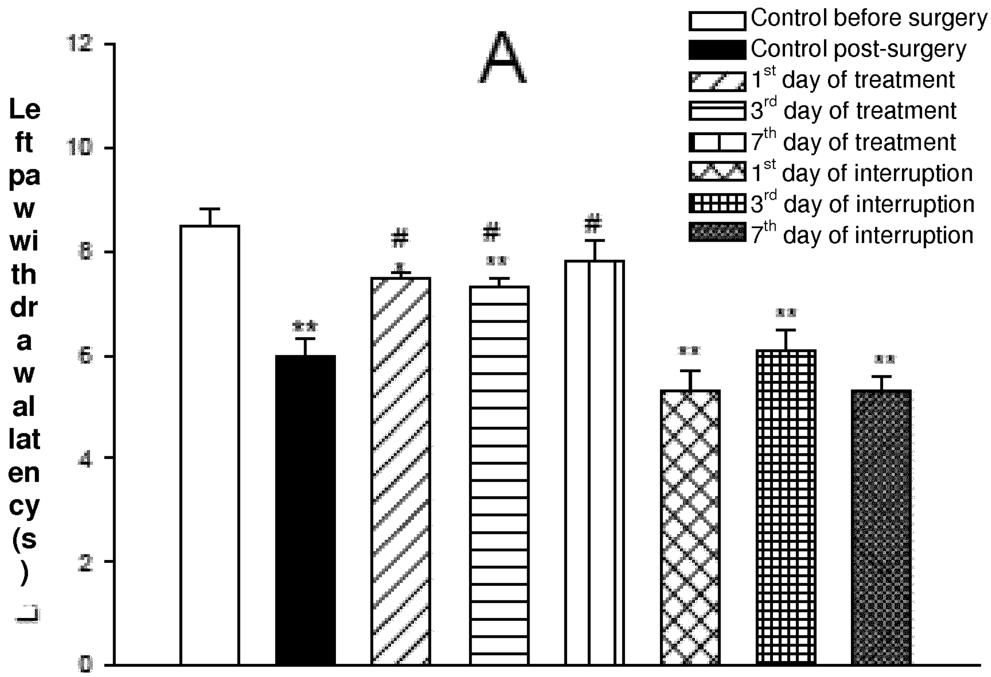
A
Left paw withdrawal latency (s)
12
10
8
6
4
2
0
Control before surgery
Control post-surgery
1st day of treatment
3rd day of treatment
7th day of treatment
1st day of interruption
3rd day of interruption
7th day of interruption
**
#
*
#
**
#
**
**
**

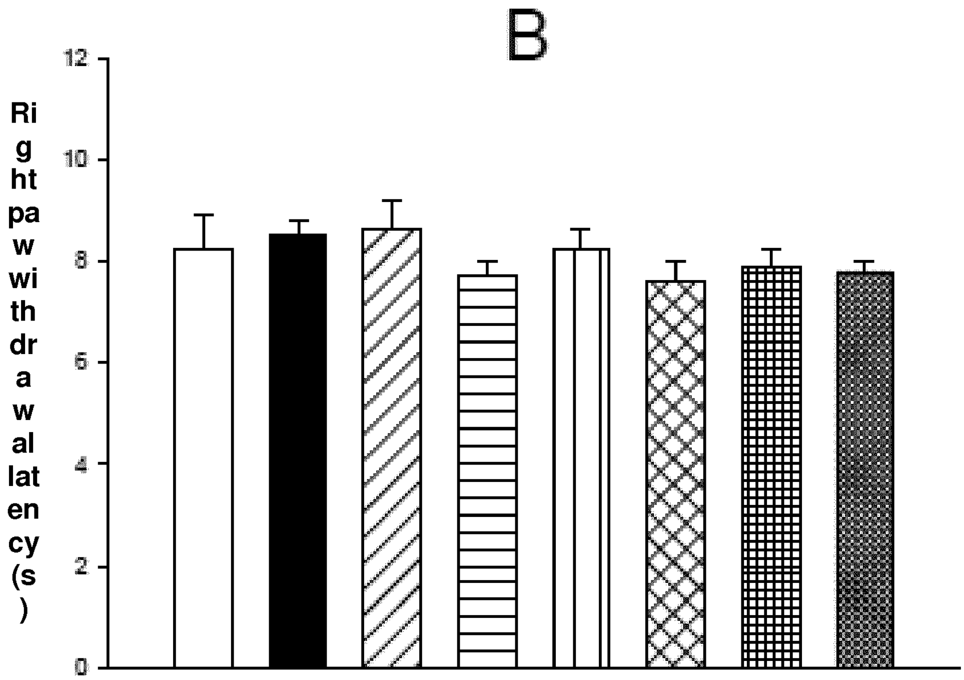
B
Right paw withdrawal latency (s)
12
10
8
6
4
2
0

Figure 15

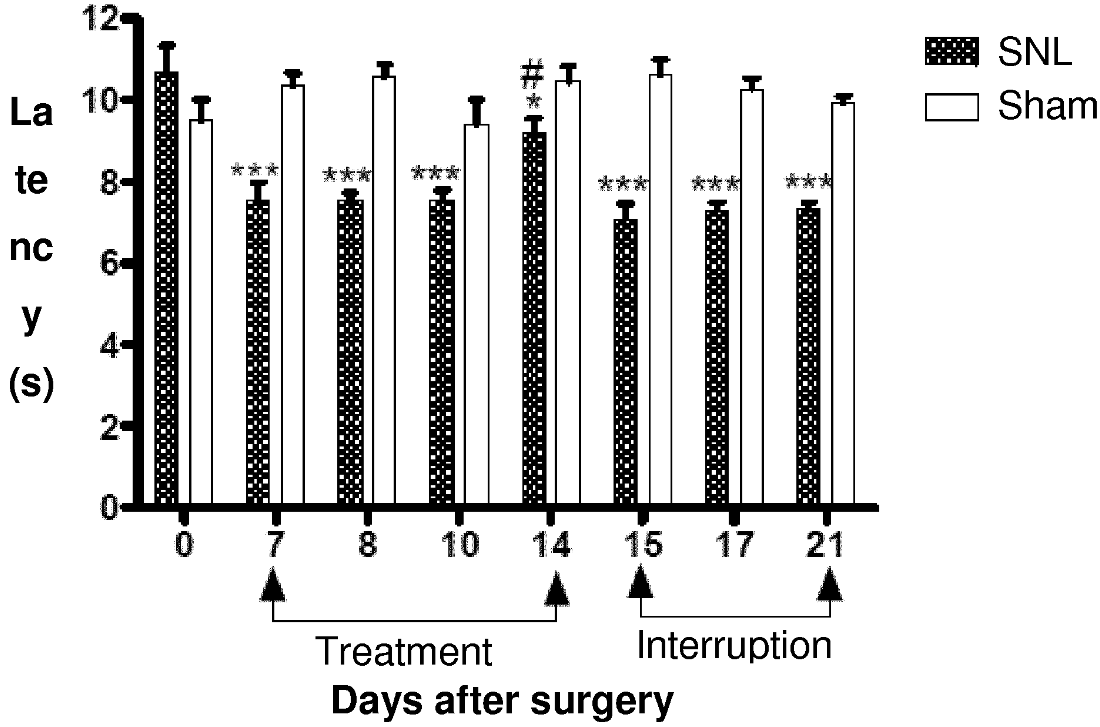
Latency (s)
12
10
8
6
4
2
0
SNL
Sham
***
#
*
0
7
8
10
14
15
17
21
Treatment
Interruption
Days after surgery

Figure 16

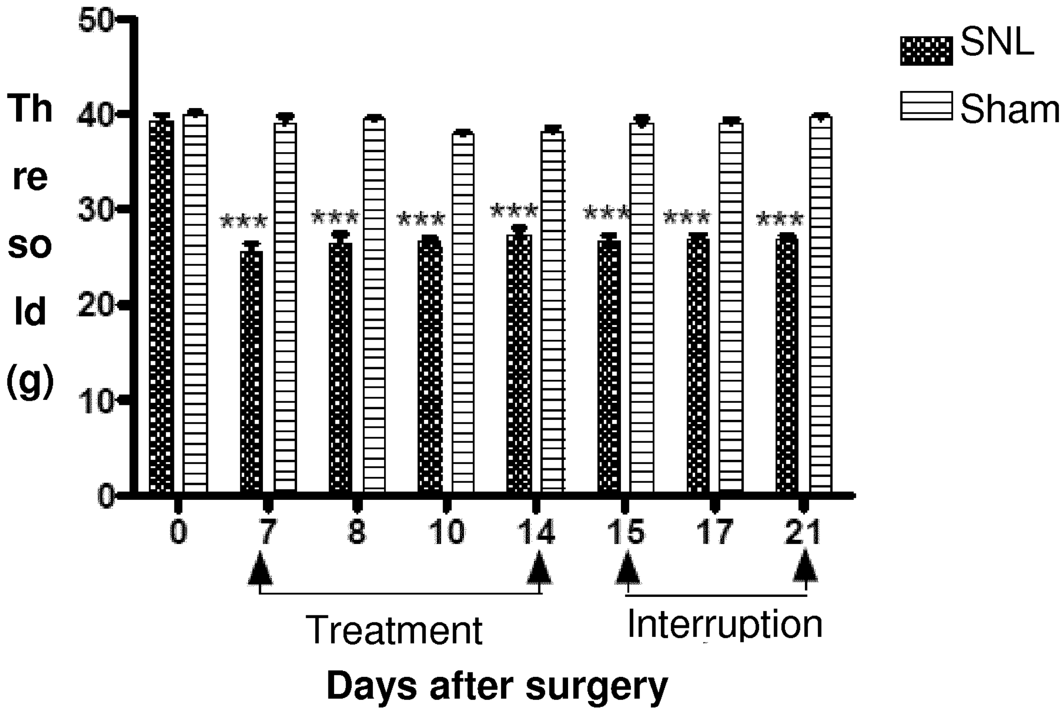
SNL
Sham
Threshold (g)
50
40
30
20
10
0
***
0
7
8
10
14
15
17
21
Treatment
Interruption
Days after surgery

Figure 17

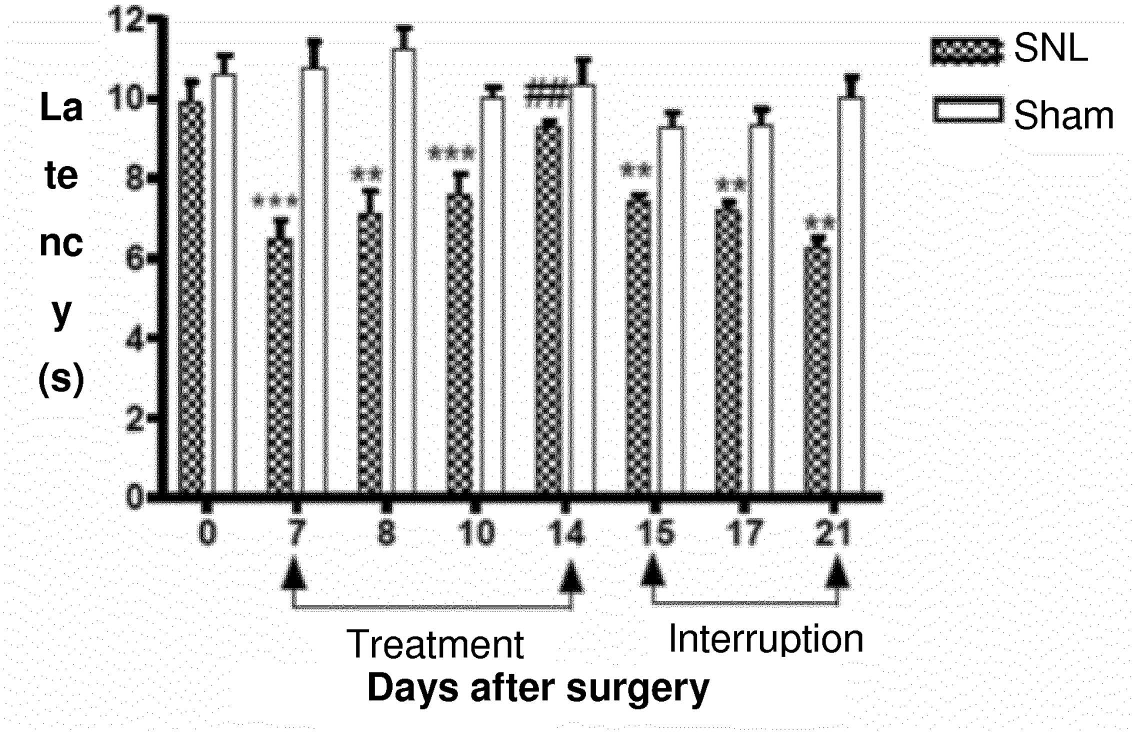
Latency (s)
12
10
8
6
4
2
0
***
**
***
###
**
**
**
0
7
8
10
14
15
17
21
Treatment
Interruption
Days after surgery
SNL
Sham

Figure 18

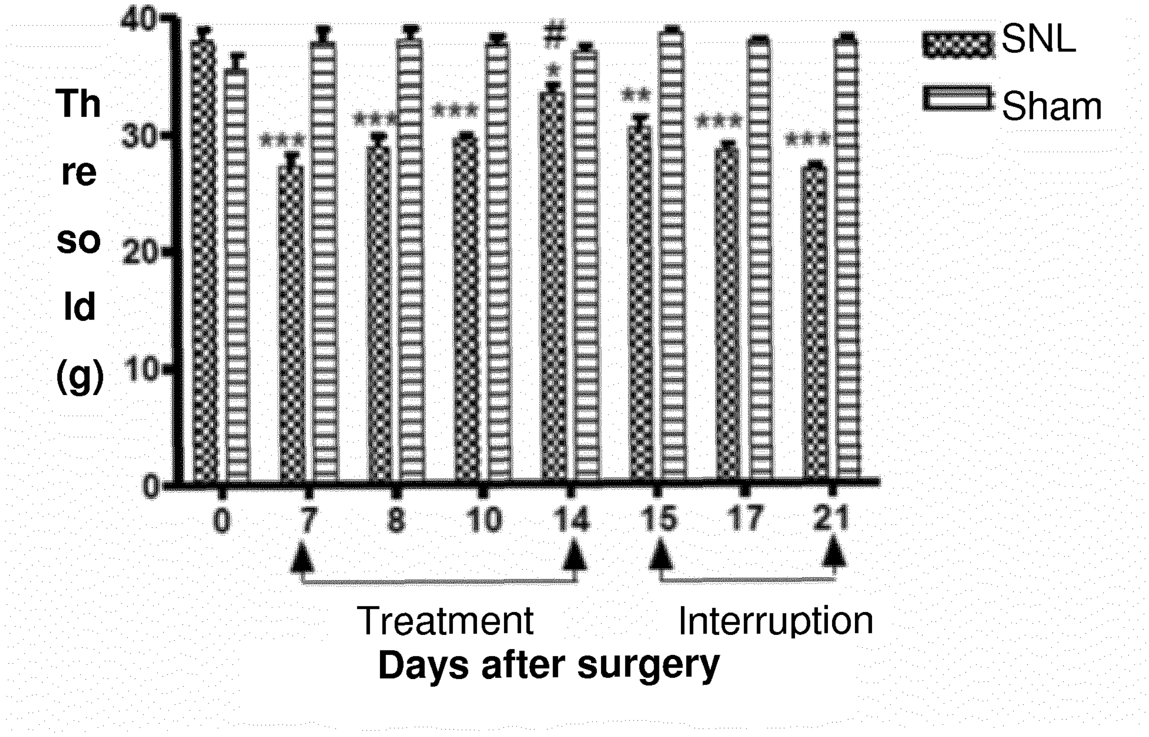
Threshold (g)
40
30
20
10
0
SNL
Sham
***
#
*
**
0
7
8
10
14
15
17
21
Treatment
Interruption
Days after surgery

Figure 19

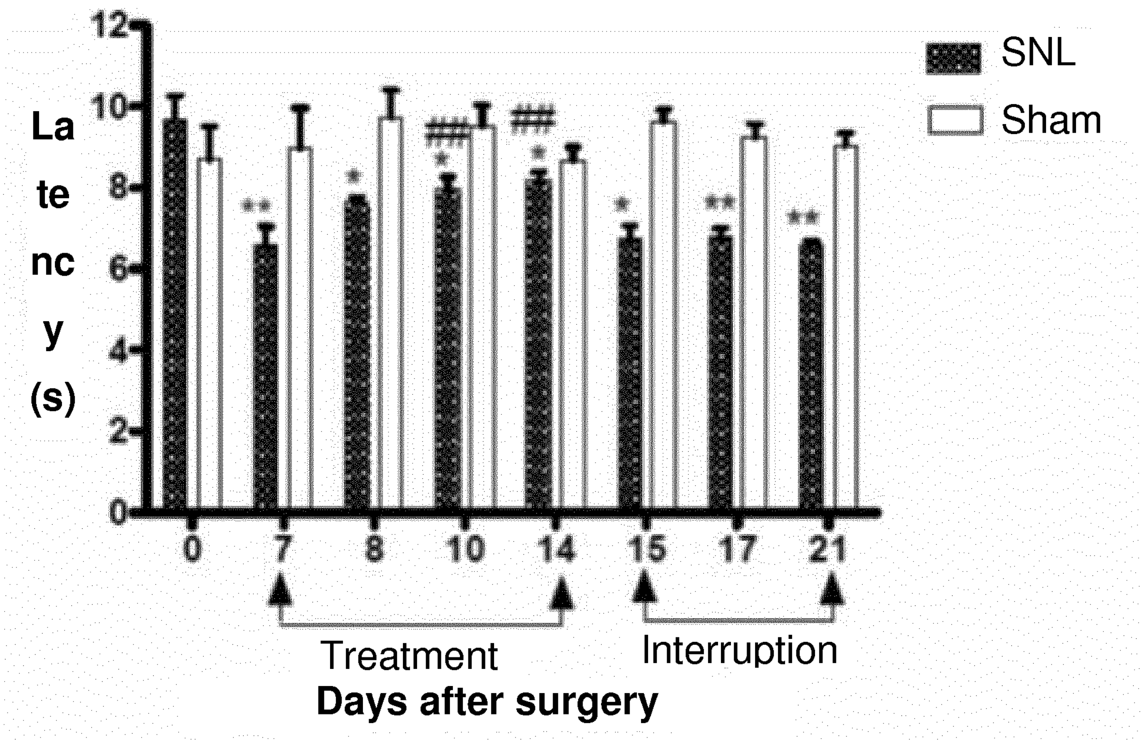
Latency (s)
12
10
8
6
4
2
0
SNL
Sham
**
*
###
*
##
*
*
**
**
0
7
8
10
14
15
17
21
Treatment
Interruption
Days after surgery

Figure 20

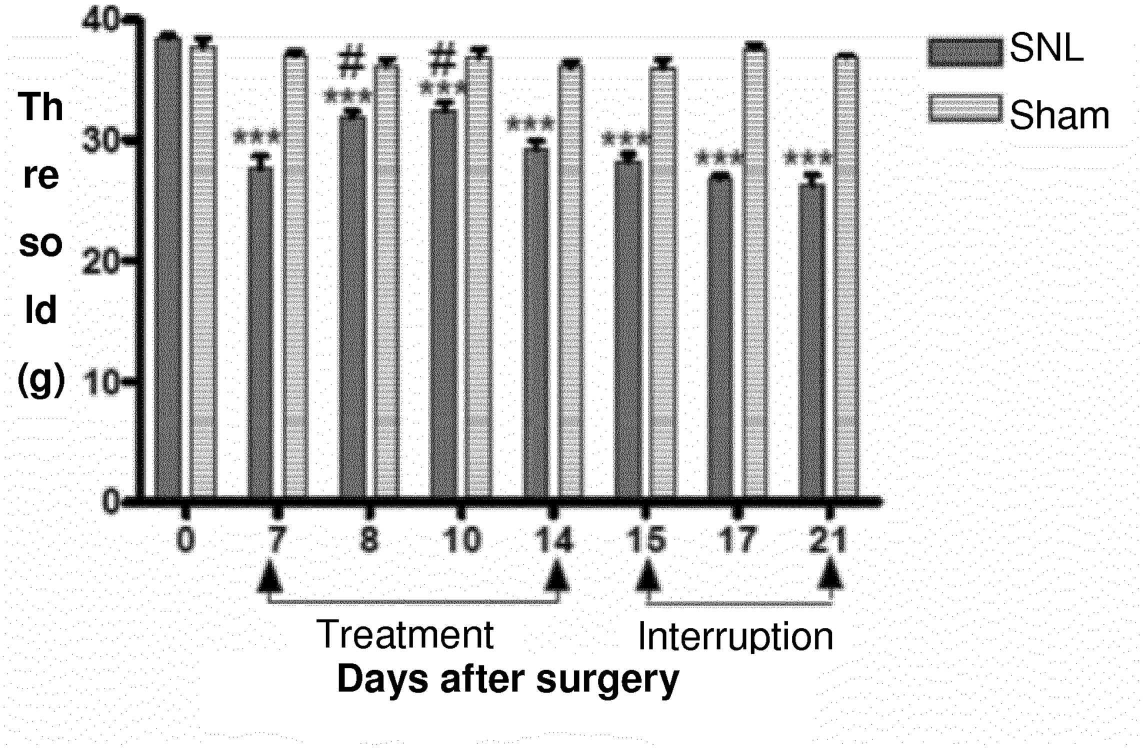
40
30
20
10
0
Th
re
so
ld
(g)
SNL
Sham
#
#
***
0
7
8
10
14
15
17
21
Treatment
Interruption
Days after surgery

Figure 21

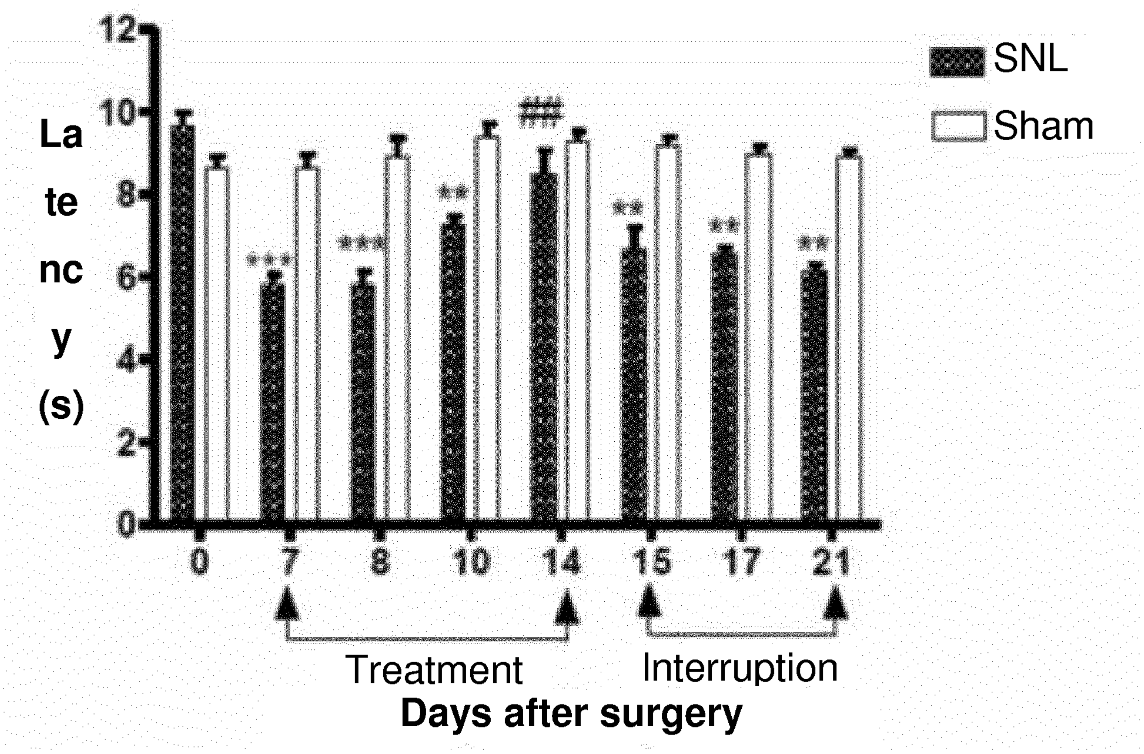
Latency (s)
12
10
8
6
4
2
0
SNL
Sham
***
***
**
###
**
**
**
0
7
8
10
14
15
17
21
Treatment
Interruption
Days after surgery

Figure 22

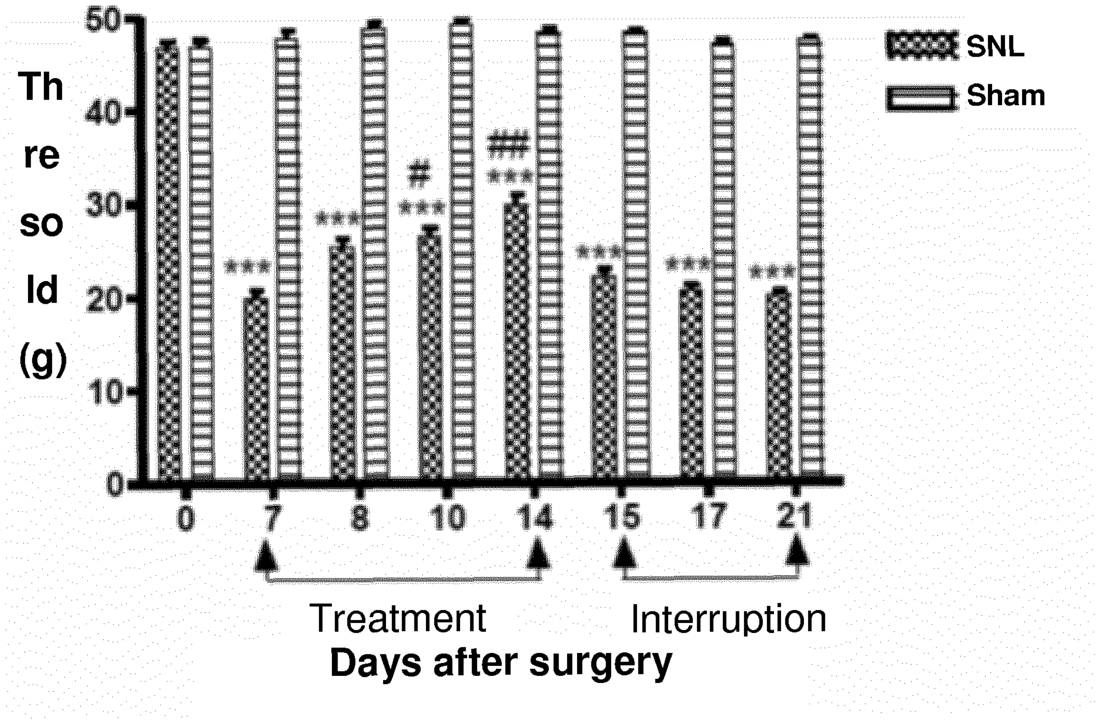
Th
re
so
ld
(g)
50
40
30
20
10
0
SNL
Sham
***
***
#
***
##
***
***
***
***
0
7
8
10
14
15
17
21
Treatment
Interruption
Days after surgery

Figure 23

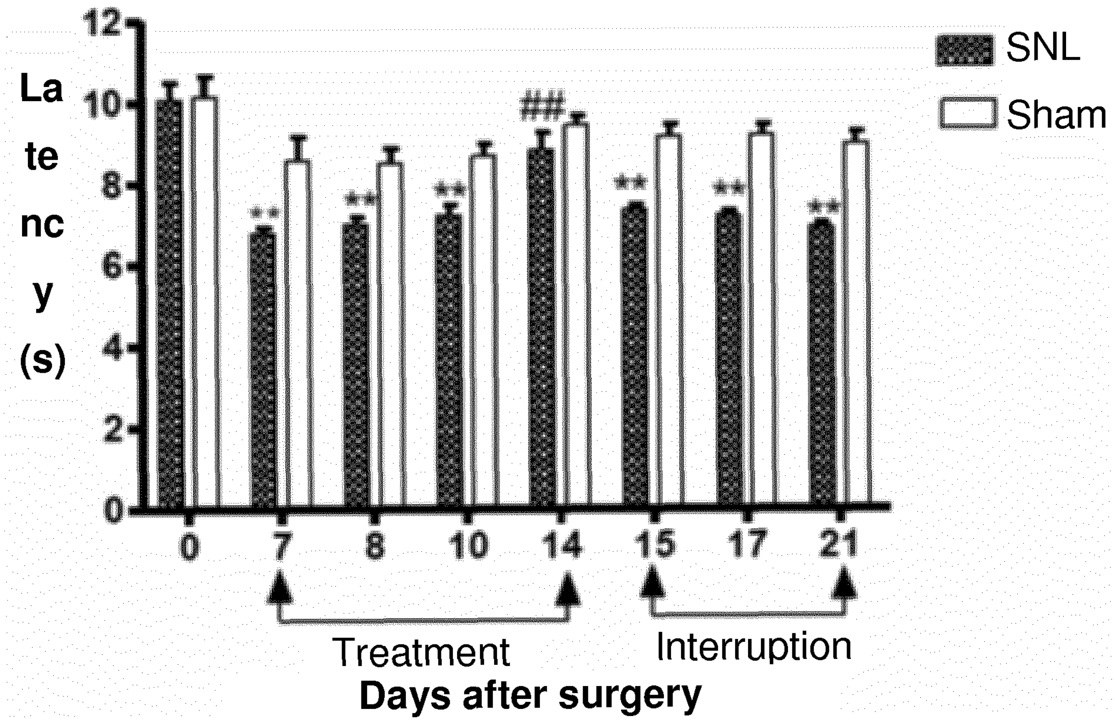
SNL
Sham
Latency (s)
12
10
8
6
4
2
0
##
**
0
7
8
10
14
15
17
21
Treatment
Interruption
Days after surgery

Figure 24

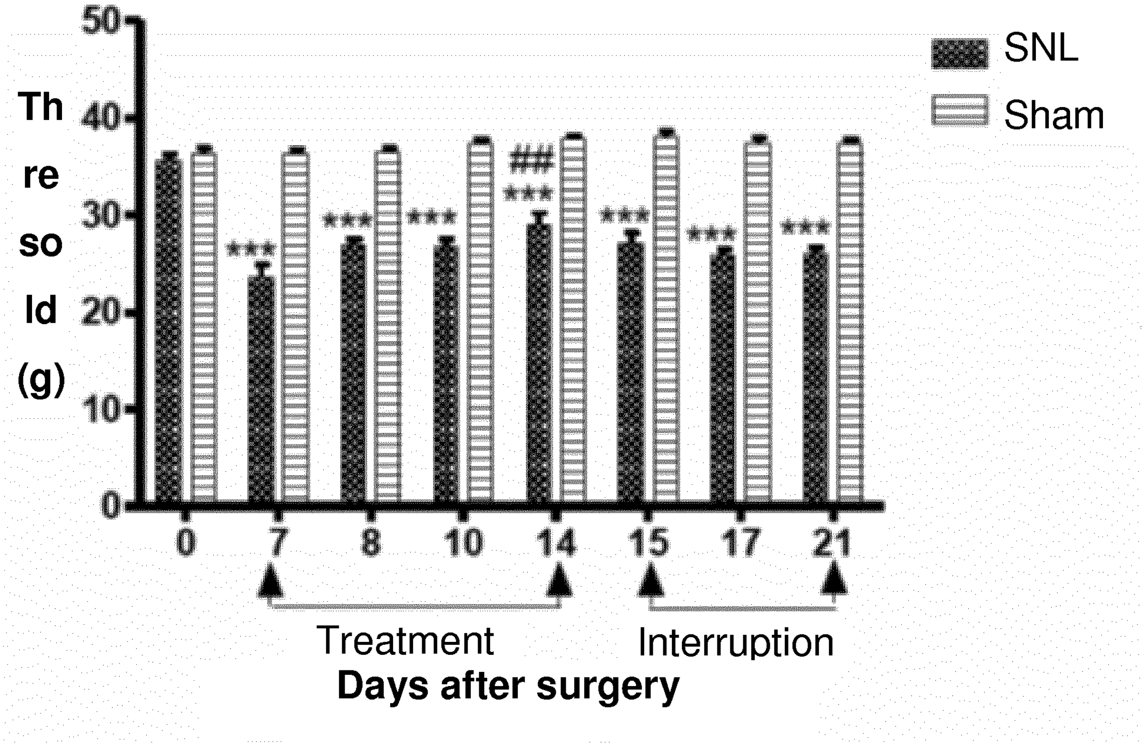
SNL
Sham
Threshold (g)
50
40
30
20
10
0
***
***
***
##
***
***
***
***
0
7
8
10
14
15
17
21
Treatment
Interruption
Days after surgery

Figure 25

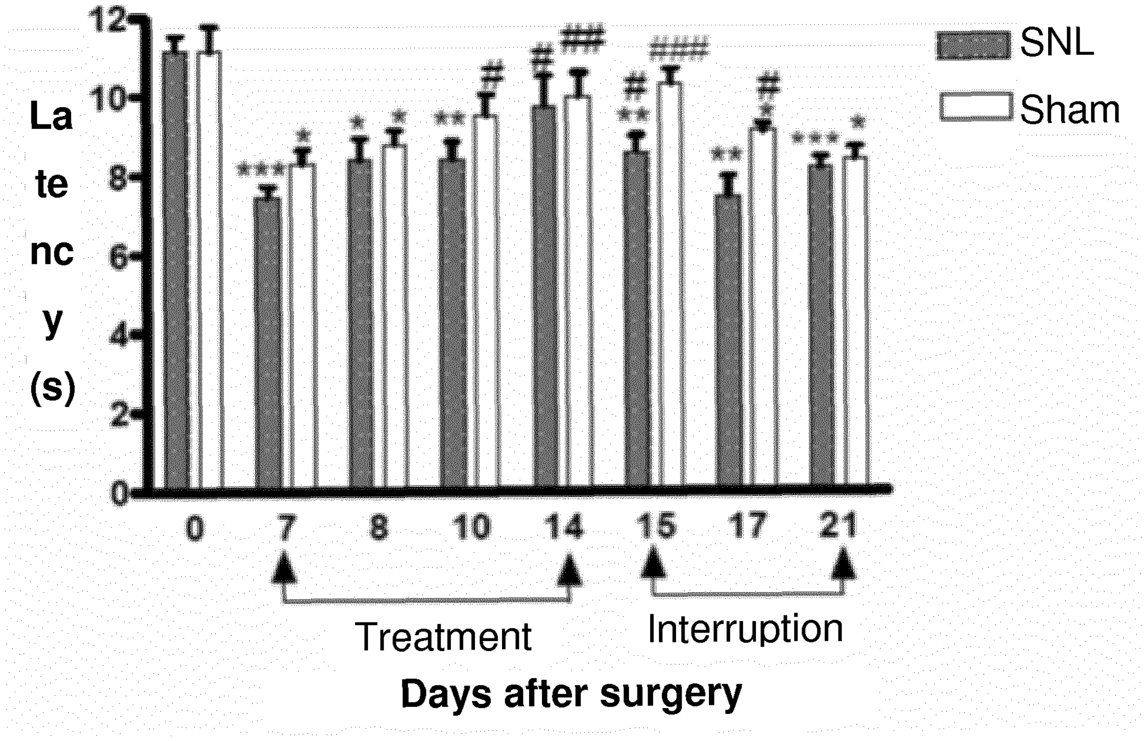
Latency (s)
12
10
8
6
4
2
0
SNL
Sham
0
7
8
10
14
15
17
21
Treatment
Interruption
Days after surgery

Figure 26

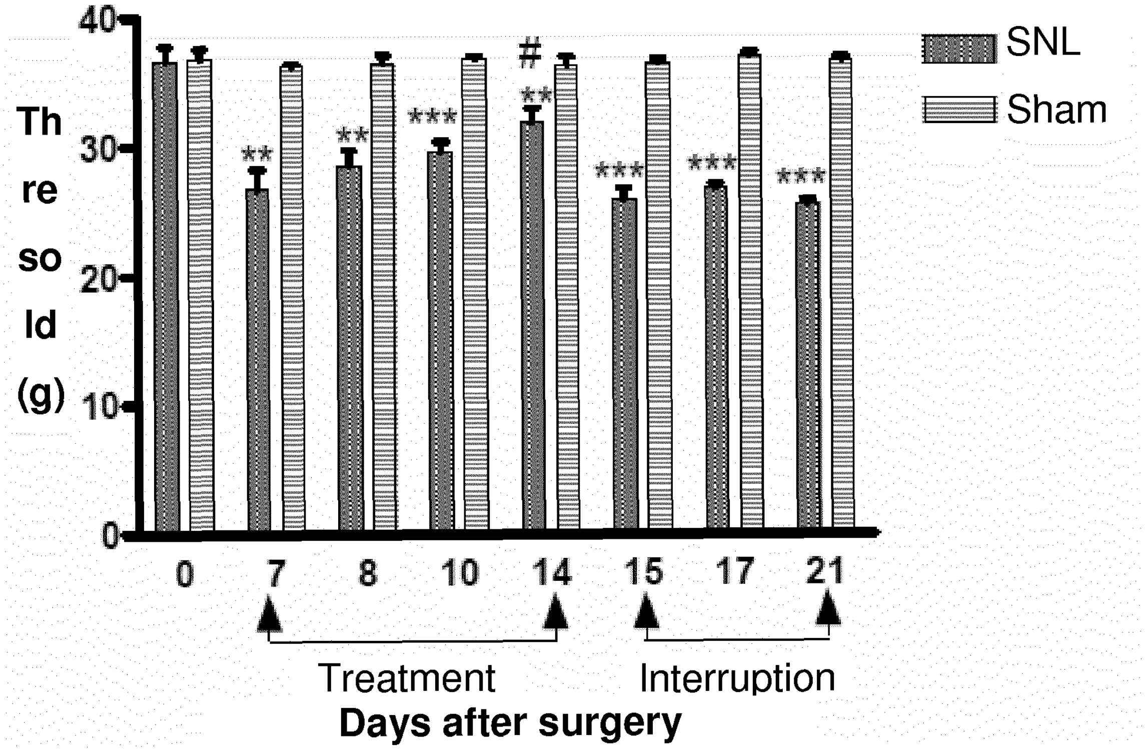
40
30
20
10
0
Threshold (g)
SNL
Sham
#
**
***
0
7
8
10
14
15
17
21
Treatment
Interruption
Days after surgery

Figure 27

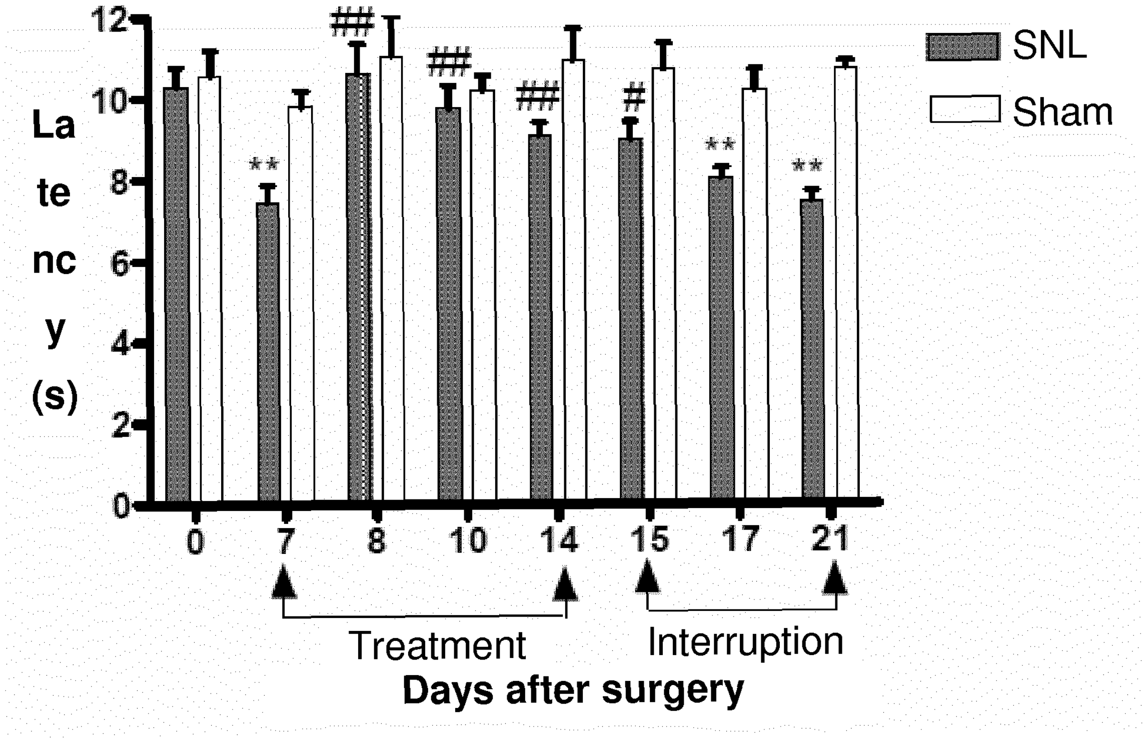
12
10
8
6
4
2
0
La
te
nc
y
(s)
##
##
##
#
**
**
**
SNL
Sham
0
7
8
10
14
15
17
21
Treatment
Interruption
Days after surgery

Figure 28

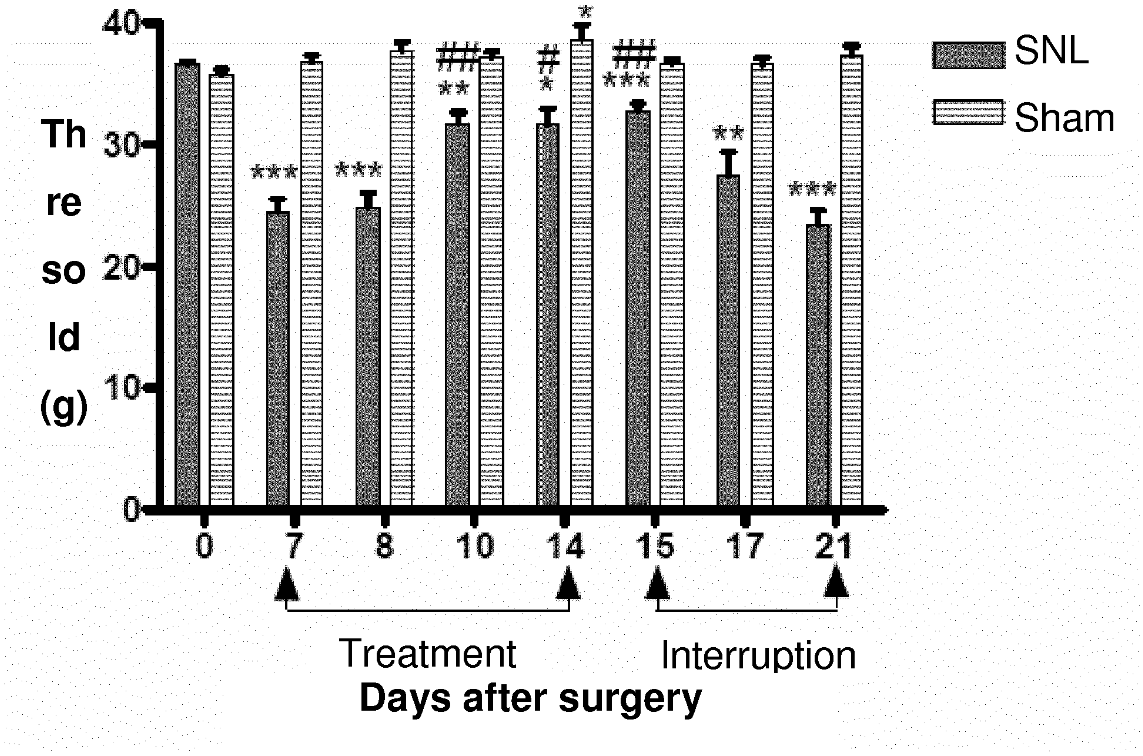
40
30
20
10
0
Th re so ld (g)
SNL
Sham
***
***
##
**
#
*
*
##
***
**
***
0
7
8
10
14
15
17
21
Treatment
Interruption
Days after surgery

Figure 29

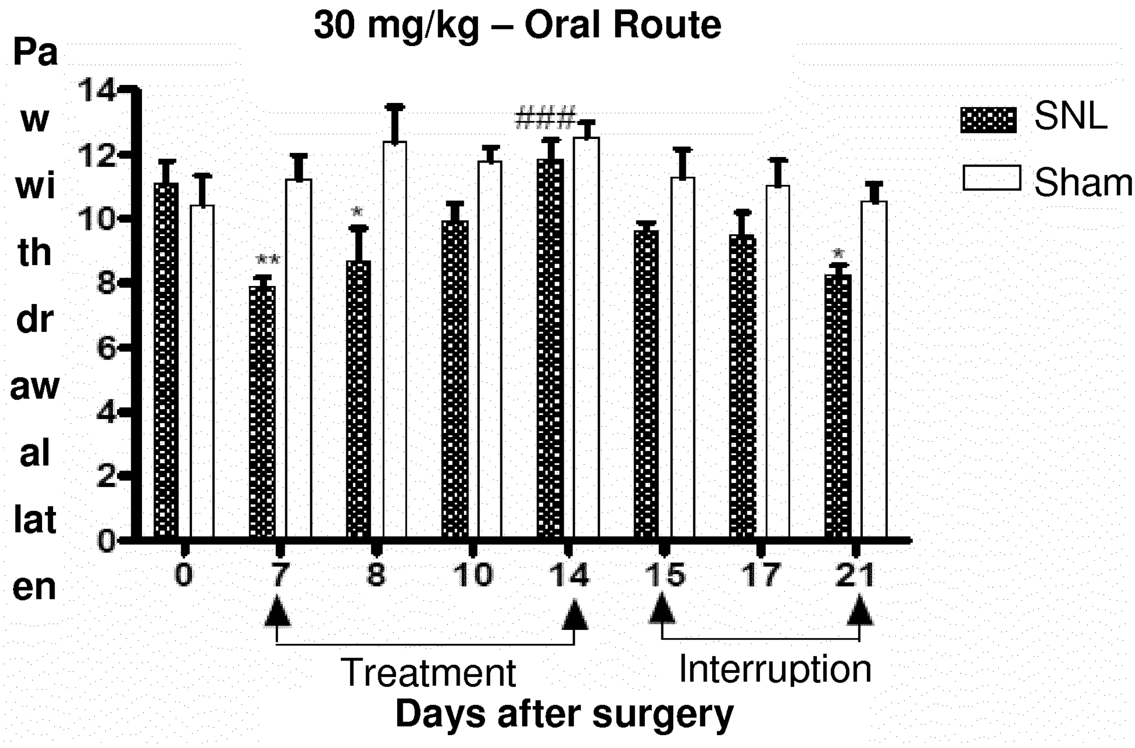
30 mg/kg – Oral Route
Paw withdrawal laten
0
2
4
6
8
10
12
14
**
*
###
*
0
7
8
10
14
15
17
21
SNL
Sham
Treatment
Interruption
Days after surgery

Figure 30

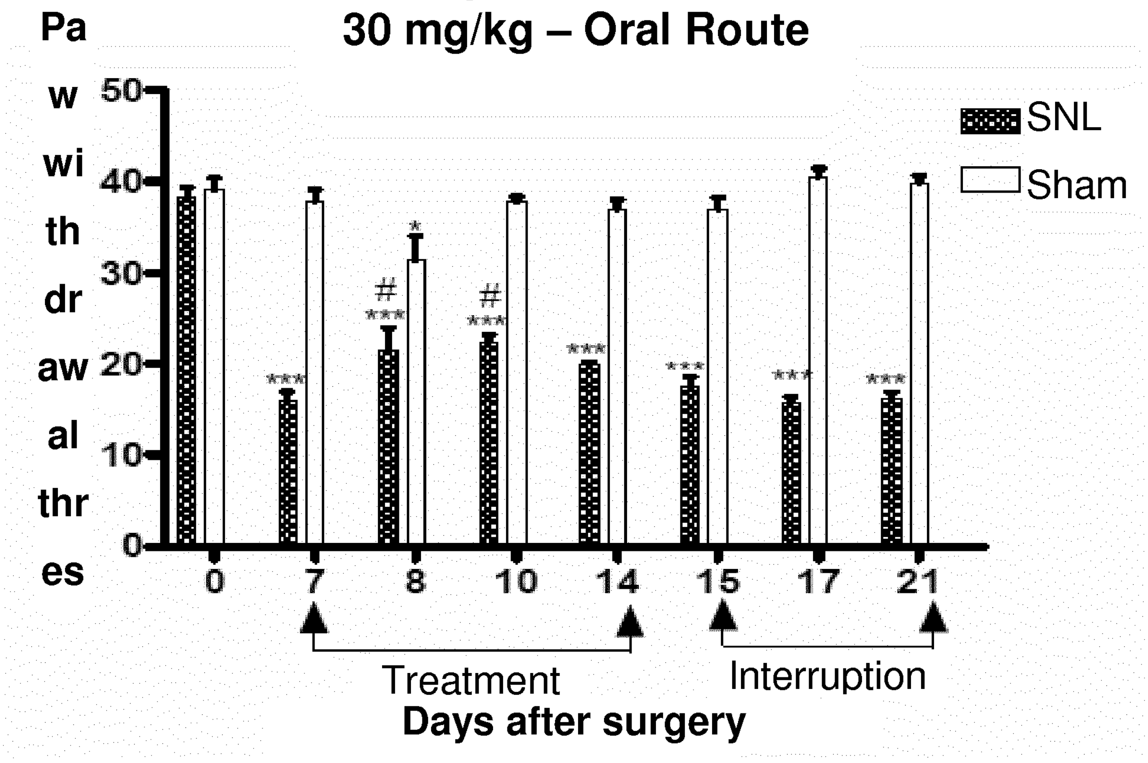
30 mg/kg – Oral Route
Paw withdrawal thres
0
10
20
30
40
50
SNL
Sham
0
7
8
10
14
15
17
21
Treatment
Interruption
Days after surgery

Figure 31

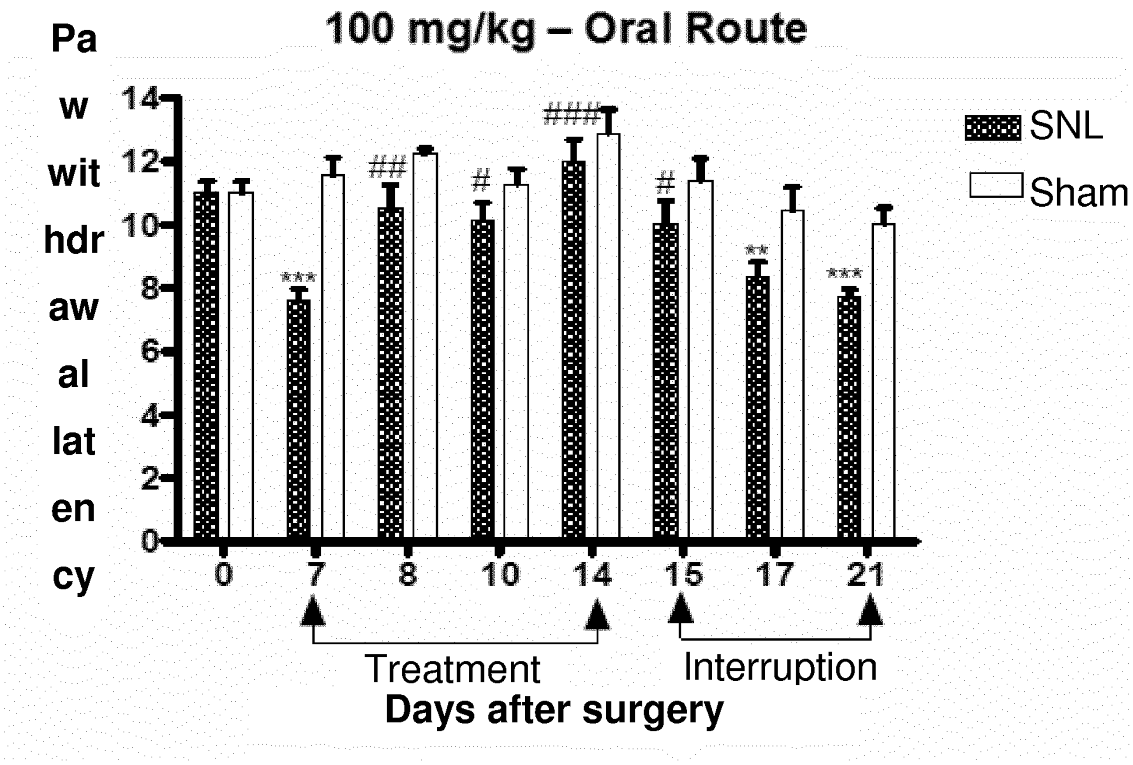
100 mg/kg – Oral Route
Pa
w
wit
hdr
aw
al
lat
en
cy
14
12
10
8
6
4
2
0
***
##
#
####
#
**
***
SNL
Sham
0
7
8
10
14
15
17
21
Treatment
Interruption
Days after surgery

Figure 32

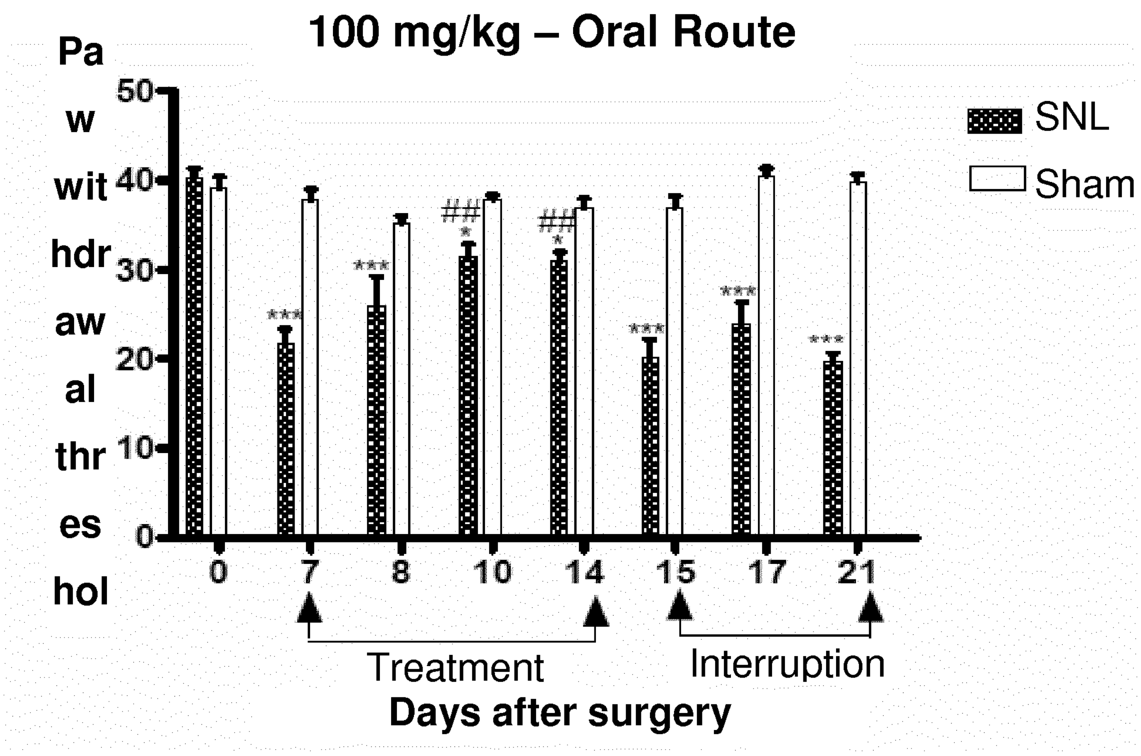
100 mg/kg – Oral Route
Pa
w
wit
hdr
aw
al
thr
es
hol
50
40
30
20
10
0
SNL
Sham
##
*
***
0
7
8
10
14
15
17
21
Treatment
Interruption
Days after surgery

**INTERNATIONAL SEARCH REPORT**

| International application No. |
|---|
| PCT/BR2013/000372 |

A. CLASSIFICATION OF SUBJECT MATTER

**C07D 231/12 (2006.01), C07D 233/54 (2006.01), C07D 239/20 (2006.01), C07D 401/06 (2006.01), C07D 413/06 (2006.01), C07D 419/06 (2006.01), A61P 25/06 (2006.01)**

According to International Patent Classification (IPC) or to both national classification and IPC

B. FIELDS SEARCHED

Minimum documentation searched (classification system followed by classification symbols)

Documentation searched other than minimum documentation to the extent that such documents are included in the fields searched

**SINPI- Base de dados do INPI-BR; Periódicos Capes**

Electronic data base consulted during the international search (name of data base and, where practicable, search terms used)

**EPODOC; ProQuest-Dialog (todas as bases de dados)**

C. DOCUMENTS CONSIDERED TO BE RELEVANT

| Category* | Citation of document, with indication, where appropriate, of the relevant passages | Relevant to claim No. |
|---|---|---|
| A | US 6525059 B1 (SEARLE CO [US])<br>25 February 2003 (2003-02-25)<br>Columns 3-10<br>Columns 29-43<br>Columns 101-114<br>Columns 125 | 1 to 26 |
| A | US 2009170833 A1 (AVENTIS PHARMA SA [FR])<br>02 July 2009 (2009-07-02)<br>**Abstract**<br>Pages 1 to 3 | 1 to 26 |
| A | WO 2012082947 A1 ( YANG YANG [US])<br>21 July 2012 (2012-06-21)<br>Pages 1 to 4 | 1 to 26 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 25 November 2013 | 03 12 13 |

| Name and mailing address of the ISA/BR | Authorized officer |
|---|---|
| INPI<br>INSTITUTO NACIONAL DA PROPRIEDADE INDUSTRIAL<br>Rua Sao Bento nº 1, 17º andar<br>cep: 20090-010, Centro - Rio de Janeiro/RJ | Rodinelli Borges de Oliveira |
| Facsimile No. +55 21 3037-3663 | Telephone No. +55 21 3037-3493/3742 |

Form PCT/ISA/210 (second sheet) (July 2009)

**INTERNATIONAL SEARCH REPORT**

International application No.
PCT/BR2013/000372

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 27 e 28
because they relate to subject matter not required to be searched by this Authority, namely:

**Box II.1**

**Claims 27 and 28 contain subject matter that falls under the provisions of PCT Rule 39.1(iv) relating to methods for treatment of the human or animal body by surgery or therapy.**

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

| International application No. |
|---|
| PCT/BR2013/000372 |

| C (Continuation). DOCUMENTS CONSIDERED TO BE RELEVANT | | |
|---|---|---|
| Category* | Citation of document, with indication, where appropriate of the relevant passages | Relevant to claim No. |
| A | EP 0378255 A2 (JANSSEN PHARMACEUTICA NV [BE])<br>18 July 1990 (1990-07-18)<br>Pages 3-5<br>----------------------------- | 1 a 26 |
| A | EP 1676574 A2 (JOHNSON JOHNSON VISION CARE [US])<br>05 July 2006 (2006-07-05)<br>Pages 11-17<br>----------------------------- | 1 a 26 |
| A | US 3821234 A (MERCK PATENT GMBH)<br>28 June 1974 (1974-06-28)<br>Claims 1-17<br>----------------------------- | 1 a 26 |
| A | EP 0776893 A1 (SANKYO CO [JP])<br>04 June 1997 (1997-06-04)<br>Claims 1-62<br>----------------------------- | 1 a 26 |

Form PCT/ISA/210 (continuation of second sheet) (July 2009)

**INTERNATIONAL SEARCH REPORT**
Information on patent family members

International application No.
PCT/BR2013/000372

| | | | |
|---|---|---|---|
| US 6525059 B1 | 2003-02-25 | AP 9901715 D0 | 1999-12-31 |
| | | AP 1246 A | 2004-02-07 |
| | | AP 200102172 D0 | 2001-06-30 |
| | | AR 035836 A1 | 2004-07-21 |
| | | AT 278685 T | 2004-10-15 |
| | | AT 373649 T | 2007-10-15 |
| | | AU 754830 B2 | 2002-11-28 |
| | | AU 774262 B2 | 2004-06-24 |
| | | AU 2145400 A | 2000-06-13 |
| | | AU 7588398 A | 1998-12-11 |
| | | BG 64313 B1 | 2004-09-30 |
| | | BG 103964 A | 2000-08-31 |
| | | BG 105620 A | 2002-01-31 |
| | | BR 9809147 A | 2000-08-01 |
| | | BR 9915420 A | 2002-01-22 |
| | | CA 2291115 A1 | 1998-11-26 |
| | | CA 2351725 A1 | 2000-06-02 |
| | | CN 1264377 A | 2000-08-23 |
| | | CN 1342157 A | 2002-03-27 |
| | | CZ 20011714 A3 | 2001-12-12 |
| | | DE 69920966 D1 | 2004-11-11 |
| | | DE 69937163 D1 | 2007-10-31 |
| | | DK 1144403 T3 | 2004-12-20 |
| | | DK 1500657 T3 | 2008-01-02 |
| | | EA 003925 B1 | 2003-10-30 |
| | | EA 005205 B1 | 2004-12-30 |
| | | EE 9900527 A | 2000-06-15 |
| | | EE 200100268 A | 2002-12-16 |
| | | EP 1000055 A1 | 2000-05-17 |
| | | EP 1144403 A1 | 2001-10-17 |
| | | EP 1500657 A1 | 2005-01-26 |
| | | ES 2229809 T3 | 2005-04-16 |
| | | ES 2289411 T3 | 2008-02-01 |
| | | GE P20033053 B | 2003-02-10 |
| | | GE P20053421 B | 2005-01-25 |
| | | HK 1040705 A1 | 2005-03-04 |
| | | HR P20010363 A2 | 2005-02-28 |
| | | HU 0001880 A2 | 2001-03-28 |
| | | HU 0200130 A2 | 2002-06-29 |
| | | ID 22982 A | 1999-12-23 |
| | | ID 29993 A | 2001-10-25 |
| | | IL 132991 D0 | 2001-03-19 |
| | | IL 143120 D0 | 2002-04-21 |
| | | IS 5257 A | 1999-11-19 |
| | | IS 5938 A | 2001-05-09 |
| | | JP 2002508754 A | 2002-03-19 |
| | | JP 2002530397 A | 2002-09-17 |
| | | KR 20010012854 A | 2001-02-26 |
| | | KR 20030002291 A | 2003-01-08 |
| | | NO 995695 D0 | 1999-11-19 |
| | | NO 20012456 D0 | 2001-05-18 |
| | | NZ 501112 A | 2002-10-25 |
| | | NZ 512344 A | 2003-11-28 |

Form PCT/ISA/210 (patent family annex) (July 2009)

**INTERNATIONAL SEARCH REPORT**
Information on patent family members

International application No.
PCT/BR2013/000372

| Documentos de patente citados no relatório de pesquisa | Data de publicação | Membro(s) da família de patentes | Data de publicação |
|---|---|---|---|
| | | OA 11914 A | 2006-04-11 |
| | | OA 12981 A | 2006-10-13 |
| | | PL 337020 A1 | 2000-07-31 |
| | | PL 353853 A1 | 2003-12-01 |
| | | PT 1144403 E | 2005-01-31 |
| | | PT 1500657 E | 2007-12-05 |
| | | SK 157899 A3 | 2000-08-14 |
| | | SK 6862001 A3 | 2002-06-04 |
| | | TR 200000235 T2 | 2000-05-22 |
| | | TR 200102001 T2 | 2001-12-21 |
| | | US 6423713 B1 | 2002-07-23 |
| | | US 6514977 B1 | 2003-02-04 |
| | | US 6617324 B1 | 2003-09-09 |
| | | US 6979686 B1 | 2005-12-27 |
| | | US 7071198 B1 | 2006-07-04 |
| | | US 2007078146 A1 | 2007-04-05 |
| | | US 2004176433 A1 | 2004-09-09 |
| | | US 7153959 B2 | 2006-12-26 |
| | | WO 0031063 A1 | 2000-06-02 |
| | | WO 9852940 A1 | 1998-11-26 |
| | | YU P35601 A | 2005-07-19 |
| | | ZA 9804358 A | 1999-05-24 |
| | | ZA 200103882 A | 2002-10-14 |
| US 2009170833 A1 | 2009-07-02 | AR 046632 A1 | 2005-12-14 |
| | | AT 428699 T | 2009-05-15 |
| | | AU 2004293214 A1 | 2005-06-09 |
| | | BR PI0416926 A | 2007-01-16 |
| | | CA 2544519 A1 | 2005-06-09 |
| | | CN 1906172 A | 2007-01-31 |
| | | CN 102174017 A | 2011-09-07 |
| | | DE 602004020660 D1 | 2009-05-28 |
| | | DK 1689720 T3 | 2009-08-10 |
| | | ES 2325446 T3 | 2009-09-04 |
| | | FR 2862647 A1 | 2005-05-27 |
| | | HR P20090391 T1 | 2009-08-31 |
| | | IL 175539 D0 | 2006-09-05 |
| | | JP 2007536213 A | 2007-12-13 |
| | | JP 4833078 B2 | 2011-12-07 |
| | | KR 20060123193 A | 2006-12-01 |
| | | MA 28147 A1 | 2006-09-01 |
| | | MY 141218 A | 2010-03-31 |
| | | NO 20062954 A | 2006-08-25 |
| | | NZ 548071 A | 2010-03-26 |
| | | PT 1689720 E | 2009-07-20 |
| | | RS 50836 B | 2010-08-31 |
| | | RU 2006122553 A | 2008-01-10 |
| | | RU 2376290 C2 | 2009-12-20 |
| | | RU 2009122368 A | 2010-12-20 |
| | | SG 131945 A1 | 2007-05-28 |
| | | SI 1689720 T1 | 2009-08-31 |
| | | TW I343811 B | 2011-06-21 |
| | | US 2005165005 A1 | 2005-07-28 |
| | | US 7524838 B2 | 2009-04-28 |

Form PCT/ISA/210 (patent family annex) (July 2009)

**INTERNATIONAL SEARCH REPORT**
Information on patent family members

International application No.
PCT/BR2013/000372

| Documentos de patente citados no relatório de pesquisa | Data de publicação | Membro(s) da família de patentes | Data de publicação |
|---|---|---|---|
| | | US 8048893 B2 | 2011-11-01 |
| | | UY 28635 A1 | 2005-06-30 |
| | | WO 2005051917 A1 | 2005-06-09 |
| | | ZA 200604051 A | 2007-12-27 |
| | | ZA 200706854 A | 2008-09-25 |
| WO 2012082947 A1 | 2012-06-21 | None | |
| EP 0378255 A2 | 1990-07-18 | AT 104971 T | 1994-05-15 |
| | | AU 617918 B2 | 1991-12-05 |
| | | AU 4777990 A | 1990-07-12 |
| | | CA 2007200 A1 | 1990-07-09 |
| | | CN 1044094 A | 1990-07-25 |
| | | CN 1034865 C | 1997-05-14 |
| | | DE 69008366 D1 | 1994-06-01 |
| | | DK 0378255 T3 | 1994-05-24 |
| | | ES 2055860 T3 | 1994-09-01 |
| | | FI 900085 A | 1990-07-10 |
| | | FI 94525 B | 1995-06-15 |
| | | GB 8900382 D0 | 1989-03-08 |
| | | HU 900064 D0 | 1990-03-28 |
| | | HU T52770 A | 1990-08-28 |
| | | HU 203747 B | 1991-09-30 |
| | | IE 900069 L | 1990-07-09 |
| | | IE 62874 B1 | 1995-03-08 |
| | | IL 92730 D0 | 1990-09-17 |
| | | JO 1605 B | 1990-07-01 |
| | | JP H02225482 A | 1990-09-07 |
| | | JP 2938492 B2 | 1999-08-23 |
| | | KR 0159099 B1 | 1998-12-01 |
| | | MA 21722 A1 | 1990-10-01 |
| | | NO 900071 D0 | 1990-01-08 |
| | | NO 173139 B | 1993-07-26 |
| | | NZ 231788 A | 1990-12-21 |
| | | PT 92806 A | 1990-07-31 |
| | | RU 2028297 C1 | 1995-02-09 |
| | | US 5140029 A | 1992-08-18 |
| | | US 5256659 A | 1993-10-26 |
| | | US 5284854 A | 1994-02-08 |
| | | ZA 9000123 A | 1991-09-25 |
| | | ZW 290 A1 | 1991-09-11 |
| EP 1676574 A2 | 2006-07-05 | EP 1676574 A3 | 2006-07-26 |
| US 3821234 A | 1974-06-28 | AT 317892 B | 1974-09-25 |
| | | AT 318604 B | 1974-11-11 |
| | | AT 320650 B | 1975-02-25 |
| | | AT 320651 B | 1975-02-25 |
| | | AT 320652 B | 1975-02-25 |
| | | AU 457015 B2 | 1974-12-19 |
| | | AU 3591071 A | 1972-05-24 |
| | | BE 776406 A1 | 1972-06-08 |
| | | BR 7108208 D0 | 1973-07-03 |
| | | CA 967575 A1 | 1975-05-13 |

Form PCT/ISA/210 (patent family annex) (July 2009)

**INTERNATIONAL SEARCH REPORT**
Information on patent family members

International application No.
PCT/BR2013/000372

| Documentos de patente citados no relatório de pesquisa | Data de publicação | Membro(s) da família de patentes | Data de publicação |
|---|---|---|---|
| | | CH 570997 A5 | 1975-12-31 |
| | | CH 571513 A5 | 1976-01-15 |
| | | CH 571514 A5 | 1976-01-15 |
| | | CH 571515 A5 | 1976-01-15 |
| | | CH 581650 A5 | 1976-11-15 |
| | | CS 166036 B2 | 1976-01-29 |
| | | CS 166037 B2 | 1976-01-29 |
| | | CS 166038 B2 | 1976-01-29 |
| | | CS 166039 B2 | 1976-01-29 |
| | | CS 166040 B2 | 1976-01-29 |
| | | DD 94994 A5 | 1973-01-12 |
| | | DE 2060816 A1 | 1972-06-29 |
| | | DK 133780 B | 1976-07-19 |
| | | ES 397786 A1 | 1975-05-16 |
| | | FR 2117971 A1 | 1972-07-28 |
| | | GB 1322270 A | 1973-07-04 |
| | | HU 164941 B | 1974-05-28 |
| | | IL 38177 D0 | 1972-01-27 |
| | | IT 7948130 D0 | 1979-02-26 |
| | | JP S5518711 B1 | 1980-05-21 |
| | | NL 7116991 A | 1972-06-13 |
| | | PL 83235 B1 | 1975-12-31 |
| | | PL 84240 B1 | 1976-03-31 |
| | | PL 84241 B1 | 1976-03-31 |
| | | PL 84242 B1 | 1976-03-31 |
| | | PL 84493 B1 | 1976-04-30 |
| | | RO 59096 A1 | 1976-01-15 |
| | | RO 59974 A1 | 1976-05-15 |
| | | RO 60110 A1 | 1976-06-15 |
| | | SE 390306 B | 1976-12-13 |
| | | YU 299271 A | 1979-09-10 |
| | | YU 34797 B | 1980-03-15 |
| | | ZA 7107685 A | 1972-08-30 |
| EP 0776893 A1 | 1997-06-04 | AT 213738 T | 2002-03-15 |
| | | AU 719158 B2 | 2000-05-04 |
| | | AU 7406596 A | 1997-06-05 |
| | | CA 2191815 A1 | 1997-06-02 |
| | | CN 1157286 A | 1997-08-20 |
| | | CN 1142932 C | 2004-03-24 |
| | | CZ 9603521 A3 | 1997-06-11 |
| | | CZ 288498 B6 | 2001-06-13 |
| | | DE 69619479 D1 | 2002-04-04 |
| | | DK 0776893 T3 | 2002-03-18 |
| | | ES 2170211 T3 | 2002-08-01 |
| | | HK 1011366 A1 | 2002-08-02 |
| | | HU 9603298 D0 | 1997-01-28 |
| | | HU 224225 B1 | 2005-06-28 |
| | | IL 119729 D0 | 1997-03-18 |
| | | JP H10182649 A | 1998-07-07 |
| | | JP 3017147 B2 | 2000-03-06 |
| | | JP H09235275 A | 1997-09-09 |
| | | JP 3088672 B2 | 2000-09-18 |
| | | JP H10152478 A | 1998-06-09 |

Form PCT/ISA/210 (patent family annex) (July 2009)

**INTERNATIONAL SEARCH REPORT**
Information on patent family members

International application No.
PCT/BR2013/000372

| Documentos de patente citados no relatório de pesquisa | Data de publicação | Membro(s) da familia de patentes | Data de publicação |
|---|---|---|---|
| | | JP H10182650 A | 1998-07-07 |
| | | JP 2000103791 A | 2000-04-11 |
| | | MX 9606082 A | 1998-08-30 |
| | | NO 965125 D0 | 1996-12-02 |
| | | NO 308300 B1 | 2000-08-28 |
| | | NZ 299859 A | 1997-11-24 |
| | | PT 776893 E | 2002-06-28 |
| | | RU 2135494 C1 | 1999-08-27 |
| | | US 6159967 A | 2000-12-12 |
| | | US 6448247 B1 | 2002-09-10 |
| | | ZA 9610116 A | 1997-06-02 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6403575 B1, Holladay
- EP 1065205 B1, Nakazato
- US 6806275 B2
- WO 200705425 A
- WO 2005075458 A
- US 20100249094 A
- EP 1316546 A
- WO 2011076744 A
- WO 2008011611 A


### Non-patent literature cited in the description


- The potencial of Epidemiology. **CROMBIE I.K.** Epidemiology of pain: a report of the Task Force on Epidemiology. Seattle: IASP Press, 1999, 1-5
- **ALMEIDA, RF ; ROIZENBLATT, S. ; TUFIK, S.** Afferent pain pathways: a neuroanatomical review. *Brain Res,* 2004, vol. 1000, 40-56
- **ASHBURN, M. A. ; STAATS, P. S.** Management of chronic pain. *Lancet,* 1999, vol. 353, 1865-1869
- **EL-SAYED, M.A.-A. ; ABDEL-AZIZ, N.I. ; ABDEL-AZIZ, A.A.-M. ; EL-AZAB, A.S. ; ELTAHIR, K.E.H.** *Bioorg. Med Chem.,* 2012, vol. 20, 3306-3316
- **GUPTON, J.T. et al.** *Tetrahedron,* 2006, vol. 62, 8243-8255
- **LAROCK, R.C.** Comprehensive Organic Transformations: A Guide to Functional Group Preparations. John Wiley & Sons, 1989
- **MIAO, L. ; SHU, H. ; NOBLE, A.R. ; FOURNET, S.P. ; EDWIN D. ; STEVENS, E.D. ; TRUDELL, M.L.** *ARKIVOC,* 2010, vol. iv, 6-14
- **STEHL, A. ; SEITZ, G. ; SCHULZ, K.** *Tetrahedron,* 2002, vol. 58, 1343-1354
- **SPRINGER, D.M. et al.** *Bioorg. Med Chem.,* 2000, vol. 8, 1087-1109
- **HOLUB, J.M. ; BURNHAM, B.S. et al.** *Molecules,* 2004, vol. 9, 135-157
- **LAROCK, R.C.** Comprehensive Organic Transformations: A Guide to Functional Group Preparations. John Wiley & Sons
- **JIANG, Y. et al.** *Angew. Chem. Int Ed,* 2011, vol. 50, 7304-7307
- **GREENE, T. W ; WUTS, P.G.M.** Protetive Groups in Organic Synthesis. John Wiley & Sons, 1991
- **LEJBEDEV, A.V. ; LEBEDEVA, A.B. ; SHELUDYAKOV, V.D. ; KOVALEVA, E.A. ; USTINOVA, O.L. ; KOZHEVNIKOV, I.B. ; RUSS, J.** *Gen. Chem.,* 2005, vol. 75, 782
- **MALLADI, S. ; ISLOOR, A.M. ; PEETHAMBAR, S.K. ; GANESH, B.M. ; GOUD, P.S.** *Der Pharma Chemica,* 2012, vol. 4, 43-45
- Remington's Pharmaceutical Sciences. 2011
- Hot Plate Model LE7406. Scientific Inc,
- **KURAISHI, Y. ; HARADA, Y. ; ARATANI, S. ; SATOH, M. ; TAKAGI, H.** *Brain Research,* 1983, vol. 273, 245-2523
- **LE BARS, D. ; GOZARIU, M. ; CADDEN, S.W.** Animal models of nociception. *Pharmacol. Rev.,* 2001, vol. 53 (4), 597-652
- **BENNETT. G. J. ; XIE, Y.K.** *Pain,* 1998, vol. 33, 87-107
- **HARGREAVES K. ; DUBNER R. ; BROWN F. ; FLORES C. ; JORIS J.** *Pain,* 1988, vol. 32 (1), 77-88
- **LE BARS D. ; GOZARIU M. ; CADDEN S.W.** *Pharmacology Reviews,* 2001, vol. 53 (4), 597-652
- **KIM, S.H. ; CHUNG, J.M.** *Pain,* 1992, vol. 50, 355-363
- **DUNHAM M.S. ; MIYA. R.T.** *J. Amer. Pharmac. Assoc. Sci. Edit.,* 1957, vol. 46, 208-209